(19) Europäisches Patentamt / European Patent Office / Office européen des brevets

(11) **EP 4 345 168 A2**

(12) **EUROPEAN PATENT APPLICATION**

(43) Date of publication:
03.04.2024 Bulletin 2024/14

(21) Application number: 24156863.3

(22) Date of filing: 17.11.2017

(51) International Patent Classification (IPC):
*C12Q 1/6827* *(2018.01)*

(52) Cooperative Patent Classification (CPC):
(C-Sets available)
**C12Q 1/6827** (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.11.2016 US 201662423574 P**

(62) Document number(s) of the earlier application(s) in accordance with Art. 76 EPC:
**17871912.6 / 3 541 934**

(71) Applicant: **LGC Clinical Diagnostics, Inc.**
**Milford, MA 01757 (US)**

(72) Inventor: **Konigshofer, Yves**
**Maryland (US)**

(74) Representative: **Potter Clarkson**
**Chapel Quarter**
**Mount Street**
**Nottingham NG1 6HQ (GB)**

Remarks:
• The complete document including Reference Table(s) and the Sequence Listing(s) can be downloaded from the EPO website
• This application was filed on 09-02-2024 as a divisional application to the application mentioned under INID code 62.

(54) **METHODS FOR PREPARING DNA REFERENCE MATERIAL AND CONTROLS**

(57)    Disclosed are controls and reference materials for use in identifying any of a number of genotypes and/or for use in identifying or characterizing a disease or condition. The controls and reference materials may be particularly useful for diagnostic tests that utilize circulating cell-free DNA (cfDNA). Also disclosed herein are methods of generating large quantities of cfDNA that preserve the original cfDNA size and input sequences. In some embodiments, the methods comprise amplifying, digesting, and purifying cfDNA isolated from a subject. The cfDNA may be circulating tumor DNA ("ctDNA). The methods disclosed herein result in a cfDNA or ctDNA clone library that is significantly more "commutable" compared to existing methods.

Figure 1
A

SAPL RESTRICTION ENZYME

RECOGNITION SEQUENCE
(NOT A PALINDROME; 1 IN 8,192 BASES)

(52) Cooperative Patent Classification (CPC): (Cont.)

C-Sets
**C12Q 1/6827, C12Q 2525/191, C12Q 2563/179;**
**C12Q 1/6827, C12Q 2525/204, C12Q 2535/122,**
**C12Q 2545/101, C12Q 2545/113,**
**C12Q 2600/166**

**Description**

**RELATED APPLICATION**

This application claims the benefit of priority to U.S. Provisional Patent Application serial number 62/423,574, filed November 17, 2016.

**BACKGROUND**

Most common human diseases and cancers are associated with the human genome. Some genetic alterations are inherited while other genetic alterations may accumulate at any instance during an individual's lifetime. Screening for genetic alterations associated with cancer and disease has been embedded in healthcare for decades. For example, Noninvasive prenatal testing (NIPT) has been developed for prenatal screening of trisomy 21, 18, and 13, and Next-Generation Sequencing (NGS) has been recently used to identify mutations associated with tumorigenesis in breast cancer. The human genome project laid the groundwork for genome-wide analysis of cancers and disease.

Traditionally, molecular diagnostics have been used to analyze tumors and genetic diseases. For example, molecular diagnostics have consisted of antibody-based tests (immunohistochemistry), in-situ hybridization with DNA probes (fluorescence in situ hybridization), or PCR and DNA sequencing based tests that query specific nucleotide sequences. DNA Sequencing is the precise process that determines the accurate ordering of the nucleotides in a DNA molecule. Until recently, DNA sequencing as a molecular diagnostic tool has been generally limited to the coding exons of one or two genes.

The first genome to be fully sequenced was the bacteriophage φX174 in 1977 by Sanger, while the first "long" genome to be fully sequenced was Epstein-Barr virus (170 Mbp) in 1984. DNA sequencing methods were non-automated techniques until the announcement of the first fully automated DNA sequencing platform by Applied Biosystems in 1987. In the following decade, DNA sequencing was pushed to the next level by the development of NGS techniques. These included the establishment of DNA pyrosequencing, colony sequencing, and massively parallel sequencing (MPSS).

Reference materials and controls are essential for ensuring that assays are accurate and precise. The advanced capabilities of NGS, such as the ability to sequence a random sampling of the human genome, have presented challenges for the design of reference materials that are commutable and retain the quality, and complexity of the sample.

Commutability is critically important for a reference material and a control, such as when a contrived analyte must perform similar to a natural analyte. The priority of commutability is the same regardless of the sample (*e.g.*, circulating cell free DNA ("cfDNA" or "ccfDNA") or circulating tumor DNA ("ctDNA")) or application (*e.g.*,. NIPT, NGS). For example, cfDNA isolated from blood can generally be incorporated with a reasonable efficiency into NGS libraries that rely on the ligation of double-stranded adapters to double-stranded cfDNA. cfDNA in blood is typically short - with an average length of about 170 bp - and similarly short DNA may be prepared from much longer genomic DNA by sonication. However, cfDNA reference materials that are derived from sonicated DNA can have significant limitations in that their length distributions are likely different from that of cfDNA, in that they do not have the same genomic biases found in cfDNA and their incorporation efficiency into libraries for sequencing can be significantly lower than for natural cfDNA. In regard to the latter, sonication of the input DNA has been demonstrated to damage the 5' and 3' ends of the input DNA so that only a small portion of DNA, *e.g.*, typically 5-20% of the input DNA, may be properly ligated to adapters at the 5' and 3' ends. Therefore, only a minimum amount of the input DNA is functional for sequencing methods that start with ligation of double-stranded adapters onto the 5' and 3' ends of the DNA. (See Aigrain et al., BMC Genomics, June 13 2016, 17:458) This may be lead to an overall limited quantity of output DNA for downstream analysis. For example, circulating tumor DNA (ctDNA) assays must detect variants at low frequencies from about 0.01% to about 0.5%. If an aliquot of a sample contains 4 copies of a mutation, but not a single copy is usable due to sonication, then the sequenced sample will appear to contain no mutations. Such problems cause sonicated DNA to be less commutable for sequencing assays, as more input DNA is needed than would be needed for a typical sample and the output library may have a different relative genomic representation than that of the input DNA.

When an NGS library is prepared, there may be a PCR amplification step that produces a sufficient concentration for sequencing. While this amplification step could be used to prepare a reference material that could be analysed on multiple sequencers, it cannot directly serve as a control or reference material for the full sequencing process. Furthermore, a typical NGS library is not necessarily directly compatible with other NGS assays and NGS platforms due to the presence of additional assay- and platform-specific adapters that have been attached at the 5' and 3' ends of the DNA. If not removed, the presence of additional adapters may lead to unexpected sequences and results.

Recently, cfDNA has been a source for molecular diagnostics in the analysis of tumors and genetic diseases. cfDNA is thought to be derived from cells undergoing apoptosis, and it is typically comprised of small DNA molecules that are around 170 base pairs (bp) in length. cfDNA has been reported to be unstable and short lived, with a half-life on the order of hours in circulation. At a typical serum concentration of just 5 ng/mL, cfDNA represents the diploid genomes of only about 1,000 cells/mL. In comparison, whole blood contains about 5,000,000 white blood cells/mL, which each contains a diploid genome.

In NIPS, cfDNA in the pregnant mother's blood has been a source for molecular diagnostics to determine whether the fetal component is indicative of one or more genetic abnormalities. The fetal fraction of cfDNA is an important component of the sensitivity and specificity of NIPS. However, the limited amount of fetal DNA in maternal plasma, use of standard library preparations that introduce artifacts into the starting sample, and the need to differentiate the fetal genotype from the maternal background present challenges that have hindered methods related to DNA sequencing in clinical diagnostics for NIPT. With a 10% fetal fraction, a trisomy only represents an increase from about 2,000 copies/mL to about 2,200 copies/mL of an affected chromosome in the cfDNA of a pregnant mother's blood. Detecting such an increase - or decrease - can be accomplished in many ways, and the methodologies of detection vary between assays. The ability to detect extra or missing copies is influenced by Poisson distributions and other sources of variance. For example, a digital PCR-based assay that measures the concentration of a single locus using about 5 ng of input DNA would be expected to measure about 2,000 copies in the presence of a normal fetus and about 2,100 copies in the presence of a fetus with Down syndrome. If a positive/negative cutoff is set in the middle at 2,050 copies, then approximately 13% of samples from women with a normal fetus would be false positives. Sensitivity and specificity could be improved by increasing the amount on input DNA, and using about 25 ng could reduce the false positive rate to below 1%, assuming no other sources of variance and a 10% fetal fraction. With a 4 % fetal fraction and about 25 ng of input DNA, the false positive rate would soar to 16% - and to over 30% with about 5 ng of input DNA. Sensitivity could also be improved by increasing the number of loci that are measured and because cfDNA samples are often limiting, commercial NIPS assays rely on this approach and perform many thousands of independent measurements of different chromosomes or parts thereof.

Even with thousands of measurements, detecting whether a chromosomal abnormality exists is further challenging because not all chromosomal sequences are represented equally. However, in all cases, a higher fetal fraction increases the ability to detect a fetal chromosomal abnormality in the pregnant mother's blood while a lower fetal fraction decreases it and could lead to a false negative result. Samples containing a fetal fraction at or below 4% present difficulties for all current assays, and about 5% of samples submitted to clinical laboratories for testing have a fetal fraction at or below 4%.

In order for any type of molecular diagnostic of cfDNA to perform within specifications, controls are generally required. In clinical testing, controls are critical to reduce the risk of reporting incorrect results due to otherwise undetected assay failures. For example, in Prenatal Testing, fetuses with chromosomal abnormalities are rare - even in "high risk" pregnant women - and they are becoming even rarer as more average- and low-risk women undergo NIPS testing. In NIPS testing, multiple samples are typically analyzed in parallel and, but even then, there is a high likelihood that none of the samples will contain fetal-derived cfDNA with chromosomal abnormalities. Consequently, many samples do not contain detectable amounts of the cfDNA for DNA sequencing, and thus, controls are not able to detect abnormalities in the samples analyzed. By including a control that mimics cfDNA that contains fetal-derived cfDNA with one or more chromosomal abnormalities, it becomes possible to evaluate whether an assay is capable of detecting such abnormalities in the other samples that were analyzed.

Lastly, the commutability of a reference material is critically important for assays using cfDNA or ctDNA. Currently, the median mutant allele fraction in clinical cfDNA samples has been reported to be around 0.5% (FDA-AACR meeting on liquid biopsies). Reference materials should allow laboratories to determine whether cfDNA assays are able to detect even lower mutant allele fractions. In approximately 1 mL of plasma, there is about 5 ng of cfDNA present, with a haploid genome containing about 3.5 pg of DNA, and an estimated 1,429 haploid genomes of cfDNA. At a mutant allele fraction of 0.5%, 1 mL of plasma would only contain approximately 7 copies of cfDNA. The efficiency of recovering sonicated DNA in an NGS library through adapter ligation has been reported to be about 5 to 20%. Thus, it is possible that when 5 ng of sonicated DNA are analyzed, not a single copy of cfDNA is recovered in the library. Due to poor sample commutability, a laboratory could reach the incorrect conclusion that an assay cannot reliably detect mutant alleles at a fraction of 0.5%. On the other hand, that very same assay might work with 5 ng of ccfDNA from a cancer patient, where mutant alleles are present at 0.5%.

Thus, the vast potential of molecular diagnostics for genetic diseases has yet to be realized (*e.g.*, fetal testing, paternity testing, predicting response to drug treatment, diagnosing or monitoring a medical condition, Mendelian disorders, genetic mosaicism, pathogen screening, microbiome profiling, non-invasive liquid tumor biopsy, and organ transplant monitoring). To date, existing molecular diagnostics approaches lack efficient solutions, including: (1) cfDNA assay controls to improve the quality of diagnostic testing, (2) amplification of individual DNA molecules from limited sources to generate large quantities of cfDNA that is commutable and accurately represents the starting sample, and (3) solutions to efficiently target sequencing to specific genomic loci, with sensitivity sufficient to discriminate true positive test results from false positive signals that arise during sample processing. Therefore, there is a significant need for improved, reproducible, and reliable methods to amplify individual DNA molecules from limited sources for screening and diagnosing human diseases and cancers, as well as improved controls for cfDNA assays would improve the quality of diagnostic testing.

**SUMMARY**

The present disclosure is generally directed to controls and reference materials for use in assays that analyze cell-free DNA (cfDNA), and to methods of generating large quantities of DNA that preserve the majority of the original DNA size and input sequences. In some aspects, the DNA is sonicated prior to library preparation. In other aspects, the DNA is not sonicated prior to library preparation. In some aspects, the methods comprise amplifying, digesting, and purifying cfDNA isolated from a subject. The cfDNA may be circulating tumor DNA ("ctDNA"). The methods disclosed herein result in amplified DNA, cfDNA or ctDNA that can be used as input material for assays and which is significantly more "commutable" compared to existing methods of producing controls and reference materials for such assays.

In some aspects, the present disclosure relates to a control or a reference material for use in identifying a genotype, comprising cell-free DNA (cfDNA), and a first mixture of nucleic acids; wherein the cfDNA has a base pair (bp) length of about 75 bps to about 600 bps, the first mixture of nucleic acids comprises a nucleotide sequence that encodes the genotype, and the first mixture of nucleic acids constitutes no more than about 10% of the total nucleic acids of the control or the reference material. The control or the reference material may further comprise a second mixture of nucleic acids comprising a nucleotide sequence that encodes a second genotype, wherein the genotype and the second genotype are alternate genotypes that occur at the same genetic locus. The genotype may be associated with a neoplasm, a provirus, or a hereditary disease.

In some aspects, the present disclosure relates to a control or a reference material for use in identifying a plurality of genotypes, comprising a sample of amplified cfDNA, wherein the amplified cfDNA has a base pair (bp) length of about 75 bps to about 600 bps, and a first mixture of nucleic acids, wherein the first mixture of nucleic acids comprises a first plurality of nucleotide sequences, each nucleotide sequence of the first plurality encodes a genotype of the plurality of genotypes, and the first mixture of nucleic acids constitutes no more than about 10% of the total nucleic acids of the control or the reference material.

In some aspects, the present disclosure relates to a control or a reference material for use in determining the ploidy of a chromosome in a fetus. The control or the reference material may comprise a first nucleotide sequence and a second nucleotide sequence, wherein the first nucleotide sequence has sequence homology with the chromosome; the second nucleotide sequence has sequence homology with a different chromosome; and the ratio of the copy number of the first nucleotide sequence to the copy number of the second nucleotide sequence is greater than 1: 1; a second mixture of nucleic acids comprising the first nucleotide sequence and the second nucleotide sequence, wherein the ratio of the copy number of the first nucleotide sequence to the copy number of the second nucleotide sequence is about 1: 1; and a sample of cfDNA, wherein the cfDNA has a base pair (bp) length of about 75 bps to about 600 bps, wherein no more than about 10% of the total nucleic acids of the control or the reference material are associated with the cfDNA. The control or the reference material may be, for example, human chromosome 8, 9, 13, 18, 21, 22, or X, which may display aneuploidy in a viable fetus. The different chromosome may be, for example, human chromosome 1, 6, or 7.

In some aspects, the present disclosure relates to a control or a reference material for use in determining the ploidy of a chromosome in a fetus. The control or the reference material may comprise a first mixture of nucleic acids comprising a first nucleotide sequence and a second nucleotide sequence, wherein the first nucleotide sequence has sequence homology with the chromosome; the second nucleotide sequence has sequence homology with a different chromosome; and the ratio of the copy number of the first nucleotide sequence to the copy number of the second nucleotide sequence is greater than 1: 1; a second mixture of nucleic acids comprising the first nucleotide sequence and the second nucleotide sequence, wherein the ratio of the copy number of the first nucleotide sequence to the copy number of the second nucleotide sequence is about 1: 1; and a sample of cfDNA, wherein the cfDNA has a base pair (bp) length of about 75 bps to about 600 bps, wherein no more than about 10% of the total nucleic acids of the control or the reference material are associated with the cfDNA.

In some aspects, the present disclosure relates to a control or a reference material for use in determining the ploidy of a chromosome in a fetus. The control or the reference material may comprise a first mixture of nucleic acids comprising a first plurality of nucleotide sequences and a second plurality of nucleotide sequences, wherein the first plurality of nucleotide sequences has sequence homology with the chromosome; the second plurality of nucleotide sequences has sequence homology with at least one autosome, wherein the at least one autosome does not comprise the chromosome; and the ratio of the copy number for any nucleotide sequence in the first plurality to the copy number for any nucleotide sequence in the second plurality is about 3:2; a second mixture of nucleic acids comprising the first plurality of nucleotide sequences and the second plurality of nucleotide sequences, wherein the ratio of the copy number for any nucleotide sequence in the first plurality to the copy number for any nucleotide sequence in the second plurality is about 1: 1; and a sample of cfDNA, wherein the cfDNA has a base pair (bp) length of about 75 bps to about 600 bps, wherein no more than about 10% of the total nucleic acids of the control or the reference material are associated with the cfDNA.

In some aspects, the present disclosure relates to a method for quantitative analysis of cell-free DNA (cfDNA) comprising treating the 5' end or 3' end or both of cfDNA with one or more end-repair enzymes to generate end-repaired cfDNA; ligating one or more adaptors, wherein the adaptors comprise a restriction enzyme site and a sequence that is dissimilar from any other sequence found in the organism that serves as the source of cfDNA, such as a sequence from respiratory syncytial virus (RSV) for human cfDNA, to each end of the end-repaired cfDNA to generate a cfDNA library; amplifying the cfDNA library to generate a cfDNA library clones; digesting the cfDNA library clones with the a restriction enzyme to generate a modified cfDNA clone library; wherein digesting the cfDNA library clones with the restriction enzyme removes the one or more adaptors; purifying the modified cfDNA clone library; and performing quantitative analysis of one or more target loci in the modified cfDNA library clones.

In some aspects, the present disclosure relates to a method of predicting, diagnosing, or monitoring a genetic disease in a subject comprising isolating or obtaining a sample of cfDNA from a subject; treating the 5' end or 3' end or both of cfDNA with one or more end-repair enzymes to generate end-repaired cfDNA; ligating one or more adaptors, wherein the adaptors comprise a restriction enzyme site and a sequence that is dissimilar from any other sequence found in the organism that serves as the source of cfDNA, such as a sequence from respiratory syncytial virus (RSV) for human cfDNA, to each end of the end-repaired cfDNA to generate a cfDNA library; amplifying the cfDNA library to generate a cfDNA library clone; digesting the cfDNA library clone with a restriction enzyme to generate a modified cfDNA clone library; wherein the digesting the cfDNA library clone with the restriction enzyme removes the one or more adaptors; purifying the modified cfDNA clone library; and performing a quantitative analysis of one or more target loci in the modified cfDNA library clone, wherein the detection of one or more genetic lesions in the one or more target genetic loci is prognostic for, diagnostic of, or monitors the progression of the genetic disease.

In some aspects, the present disclosure relates to a method of predicting, diagnosing, or monitoring a cancer in a subject comprising isolating or obtaining a sample of circulating tumor DNA ("ctDNA") from a subject; treating the 5' end or 3' end or both of ctDNA with one or more end-repair enzymes to generate end-repaired ctDNA; ligating one or more adaptors, wherein the adaptors comprise a restriction enzyme site and a sequence that is dissimilar from any other sequence found in the organism that serves as the source of cfDNA, such as a sequence from respiratory syncytial virus (RSV) for human cfDNA, to each end of the end-repaired ctDNA to generate a ctDNA library; amplifying the ctDNA library to generate a ctDNA library clone; digesting the ctDNA library clone with a restriction enzyme to generate a modified ctDNA clone library; wherein the digesting the ctDNA library clone with the restriction enzyme removes the one or more adaptors; purifying the modified ctDNA clone library; and performing a quantitative analysis of one or more target loci in the modified ctDNA library clone, wherein the identification or detection of one or more somatic alterations in the one or more target loci is prognostic for, diagnostic of, or monitors the progression of the cancer.

In some aspects, the present disclosure relates to a method for determining whether a sample comprises a genotype, comprising performing a diagnostic test on the sample; and performing the diagnostic test on a control or a reference material as disclosed herein, wherein the control or the reference material comprises the genotype; wherein: the sample is found to comprise the genotype if the diagnostic test indicates that both the sample and the control or the reference material comprise the genotype; the sample is found to not comprise the genotype if the diagnostic test indicates that the sample does not comprise the genotype but that the control or the reference material comprises the genotype; and the diagnostic test is found to be inconclusive if the test indicates that the control or the reference material does not comprise the genotype.

In some aspects, the present disclosure relates to a method wherein the cfDNA or ctDNA, prior to amplification, is a bisulfite converted sample of DNA. In some embodiments, the method for quantitative analysis of cell-free DNA (cfDNA) comprises initially converting the cfDNA to a bisulfite converted sample of cfDNA, prior to treating the 5' end or 3' end or both of the converted cfDNA with one or more end-repair enzymes to generate end-repaired cfDNA. In other embodiments, the method of predicting, diagnosing, or monitoring a genetic disease in a subject comprises isolating or obtaining a sample of cfDNA from a subject and subsequently converting the cfDNA to a bisulfite converted sample of cfDNA, prior to treating the 5' end or 3' end or both of the converted cfDNA with one or more end-repair enzymes to generate end-repaired cfDNA. In some embodiments, the method of predicting, diagnosing, or monitoring a cancer in a subject comprises isolating or obtaining a sample of circulating tumor DNA ("ctDNA") from a subject and subsequently converting the ctDNA to a bisulfite converted sample of ctDNA, prior to treating the 5' end or 3' end or both of the converted ctDNA with one or more end-repair enzymes to generate end-repaired ctDNA..

In any of the methods disclosed herein, the amplified cfDNA or cfDNA may be used as a control or a reference material.

In any of the methods disclosed herein, the periodicity of the cfDNA or ctDNA peaks is the same as the original sample (e.g., about 166 bps, 146 bps, 136 bps, 126 bps, and/or 116 bps).

## BRIEF DESCRIPTION OF THE FIGURES

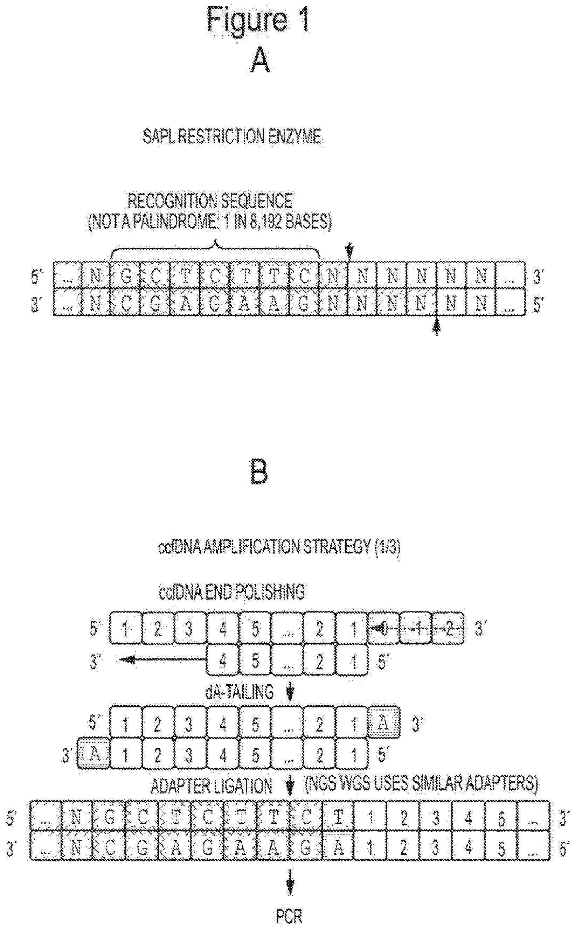

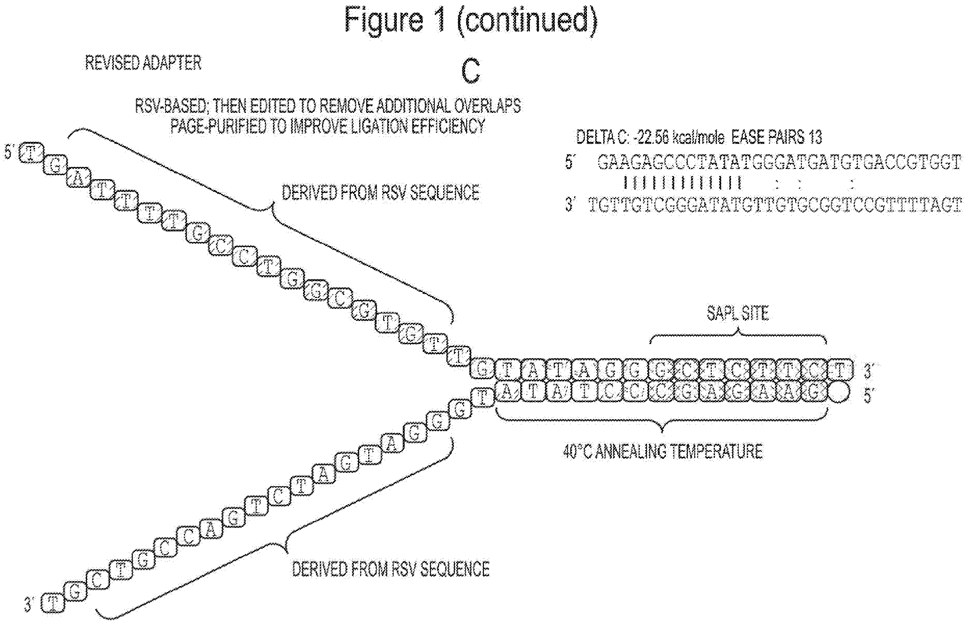

**Figure 1** consists of three panels, labelled panel (A), (B), and (C). Panel (A) depicts the SapI restriction enzyme and the enzyme's recognition site. As shown in panel (A), a SapI restriction is expected to occur approximately one in every 16,384 bases on a given strand of DNA or every 8,192 bases on both DNA strands combined since it is not palindromic.. Panel (B) depicts the steps that lead to PCR including end-polishing, dA-tailing and adapter ligation to cfDNA. Panel (C) shows the Y-style adaptor derived in part from sequences of an RNA virus (Respiratory syncytial virus (RSV)) that would not be expected when DNA is sequenced.

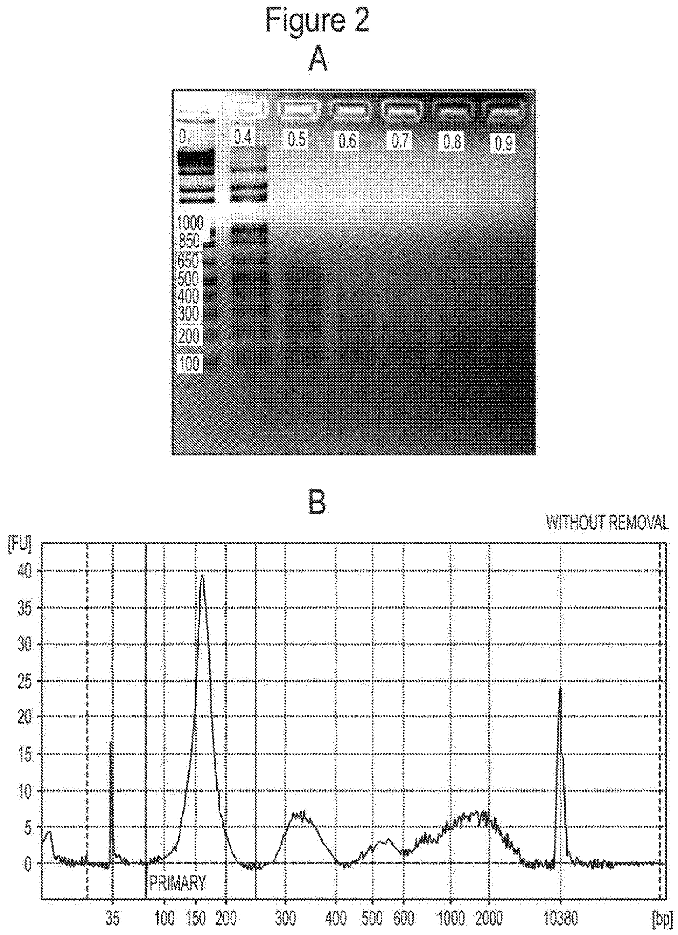

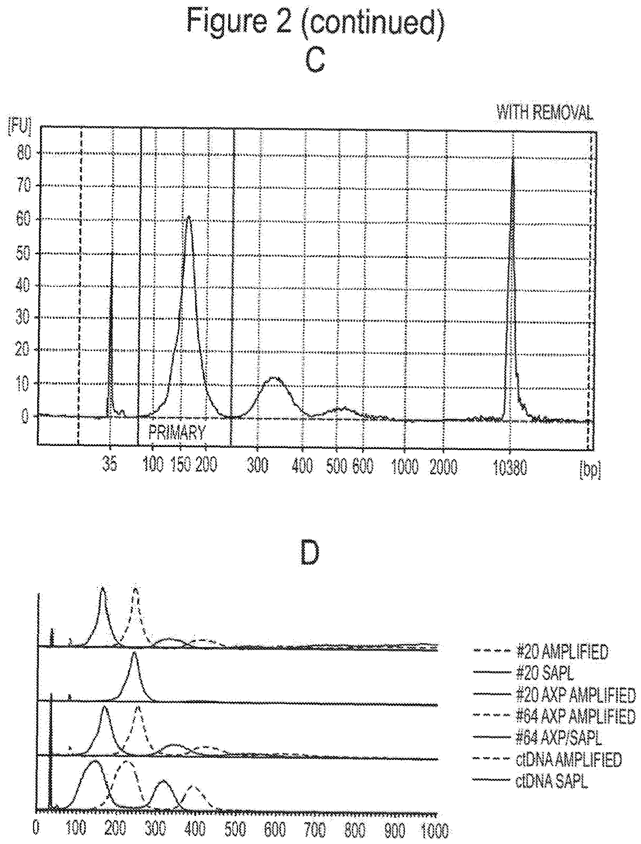

**Figure 2** consists of four panels, labelled panel (A), (B), (C), and (D). Panel (A) depicts a titration of Agencourt AMPure XP beads with DNA ladder in order to determine the appropriate ratio of bead volume to DNA volume in order to remove larger DNA molecules while retaining smaller DNA molecules. Panel (B) is a graph that depicts a Bioanalyzer trace of a cfDNA library that was not processed to remove large DNA molecules. Panel (C) is a graph that depicts a Bioanalyzer trace of a cfDNA library that was processed to remove large DNA molecules. Panel (D) depicts the size distributions of samples on a linear scale. Samples are shown in amplified and SapI digested form where larger DNA molecules are still present. The samples were also subsequently incubated with AMPure XP beads and amplified.

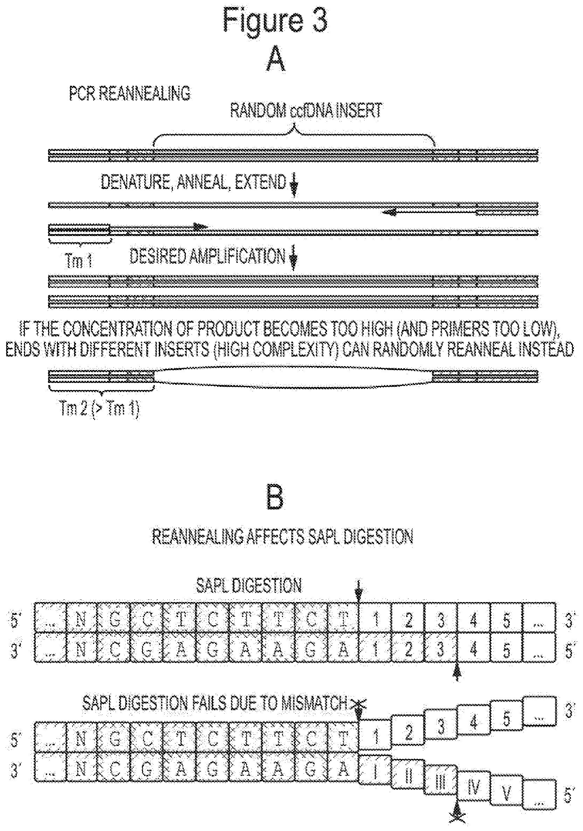

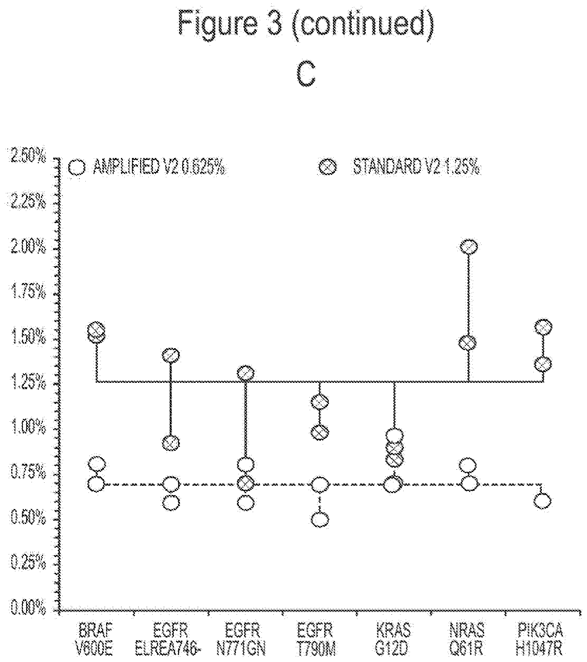

**Figure 3** consists of three panels, labelled panel (A), (B), and (C). Panel (A) depicts random cfDNA inserts undergoing repeated PCR cycles of denaturation, annealing and extension to achieve optimal amplification of the target sequence. The optimum number of cycles is established using a 2-fold serial dilution of the library. The library dilution where the amplification efficiency starts to decrease is used to determine the optimum number of PCR cycles. Panel (B) depicts how reannealing affects SapI digestion. The upper double stranded DNA molecule shows the desired PCR product that will allow for SapI digestion, while the bottom partially double stranded DNA molecule shows random reannealing that can take place during PCR may not allow for SapI digestion due to a lack of sequence complementarity where the enzyme cuts. Panel (C) depicts how the variant frequency of the amplified material were closer together in comparison with a sonicated 1.25% sample. This is consistent with improved performance and obtaining a large amount of genomic equivalents.

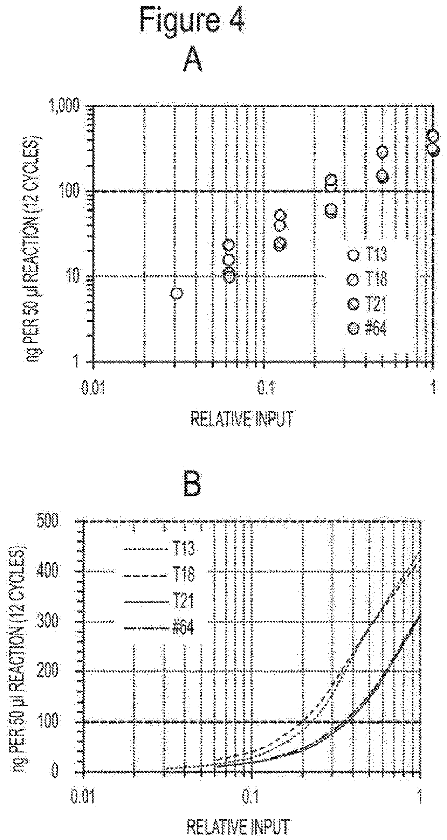

**Figure 4** consists of two panels, labelled panel (A) and (B). Panel (A) and (B) show PCR optimization as determined using a Qubit dsDNA BR assay.

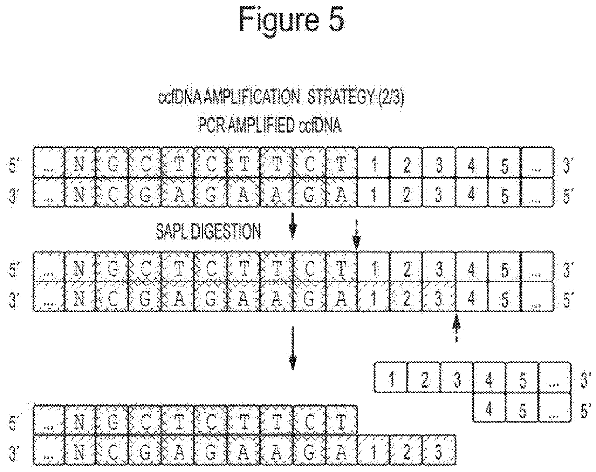

**Figure 5** depicts SapI restriction digestion of PCR amplified cfDNA products that lead to recessed 3' ends.

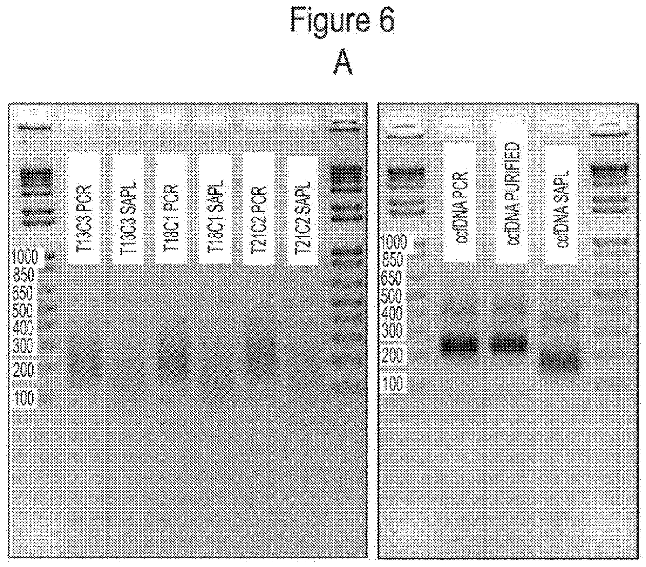

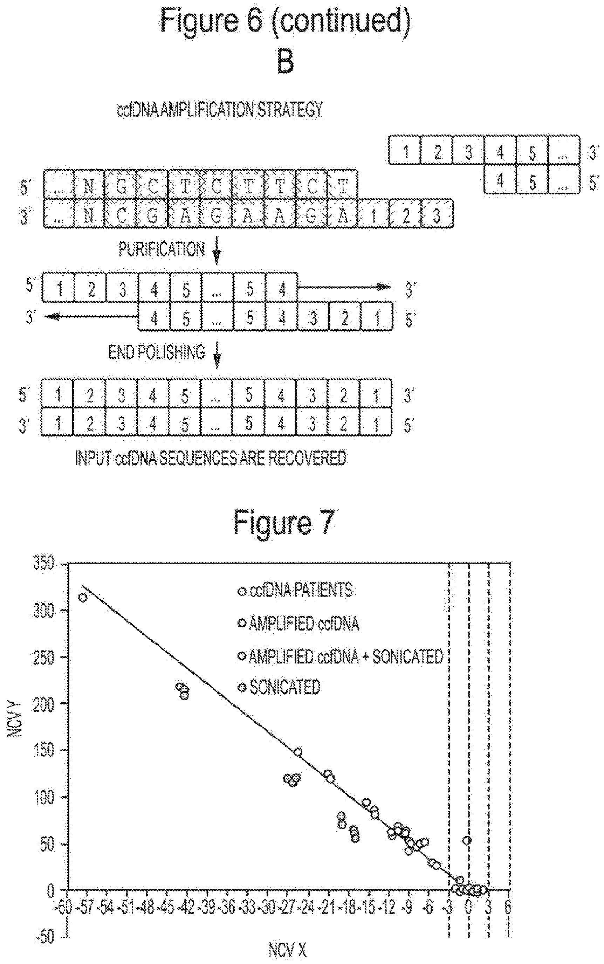

**Figure 6** consists of two panels, labelled panel (A) and (B). The gels in panel (A) show the difference in size between amplified libraries and SapI digests. The 2% agarose in 1x TAE gel on the left in panel (A) has lanes labelled T13C3, T18C1 and T21C2, which are amplified libraries and corresponding SapI digests from sonicated input DNAs that were from three trisomic placental trophoblast cell lines. The gel on the right in panel (A) shows a cfDNA-derived PCR amplified library before purification (PCR reaction), the guanidine thiocyanate purified library (remove primers, etc. from the PCR reaction) and its SapI digest in three lanes between two Invitrogen TrackIt™ 1 Kb Plus DNA Ladders. Panel (B) depicts the final steps of using the SapI digests of amplified cfDNA to recover the input cfDNA sequences through traditional end-polishing.

**Figure 7** is a graph of amplified cfDNA sent to a testing lab for analysis with an NIPT assay. The orange dot represents a sample of amplified cfDNA from a non-pregnant woman. The blue dot represents a sample of amplified cfDNA from a non-pregnant woman mixed with additional sonicated genomic DNA from a male aneuploid cell line at ~10% molar amount. Both samples were analyzed using a testing lab's NIPT assay with other samples that included the testing lab's own patient samples (grey dots) and reference materials that had been produced using sonicated genomic DNA (green dots).

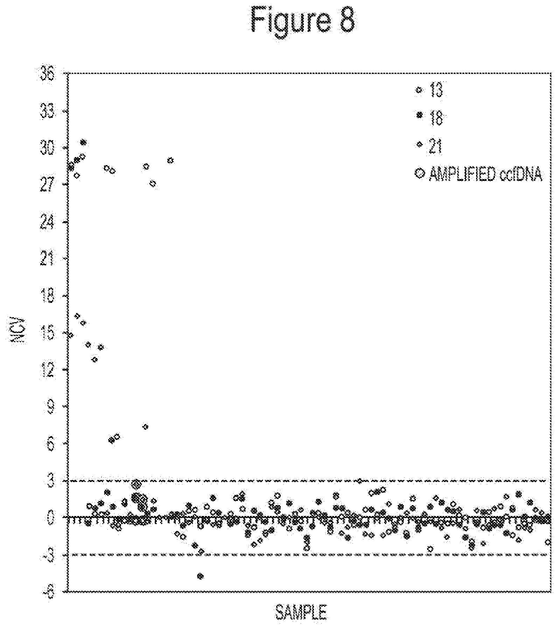

**Figure 8** is a graph of NCV (normalized chromosome values) obtained from a testing lab for chromosomes 13 (blue dots), 18 (green dots) and 21 (orange dots) on the vertical axis. A given NCV indicates the Z-score likelihood that a given chromosome is present at the normal amount. The horizontal axis shows different samples. The results for the two samples that used amplified cfDNA are circled in red with the NCV 21 results circled in bold. Sonicated DNA from a placental trophoblast cell line that is trisomic for chromosome 21 was added to the first (left) amplified sample.

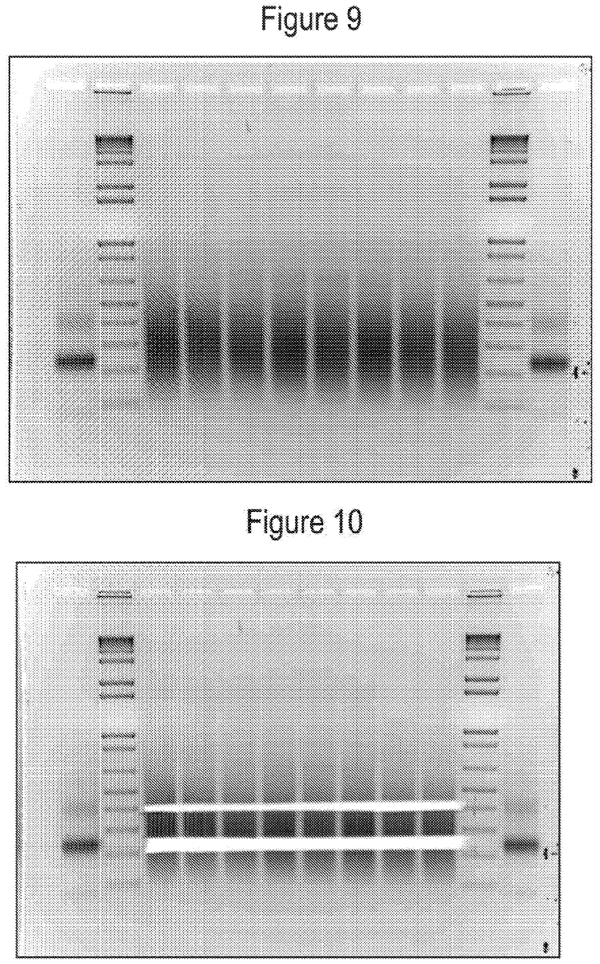

**Figure 9** depicts a DNA library derived from 10 $\mu$g of sonicated DNA separated on a 2% agarose 1x TAE gel (center lanes). Previously-amplified cfDNA was used as a guide in order to determine where library molecules would likely be found that have sizes similar to those found in cfDNA (outer lanes).

**Figure 10** depicts excision of adapter-containing sonicated DNA. DNA was extracted from the agarose using Qiagen reagents. Approximately 835 ng of DNA were recovered at approximately 8.35 ng/$\mu$l, as measured by Nanodrop.

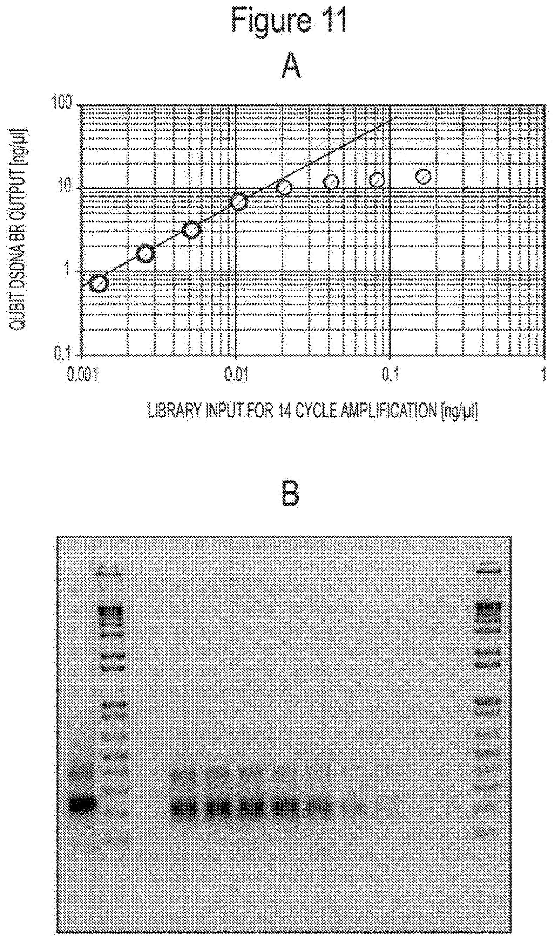

**Figure 11** consists of two panels, labelled panel (A) and (B). Panel (A) and (B) show PCR optimization as determined using a Qubit dsDNA BR assay.

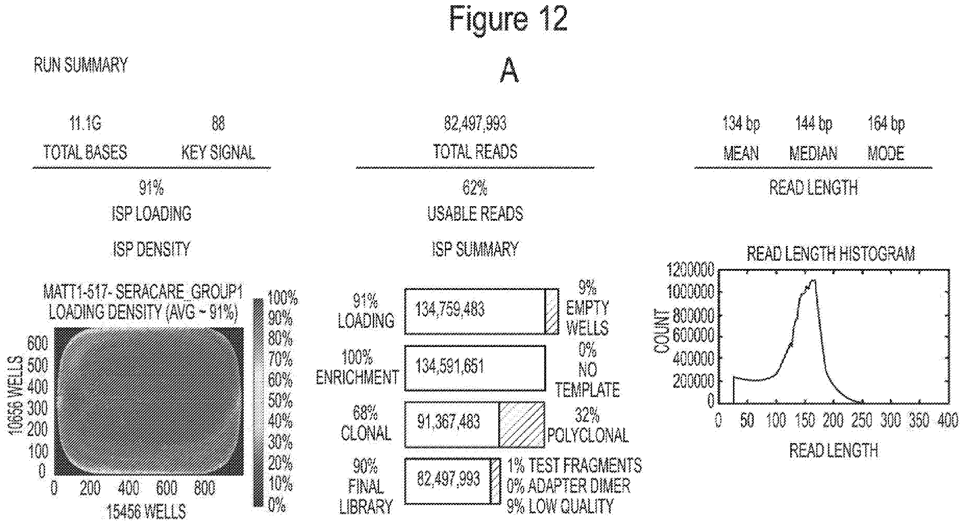

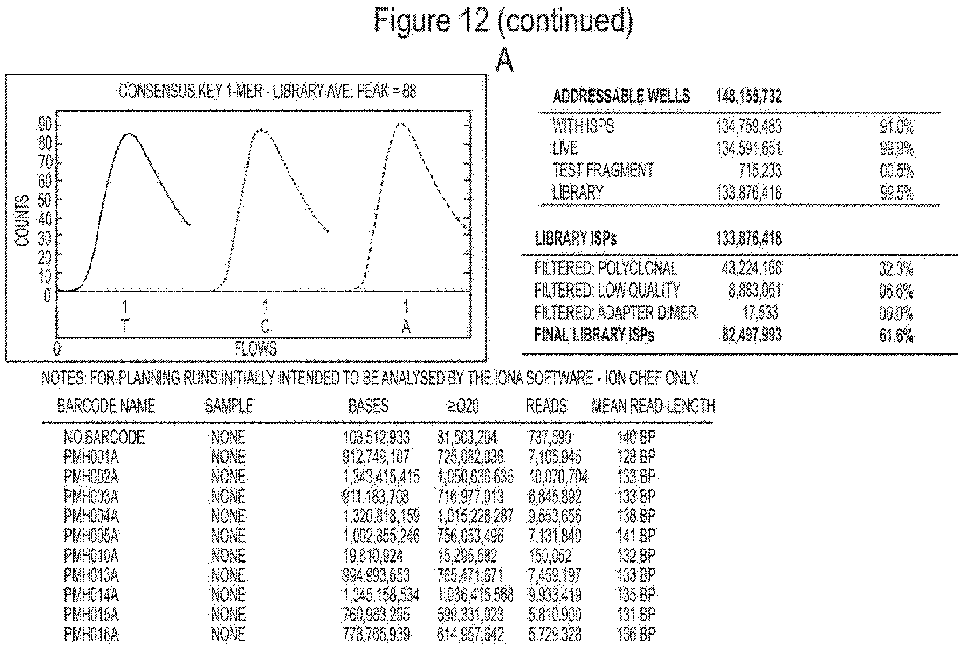

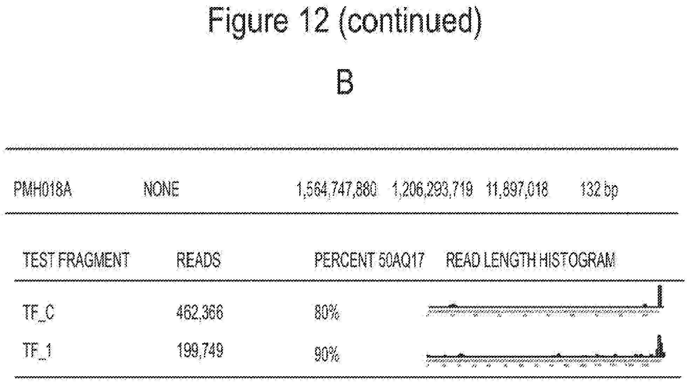

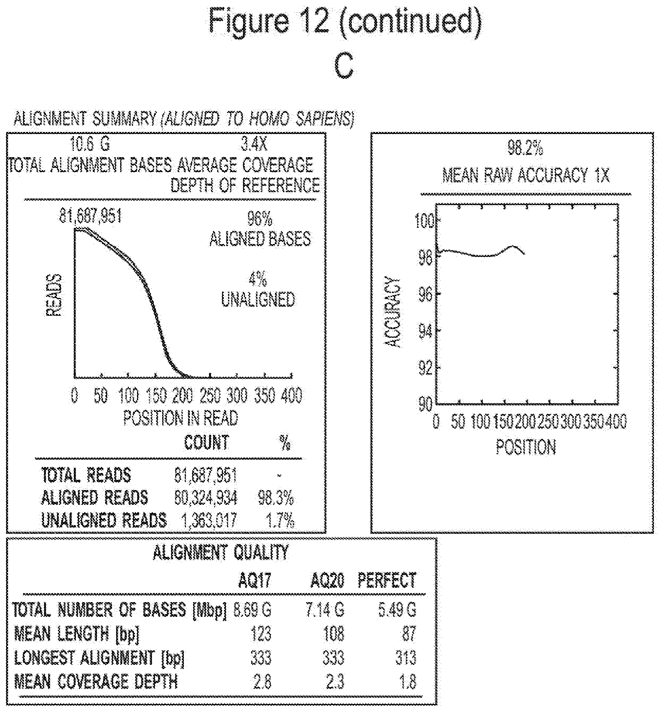

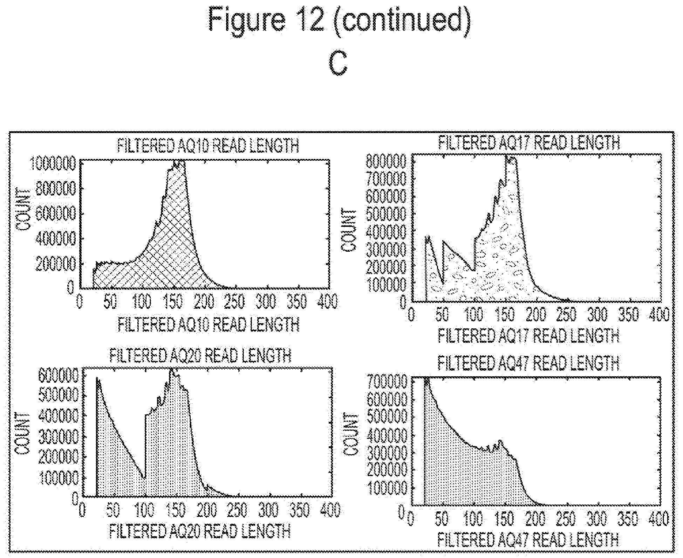

**Figure 12** consists of three panels, labelled panel (A), (B), and (C). Panels (A), (B), and (C) show an example run summary of samples using NGS technology.

**DETAILED DESCRIPTION**

*Overview*

In some aspects, disclosed herein is a broadly applicable methodology for creating whole process, commutable, and patient-like controls or reference materials for *in vitro* screening, testing, and/or diagnostics utilizing circulating cell free DNA (cfDNA) as a biomarker of interest. Disclosed herein are methods related to the generation of large quantities of cfDNA-like DNA for next-generation sequencing (NGS) based assays. The adapters in the library preparation may be modified to comprise a restriction enzyme site that allows for downstream recovery of the original input DNA. Such methods may also be used with DNA fragments, or circulating tumor DNA (ctDNA). The methods comprise amplification, digestion, and purification of circulating cell-free DNA (cfDNA) that leads to a cfDNA library clone that accurately represents the starting DNA sample ("input DNA). The methods comprise amplifying a cfDNA library and subsequently digesting the amplified cfDNA with a restriction enzyme that has the property of cutting outside the enzyme's recognition sequence. Such an enzyme is used for the removal of the adapters and the preservation of the original cfDNA size and input sequences. The removal of the adapters and the preservation of the original cfDNA size and input sequences in the methods disclosed herein result in a cfDNA clone library that is significantly more "commutable" compared to existing methods.

cfDNA is derived from the genomic DNA of a normal or diseased cell, and thus, it is an ideal biomarker for fetal genetic analysis and for identifying metastatic tumors. In this context, cfDNA is defined as DNA found in circulating blood, which is extracellular and may be associated with apoptotic bodies, nucleosomes, extracellular vesicles, or in another extracellular form. Characteristically, cfDNA is truncated in size, *e.g.*, as a result of enzymatic cleavage *in vivo* from germline DNA, which typically results in fragments that are 150-200 bp in length. Further, cfDNA is scarce in blood, with typical concentrations of 5-50 ng/mL. Applications for cfDNA analysis are expanding and include non-invasive prenatal screening/testing/diagnosis (NIPS/NIPT) and the analysis of circulating tumor DNA as it relates to cancer diagnostics and therapies.

*Definitions*

As used herein, the term "DNA" refers to deoxyribonucleic acid. In various embodiments, the term DNA refers to genomic DNA, recombinant DNA, synthetic DNA, or cDNA. In some embodiments, DNA refers to genomic DNA or cDNA. In particular embodiments, the DNA comprises a "target region." DNA libraries contemplated herein include genomic DNA libraries and cDNA libraries constructed from RNA, *e.g.*, an RNA expression library. In various embodiments, the DNA libraries comprise one or more additional DNA sequences and/or tags.

A "target genetic locus" or "target locus" or "DNA target region" refers to a region of interest within a DNA sequence. In some embodiments, targeted analyses are performed on the target locus. In other embodiments, targeted genetic analyses are performed on the target genetic locus. For example, the DNA target region may be a region of a gene that is associated with a particular genetic state, genetic condition, genetic diseases, or the region of a gene associated with fetal testing, genetic mosaicism, paternity testing, predicting response to drug treatment, diagnosing or monitoring a medical condition, microbiome profiling, pathogen screening, or organ transplant monitoring.

As used herein, the terms "circulating DNA," "circulating cell-free DNA," "cfDNA," "ccfDNA," and "cell-free DNA" are often used interchangeably and refer to DNA that is extracellular DNA, DNA that has been extruded from cells, or DNA that has been released from necrotic or apoptotic cells.

A "subject," "individual," or "patient" as used herein, includes any animal that exhibits a symptom of a condition that can be detected or identified with compositions contemplated herein. Suitable subjects include laboratory animals (such as mouse, rat, rabbit, or guinea pig), farm animals (such as horses, cows, sheep, pigs), and domestic animals or pets (such as a cat or dog). In particular embodiments, the subject is a mammal. In certain embodiments, the subject is a non-human primate and, in preferred embodiments, the subject is a human.

The term "biological fluid" herein refers to a liquid taken from a biological source and includes, for example, blood, serum, plasma, sputum, lavage fluid, cerebrospinal fluid, urine, semen, sweat, tears, saliva, and the like. The term "biological fluid" also includes DNA from the supernatant of human cells expanded in cell culture. As used herein, the terms "blood," "plasma" and "serum" expressly encompass fractions or processed portions thereof. Similarly, where a sample is taken from a biopsy, swab, smear, etc., the "sample" expressly encompasses a processed fraction or portion derived from the biopsy, swab, smear, etc.

The term "copy number", as used herein, refers to then number of times a nucleotide sequence occurs in a composition, such as a control or a mixture of nucleic acids. A nucleotide sequence may occur as a subsequence on different nucleic acids. For example, ten copies of a 35 base pair nucleotide sequence may occur in ten different nucleic acids in a mixture of nucleic acids, *e.g.*, wherein each of the ten different nucleic acids have different lengths. Similarly, the term "copy number" may refer to the concentration of a nucleotide sequence, *e.g.*, per unit volume. For example, ten copies of a 35 base pair nucleotide sequence may occur, on average, per every microliter of volume.

The term "control" may refer to a control sample, process control, run control, positive control, negative control, validation sample, proficiency sample, reference material, standard, or analytical standard. A control may be a positive control, *e.g.*, for monitoring the performance of a diagnostic test, such as sensitivity, accuracy, and/or precision. A control may be an analytical standard, *e.g.*, for calibrating a diagnostic test or for assessing its sensitivity. A control may be a process control, *e.g.*, for monitoring the sensitivity, accuracy, and/or precision of a diagnostic test during a single test or to assess trends over time (*e.g.*, drift). A process control may be used to monitor an entire process from sample preparation to data analysis or any step in between. A control may be a run control, such as a control sample, *e.g.*, for monitoring the sensitivity, accuracy, and/or precision of a diagnostic test in parallel with a patient sample. A control may be a standard, *e.g.*, for calibrating a diagnostic test or for use in measuring the nucleic acid concentration in a parallel sample (such as circulating cell-free nucleic acid). A control may be a reference material in some instances. Controls are generally used within a run (*e.g.*, a sequencing run), while reference materials are typically used to standardize assays and to validate assays.

The term "complexity" refers to a facet of a method that includes many independent measurements of a particular analyte. The controls and reference materials may be used at 0.1% or lower frequency. For example, in cancerous somatic mutations, there may only be 0.1% or lower frequency in the cfDNA of some cancer patients. With only 10 measurements of an analyte, as may result from having only 10 input copies in the library (*e.g.*, BRAF), a 0.1% V600E mutation would likely be missed because it would not be present in the library. Even if it were present in the library, a single copy in 10 input copies would now manifest as a 10% variant frequency instead of 0.1%. For this reason, the controls and reference materials disclosed herein may be used in high complexity for assays that analyze the same target region repeatedly. In NIPT, Natera's SNP-based assay also requires high complexity (the equivalent of around 1 mL of blood), as it requires many independent measurements of each SNP. Currently, however, NIPT assays that simply sequence the ends of cfDNA require less complexity (the equivalent of around 1 microliter of blood; although more is needed due to inefficiencies in library synthesis and sequencing).

The term "commutability" refers to when a contrived analyte must perform similar to a natural analyte. The priority of commutability is the same regardless of the sample (*e.g.*, circulating cell free DNA ("cfDNA") or circulating tumor DNA ("ctDNA")) or application (*e.g.*,. NIPT, NGS). For example, sonicated DNA may typically only incorporate into libraries with 5-20% efficiency (4% in my "cfDNA" example). If sonicated DNA is used as reference material for some NGS assays, then ~5- to ~20-fold more is needed than a similar mass of normal cfDNA in order to attain a given complexity in analyzable input material. This is important consideration for ctDNA assays. Also, subtle differences in cfDNA lengths can be important for assessing the proportion of fetus-derived DNA in NIPT. Sonication typically does not reproduce these subtle differences. Also, subtle differences in chromosomal region representation biases may be important in assessing the proportion of fetus-derived DNA in NIPT. Sonication typically does not reproduce this bias and can skew results (*e.g.*, it makes female PBMC-extracted genomic DNA that has been sonicated look like borderline Turner syndrome).

The term "diagnostic test" as used herein, refers to any test, screen, assay, or method that may be used to characterize a genotype, such as aneuploidy, copy number variant, allelomorphism, polymorphism, splice variant, regulatory variant, mutation, indel, trinucleotide repeat, premature stop codon, translocation, somatic rearrangement, gene fusion, genetic alteration, or the presence of foreign or exogenous nucleotide sequences (*e.g.*, a provirus), by analyzing a sample of nucleic acids. For example, a diagnostic test may refer to next generation sequencing ("NGS") or a diagnostic test may comprise NGS, *e.g.*, and subsequent analysis. Similarly, a diagnostic test may refer to any type of nucleic acid sequencing, or a diagnostic test may comprise any type of nucleic acid sequencing. In some embodiments, a diagnostic test may refer to nucleic acid hybridization, such as DNA microarray analysis. Similarly, a diagnostic test may comprise nucleic acid hybridization, such as DNA microarray analysis. In some embodiments, a diagnostic test may refer to quantitative PCR (qPCR) or digital PCR (dPCR), or a diagnostic test may comprise qPCR or dPCR.

The term "encode" as used herein refers to a property of one or more nucleotide sequences. A nucleotide sequence may encode a genotype if the nucleotide sequence comprises sufficient information to identify the genotype. For example, a nucleotide sequence encodes the Huntington's disease genotype if the nucleotide sequence comprises sufficient information to identify (1) a sequence of the Huntingtin gene and (2) a deleterious number of CAG trinucleotide repeats. A nucleotide sequence may encode an alternate genotype that occurs at the same genetic locus as the Huntington's disease genotype if the nucleotide sequence comprises sufficient information to identify (1) a sequence of the Huntingtin gene and (2) that the Huntingtin gene does not comprises a deleterious number of CAG trinucleotide repeats. Accordingly, many different nucleotide sequences may encode either the Huntington's disease genotype, an alternate genotype that occurs at the same genetic locus as the Huntington's disease genotype, or any genotype. Similarly, one or more nucleic acids may encode a genotype because nucleic acids comprise nucleotide sequences. Thus, a plurality of nucleic acids or a plurality of nucleotide sequences may encode a plurality of genotypes. Further, a mixture of nucleic acids may encode a genome or substantially all of a genome, *e.g.*, a mixture of nucleic acids may encode a plurality of genotypes that comprise substantially all of the genotypes in a genome. As used herein, a mixture of nucleic acids encodes substantially all of a genome if the mixture of nucleic acids was obtained, for example, by isolating nucleic acids from one or more cells and fragmenting the isolated nucleic acids, even though some nucleotide sequences may be depleted or lost during the isolation, fragmentation, or other steps. A mixture of nucleic acids may encode substantially all of a genome even if the mixture does not comprise, for example, mitochondrial nucleotide sequences. As defined herein, a mixture of nucleic acids may encode the ploidy of a chromosome, such as aneuploidy, if the mixture of nucleic acids comprises sufficient information to identify the ratio of the copy number of one or more nucleotide sequences that have sequence homology to the chromosome to the copy number of one or more nucleotide sequence that have sequence homology to at least one different chromosome. Similarly, a plurality of nucleotide sequences may encode the ploidy of a chromosome, such as aneuploidy, if the plurality comprises sufficient information to identify the ratio of the copy number of one or more nucleotide sequences that have sequence homology to the chromosome to the copy number of one or more nucleotide sequence that have sequence homology to at least one different chromosome.

The term "fetus" as used herein refers to a mammal at any stage of development between conception and birth.

The term "portion" is used herein in reference to the amount of sequence information of fetal and maternal nucleic acid molecules in a biological sample that in sum amount to less than the sequence information of <1 human genome.

The term "genotype" refers to a genetic trait, such as aneuploidy, copy number variant, allelomorphism, polymorphism, splice variant, regulatory variant, mutation, indel, trinucleotide repeat, premature stop codon, translocation, somatic rearrangement, gene fusion, genetic alteration, or the presence of a foreign or exogenous nucleotide sequence, such as a virus, provirus, or bacteria.

The term "mixture of nucleic acids" refers to a composition comprising at least two nucleic acids with different nucleotide sequences, i.e., a first nucleic acid may comprise a first nucleotide sequence and a second nucleic acid may comprise a second nucleotide sequence, wherein the first and second nucleotide sequences are different. Nevertheless, the first nucleotide sequence and the second nucleotide sequence may be related. For example, the first nucleotide sequence may have 100% sequence identity with a subsequence of the second nucleotide sequence, and the first and second nucleotide sequences may vary only in that the second nucleotide sequence is longer than the first nucleotide sequence. Similarly, the first nucleotide sequence and second nucleotide sequence may comprise regions with 100% sequence identity. The first nucleotide sequence and second nucleotide sequence may be related because they are derived from the same genome. In certain embodiments, each nucleic acid in a mixture of nucleic acids is either derived from a single genome (*e.g.*, a single human genome, which may be obtained from a human cell line) or designed to replicate a feature of a single genome, such as a genotype (*e.g.*, aneuploidy, polymorphism, mutation, allelomorphism, etc.). Thus, in some embodiments, a mixture of nucleic acids consists of nucleic acids that are isolated from a human cell line, such as a female cell line or a cell line comprising either a genotype or plurality of genotypes associated with a disease (*e.g.*, aneuploidy, a neoplasm, or a hereditary disease), which may be further processed, *e.g.*, to adjust the size of the nucleic acids to a desired range. A mixture of nucleic acids may comprise nucleic acids that are isolated from a single genome and additional nucleic acids, which may be added, for example, to introduce nucleotide sequences that encode a genotype, *e.g.*, to allow the mixture of nucleic acids to serve as a control for additional genotypes, or to mask a genotype, *e.g.*, in order to test the robustness of a diagnostic test. A mixture of nucleic acids may comprise nucleic acids that are isolated from a single genome but depleted of one or more nucleotide sequences, *e.g.*, to remove mitochondrial or ribosomal nucleotide sequences. A mixture of nucleic acids may be derived directly from a genome, *e.g.*, by isolating the nucleic acids from the genome, or a mixture of nucleic acids may be derived from a genome indirectly, *e.g.*, by amplifying the nucleotide sequences in a genome and/or by cloning the nucleotide sequences of a genome. A mixture of nucleic acids may comprise nucleic acids that are not derived from the same genome; for example, the mixture may be designed to replicate a feature of a single genome. For example, a mixture of nucleic acids may comprise a first nucleotide sequence with sequence homology to a first chromosome and a second nucleotide sequence with sequence homology to a second chromosome, wherein each nucleotide sequence is derived from the same genome, the first and second nucleotide sequences are derived from different genomes, or the first and/or second nucleotide sequences are synthesized and/or cloned.

The term "neoplasm" refers to tumors, benign tumors, precancerous tumors, malignant tumors, cancers, metastatic cancers, metastatic tumors, leukemia, and lymphomas, wherein a neoplastic cell has a genotype that is associated with the neoplasm.

The term "nucleic acid" refers to a DNA or RNA molecule. Single stranded nucleic acids each comprise one nucleotide sequence that spans the length of the nucleic acid and multiple different nucleotide sequences that are subsequences of the one nucleotide sequence. Similarly, double stranded nucleic acids each comprises two nucleotide sequences that span the length of the nucleic acid and multiple different nucleotide sequences that are subsequences of the two nucleotide sequences. For example, a double stranded nucleic acid that is 10 base pairs long comprises two nucleotide sequence that are each 10 nucleotides long (and related in that one sequence is the reverse complement of the other sequence); the same double stranded nucleic acid comprises four nucleotide sequences that are 9 nucleotides long and six nucleotide sequences that are 8 nucleotides long, etc.

The terms "polynucleotide", "nucleic acid" and "nucleic acid molecules" are used interchangeably and refer to a covalently linked sequence of nucleotides (i.e., ribonucleotides for RNA and deoxyribonucleotides for DNA) in which the 3' position of the pentose of one nucleotide is joined by a phosphodiester group to the 5' position of the pentose of the next, include sequences of any form of nucleic acid, including, but not limited to RNA, DNA and cfDNA molecules. The term "polynucleotide" includes, without limitation, single- and double-stranded polynucleotide.

The term "nucleotide sequence" refers to any sequence of consecutive nucleotides, *e.g.*, in a DNA or RNA molecule. A nucleotide sequence may be a subsequence of a different, longer nucleotide sequence. A mixture of nucleic acids may comprise a nucleotide sequence that is longer than the nucleic acids in the mixture, for example, when the mixture of nucleic acids is generated from longer nucleic acids (*e.g.*, by fragmenting genomic DNA); such nucleotide sequences may be identified, for example, by sequencing the nucleic acids in the mixture of nucleic acids. Nucleotide sequences are read from 5' to 3'.

The term "sequence homology" as used herein refers to a nucleotide sequence that has at least 95% sequence identity to another nucleotide sequence.

The term "sequence homology to a chromosome" as used herein refers to a nucleotide sequence that has at least 95% sequence identity to one chromosome and less than 95% sequence identity to every other chromosome in the genome from which the nucleotide sequence was derived. For example, a nucleotide sequence has sequence homology to chromosome Y if the nucleotide sequence has both at least 95% sequence identity to chromosome Y and less than 95% sequence identity with chromosomes 1-23 and X. Similarly, a nucleotide sequence has sequence homology to chromosome 1, if the nucleotide sequence has both at least 95% sequence identity to either copy of chromosome 1 in a genome and less than 95% sequence identity with every other chromosome in the genome.

The term "sequence identity" refers to the percentage of nucleotides in two nucleotide sequences that are identical upon aligning the two sequences. Two nucleotide sequences may be aligned using any alignment algorithm known in the art, such as those implemented in the BLAST or Clustal suites of programs. Alignment algorithms may introduce gaps in one or both nucleotide sequences to improve an alignment score, thereby increasing a calculated sequence identity; for sequences in which gaps improve an alignment score, "sequence identity" refers to the calculated sequence identity obtained by an alignment algorithm using default weights and default scoring functions for introducing and extending gaps (often referred to as gap penalties, such as gap opening penalties and gap extension penalties).

The term "sequence tag density" herein refers to the number of sequence reads that are mapped to a reference genome sequence. For example, the sequence tag density for chromosome 21 is the number of sequence reads generated by the sequencing method that are mapped to chromosome 21 of the reference genome. The term "sequence tag density ratio" herein refers to the ratio of the number of sequence tags that are mapped to a chromosome of the reference genome; i.e., chromosome 21, to the length of the reference genome chromosome 21.

The term "parameter" herein refers to a numerical value that characterizes a quantitative data set and/or a numerical relationship between quantitative data sets. For example, a ratio (or function of a ratio) between the number of sequence tags mapped to a chromosome and the length of the chromosome to which the tags are mapped, is a parameter.

The terms "threshold value" and "qualified threshold value" herein refer to any number that is calculated using a qualifying data set and serves as a limit of diagnosis of a copy number variation; such as an aneuploidy, in an organism.

The term "read" refers to a DNA sequence of sufficient length (*e.g.*, at least about 30 bp) that can be used to identify a larger sequence or region. A read can be aligned and specifically assigned to a chromosome or genomic region or gene.

The term "sequence tag" is herein used interchangeably with the term "mapped sequence tag" to refer to a sequence read that has been specifically assigned or mapped, to a larger sequence, such as a reference genome by alignment. Mapped sequence tags are uniquely mapped to a reference genome. For example, mapped sequence tags are assigned to a single location to the reference genome. Tags that can be mapped to more than one location on a reference genome, such as tags that do not map uniquely, are not included in the analysis.

The terms "aligned," "alignment," or "aligning" herein refer to one or more sequences that are identified as a match in terms of the order of their nucleic acid molecules to a known sequence from a reference genome. Such alignment can be done manually or by a computer algorithm, examples including the Efficient Local Alignment of Nucleotide Data (ELAND) computer program distributed as part of the Illumina Genomics Analysis pipeline. The matching of a sequence read in aligning can be a 100% sequence match or less than 100% (non-perfect match).

The term "reference genome" herein refers to any particular known genome sequence, whether partial or complete, of any organism or virus which may be used to reference identified sequences from a subject. For example, a reference genome used for human subjects as well as many other organisms is found at the National Center for Biotechnology Information at www.ncbi.nlm.nih.gov. A "genome" refers to the complete genetic information of an organism or virus, expressed in nucleic acid sequences.

The terms "artificial target sequences genome" and "artificial reference genome" herein refer to a grouping of known sequences that encompass alleles of known polymorphic sites. For example, a "SNP reference genome" is an artificial target sequences genome comprising a grouping of sequences that encompass alleles of known SNPs.

The term "clinically-relevant sequence" herein refers to a nucleic acid sequence that is known or is suspected to be associated or implicated with a cancer, genetic, or disease condition. Determining the absence or presence of a clinically-relevant sequence can be useful in determining a diagnosis or confirming a diagnosis of a medical condition, or providing a prognosis for the development of a disease.

The term "derived" when used in the context of a nucleic acid or a mixture of nucleic acids, herein refers to the means whereby the nucleic acid(s) are obtained from the source from which they originate. For example, in one embodiment, a mixture of nucleic acids that is derived from two different genomes means that the nucleic acids, such as cfDNA, were naturally released by cells through naturally occurring processes, such as necrosis or apoptosis. In another embodiment, a mixture of nucleic acids that is derived from two different genomes means that the nucleic acids were extracted from two different types of cells from a subject.

The term "plurality" is used herein in reference to a number of nucleic acid molecules or sequence tags that is sufficient to identify significant differences in copy number variations (*e.g.*, chromosome doses) in test samples and qualified samples using the disclosed methods. In some embodiments, at least about $3 \times 10^6$ sequence tags, at least about $5 \times 10^6$ sequence tags, at least about $8 \times 10^6$ sequence tags, at least about $10 \times 10^6$ sequence tags, at least about $15 \times 10^6$ sequence tags, at least about $20 \times 10^6$ sequence tags, at least about $30 \times 10^6$ sequence tags, at least about $40 \times 10^6$ sequence tags, or at least about $50 \times 10^6$ sequence tags comprising between 20 and 40 bp reads are obtained for each test sample.

The terms "library" and "sequencing library" herein refer to a collection or plurality of template molecules which share common sequences at their 5' ends and common sequences at their 3' ends.

The terms "blunt-ending," "end-repairing," and "end-polishing" are used herein interchangeably to refer to an enzymatic process that result in both strands of a double stranded DNA molecule to terminate in a base pair.

The term "dA-tailing" herein refers to an enzymatic process that adds at least one deoxyadenosine to the 3' end of DNA.

The term "adaptor-ligating" herein refers to an enzymatic process that ligates a DNA adaptor sequence to DNA fragments.

A "single nucleotide polymorphism" (SNP) occurs at a polymorphic site occupied by a single nucleotide, which is the site of variation between allelic sequences. The site is usually preceded by and followed by highly conserved sequences of the allele (*e.g.*, sequences that vary in less than 1/100 or 1/1000 members of a population). A SNP usually arises due to substitution of one nucleotide for another at the polymorphic site. A transition is the replacement of one purine by another purine or one pyrimidine by another pyrimidine. A transversion is the replacement of a purine by a pyrimidine or vice versa. SNPs can also arise from a deletion of a nucleotide or an insertion of a nucleotide relative to a reference allele. Single nucleotide polymorphisms (SNPs) are positions at which two alternative bases occur at appreciable frequency (>1%) in the human population, and are the most common type of human genetic variation.

As used herein, the term "short tandem repeat" or "STR" as used herein refers to a class of polymorphisms that occurs when a pattern of two or more nucleotides are repeated and the repeated sequences are directly adjacent to each other. The pattern can range in length from 2 to 10 base pairs (bp) (*e.g.*, (CATG)n in a genomic region) and is typically in the noncoding intron region. By examining several STR loci and counting how many repeats of a specific STR sequence there are at a given locus, it is possible to create a unique genetic profile of an individual.

As used herein, the term "miniSTR" herein refers to tandem repeat of four or more base pairs that spans less than about 300 base pairs, less than about 250 base airs, less than about 200 base pairs, less than about 150 base pairs, less than about 100 base pairs, less than about 50 base pairs, or less than about 25 base pairs. "miniSTRs" are STRs that are amplifiable from cfDNA templates.

The term "tandem SNPs" herein refers to two or more SNPs that are present within a polymorphic target nucleic acid sequence.

As used herein, the term "enriched library" herein refers to a sequencing library comprising amplified polymorphic target nucleic acid sequences. An example of an enriched library is a sequencing library comprising naturally-occurring cfDNA or ctDNA sequences and amplified target nucleic acid sequences. An "unenriched library" herein refers to a sequencing library that does not comprise naturally-occurring cfDNA or ctDNA sequences, (i.e., a library generated from naturally-occurring cfDNA sequences).

As used herein, the term "naturally-occurring cfDNA sequences" refers to cfDNA fragments as they are present in a sample, and in contrast to genomic DNA fragments that are obtained by other methods (*e.g.*, fragmentation, sonication).

The term "aneuploidy" herein refers to an imbalance of genetic material caused by a loss or gain of a whole chromosome, or part of a chromosome.

The term "chromosomal aneuploidy" herein refers to an imbalance of genetic material caused by a loss or gain of a whole chromosome, and includes germline aneuploidy and mosaic aneuploidy.

The term "partial aneuploidy" herein refers to an imbalance of genetic material caused by a loss or gain of part of a chromosome, such as partial monosomy and partial trisomy, and encompasses imbalances resulting from translocations, deletions and insertions.

The term "copy number variation" herein refers to variation in the number of copies of a nucleic acid sequence that is 1 kb or larger present in a test sample in comparison with the copy number of the nucleic acid sequence present in a reference sample. A "copy number variant" refers to the 1 kb or larger sequence of nucleic acid in which copy-number differences are found by comparison of a sequence of interest in test sample with that present in a reference sample. Copy number variants/variations include deletions, including microdeletions, insertions, including microinsertions, duplications, multiplications, inversions, translocations and complex multi-site variants. CNV encompass chromosomal aneuploidies and partial aneuplodies.

The phrase "ratio of the copy number of any nucleotide sequence that has sequence homology with a chromosome to the copy number of any nucleotide sequence that has sequence homology to a different chromosome" and similar phrases are used herein to describe the copy number of a chromosome relative to the copy number of a different chromosome from the same genome or from the same mixture of nucleic acids. Chromosomes 1, 6, and 7 are frequently used as reference chromosomes, because aneuploidy has not observed for these chromosomes in viable humans. Thus, for example, the ratio of the copy number of any nucleotide sequence that has sequence homology to chromosome 1 to the copy number of any nucleotide sequence that has sequence homology to chromosome 6 should be 1:1 in any mixture of nucleic acids that comprises a genome, that comprises substantially all of a genome, or that is designed to replicate the stoichiometry of chromosome 1 and chromosome 6 in a genome. Nevertheless, a chromosome may comprise multiple copies of a nucleotide sequence that has sequence homology to the chromosome, *e.g.*, the chromosome may comprise paralogous nucleotide sequences, such as copies of paralogous genes. The phrase "ratio of the copy number of any nucleotide sequence that has sequence homology with a chromosome to the copy number of any nucleotide sequence that has sequence homology to a different chromosome," and variants thereof, *does not* include nucleotide sequences that occur more than once in a G0 or G1 phase chromosome or more than once on a chromatid. For example, if a nucleotide sequence occurs more than once on the same chromatid, then the nucleotide sequence is not used to calculate a copy number ratio. Similarly, a chromosome may comprise nucleotide sequences that do not occur in the second copy of the chromosome, *e.g.*, for genomes that comprise heterozygous genotypes. The phrase "ratio of the copy number of any nucleotide sequence that has sequence homology with a chromosome to the copy number of any nucleotide sequence that has sequence homology to a different chromosome," and variants thereof, only includes nucleotide sequences that occur in each chromosome of a chromosome pair (*e.g.*, for disomic autosomes) or in each instance of a particular chromosome (*e.g.*, for aneuploidic autosomes). Thus, a nucleotide sequence that has sequence homology with a chromosome is *not* used to calculate a copy number ratio if the nucleotide sequence lacks sequence homology with each copy of the chromosome.

I. NUCLEIC ACIDS

In some aspects, disclosed herein are controls comprising cfDNA and a first mixture of nucleic acids, such as a control comprising cfDNA and a first mixture of nucleic acids that encodes a genotype. The control may be a control for use in determining the ploidy of a chromosome in a fetus, *e.g.*, for use in calibrating an assay or diagnostic test or for use as a run control in an assay or diagnostic test. The chromosome may be human chromosome 1, 2, 3, 4, 5, 6, 7, 8, 9, 10, 11, 12, 13, 14, 15, 16, 17, 18, 19, 20, 21, 22, X, or Y. In some embodiments, the chromosome is human chromosome 8, 9, 13, 18, 21, 22, or X. The chromosome may be an autosome or a sex chromosome. In some embodiments, the control is a control for use in identifying a genotype. The genotype may be a genetic disease or the genotype may be associated with cancer. The genotype may be associated with a neoplasm, provirus, or hereditary disease. The genotype may be associated with a virus or bacteria, such as a human pathogen. In some embodiments, the genotype is not associated with a genetic disease, *e.g.*, when the control is for use in assessing the sensitivity of a diagnostic test. The genotype may be a single nucleotide polymorphism, point mutation, premature stop codon, trinucleotide repeat, translocation, somatic rearrangement, allelomorph, single nucleotide variant, coding insertion, genetic alteration, or deletion ("indel"), splice variant, regulatory variant, copy number variant, or gene fusion. The control may be for use in identifying or characterizing a disease or condition.

The nucleic acids may comprise nucleotide sequences of any origin, such as viral, bacterial, protist, fungal, plant, or animal origin. In certain embodiments, the nucleic acids comprise human nucleotide sequences. The nucleic acids may also comprise nucleotide sequences from human pathogens, e.g., the nucleic acids may comprise viral, bacterial, protist, or fungal nucleotide sequences, wherein the virus, bacterium, protist, or fungus is a human pathogen.

In certain embodiments, the controls are substantially free of chromatin. For example, the controls may comprise nucleic acids encoding human nucleotide sequences, wherein the nucleic acids are not associated with histones and/or nucleosomes. In certain embodiments, the controls are substantially free of histones and/or nucleosomes.

The controls may comprise DNA and/or RNA. In some embodiments, the controls are substantially free of RNA.

In some embodiments, the control comprises a first mixture of nucleic acids. In some embodiments, the control comprises a first mixture of nucleic acids and a second mixture of nucleic acids.

*A first mixture of nucleic acids*

As described herein, the first mixture of nucleic acids may comprise a first genotype (a genotype of interest), such as aneuploidy, a genotype associated with a hereditary disease, a genotype associated with a communicable disease (*e.g.*, a virus, provirus, or bacteria), and/or a genotype associated with a neoplasm (*e.g.*, cancer). In other embodiments, the first genotype is not associated with disease.

The first mixture of nucleic acid may comprise a nucleotide sequence that encodes the genotype. The first mixture of nucleic acid may comprise a nucleotide sequence that encodes a genotype. For example, the first mixture of nucleic acids may comprise a nucleotide sequence that has sequence homology with a chromosome, *e.g.*, for use in detecting aneuploidy of the chromosome. In some embodiments, the first mixture of nucleic acids comprises a nucleotide sequence that encodes a gene comprising a premature stop codon, polymorphism, or trinucleotide repeat, *e.g.*, for use in detecting a hereditary disease. In some embodiments, the first mixture of nucleic acids comprises a nucleotide sequence that encodes a bacterial, viral, or protist nucleotide sequence, *e.g.*, for use in detecting a communicable disease. In some embodiments, the first mixture of nucleic acids comprises a nucleotide sequence that encodes a genetic mutation or a genetic rearrangement associated with a neoplasm, *e.g.*, for use in detecting cancer, such as metastatic cancer. In some embodiments, the genetic mutation is a genetic alteration.

The first mixture of nucleic acids may comprise one or more pluralities of nucleotide sequences, which may encode one or more genotypes, *e.g.*, one plurality of nucleotide sequences may encode one or more genotypes.

In some embodiments, the first mixture of nucleic acids comprises nucleotide sequences encoding substantially all of the genome of a cell, plurality of cells, cell line, or subject. For example, the cell line may be an immortalized lymphocyte cell line genome, a fibroblast cell line genome, or a cytotrophoblast cell line genome. In certain embodiments, the first mixture of nucleic acids comprises nucleotide sequences encoding substantially all of the genome of a human cell, human cell line, or human subject. The first mixture of nucleic acids may be obtained from a cell, plurality of cells, cell line, or donor, *e.g.*, a cell, plurality of cells, cell line, or donor that carries an aneuploidy, a fetus or fetuses, a fetus or fetuses with an aneuploidy, hereditary disease, provirus, and/or cancer mutation. The first mixture of nucleic acids need not comprise nucleotide sequences that encode an entire genome, however. For example, a mixture of nucleic acids derived from a cell may encode substantially all of the genome of the cell even though some nucleotide sequences may have been lost during processing steps, such as during isolation and/or fragmentation steps. Similarly, the first mixture of nucleic acids may be enriched or depleted of various nucleotide sequences, *e.g.*, for use in testing the robustness of an assay or diagnostic test. Alternatively, the first mixture of nucleic acids may originate from one or more non-human sources, such as a host cell comprising one or more nucleotide sequences sufficient to calibrate an assay or diagnostic test or to assess its performance. In some embodiments, the first mixture of nucleic acids encodes substantially all of the genome of a cell, cell line, or subject, *e.g.*, a human cell, plurality of human cells, human cell line, or human subject. In other embodiments, the first mixture of nucleic acids does not encode the genome of a cell, cell line, or subject. The first mixture of nucleic acids may also comprise nucleotide sequences from human pathogens, *e.g.*, the first mixture of nucleic acids may comprise viral, bacterial, protist, or fungal nucleotide sequences, wherein the virus, bacterium, protist, or fungus is a human pathogen.

In some embodiments, the first mixture of nucleic acids is obtained from a human donor, e.g., from cells or a bodily fluid of the human donor. The first mixture of nucleic acids may be obtained from peripheral blood mononuclear cells (PBMCs), lymphocytes, fibroblasts, placenta, and/or adipocytes of a human donor. In certain preferred embodiments, the first mixture of nucleic acids is obtained from PBMCs. The first mixture of nucleic acids may be obtained from the placenta of a human donor. The first mixture of nucleic acids may comprise cell free DNA obtained from a donor (*e.g.*, human donor). The cell free DNA may be obtained from blood plasma or blood serum. The cell free DNA may be obtained from urine. In certain embodiments, the human donor may be male or female. In certain embodiments, the donor is female. In certain embodiments, the donor is a pregnant female. In pregnant females, some of the cell free DNA may be derived from one or more fetuses and/or associated tissues such as placenta.

The first mixture of nucleic acids may be substantially free of chromatin, nucleosomes, and/or histones, *e.g.*, the first mixture of nucleic acids may comprise human nucleotide sequences that are substantially free of chromatin, nucleosomes, and histones. The first mixture of nucleic acids may be free of chromatin, nucleosomes, and/or histones. In some embodiments, the first mixture of nucleic acids comprises chromatin, nucleosomes, and/or histones. The first mixture of nucleic acids may comprise methylated nucleic acids or the first mixture of nucleic acids may be substantially free of methylated nucleic acids.

The first mixture of nucleic acids may comprise mitochondrial nucleotide sequences, or the first mixture of nucleic acids may be substantially free of mitochondrial nucleotide sequences.

The first mixture of nucleic acids may comprise DNA and/or RNA. In some embodiments, the first mixture of nucleic acids is substantially free of RNA.

A first nucleotide sequence of the first mixture of nucleic acids may encode a genotype of interest, such as a chromosome associated with aneuploidy, a genotype associated with a hereditary disease, a genotype associated with a communicable disease, and/or a genotype associated with a neoplasm. A second nucleotide sequence may have sequence homology to a different nucleotide sequence than the first nucleotide sequence. For example, the first nucleotide sequence may have sequence homology with a first chromosome, the second nucleotide sequence may have sequence homology with a second chromosome, and the ratio of the copy number of the first nucleotide sequence to the copy number of the second nucleotide sequence in the first mixture may be about 3:2, *e.g.*, for use with diagnostic tests that aims to determine whether the first chromosome is present in a sample as a trisomy. Thus, the first nucleotide sequence may have sequence homology to any one of chromosomes 8, 9, 13, 18, 21, 22, or X, of which trisomy may result in a viable fetus, and the second nucleotide sequence may have sequence homology with a different chromosome, *e.g.*, a different chromosome that is an autosome, such as chromosome 1, 6, or 7, which are commonly used as reference chromosomes. Nevertheless, even though other trisomic chromosomes are not known to result in viable offspring, the first nucleotide sequence may have sequence homology to any one of chromosomes 1, 2, 3, 4, 5, 6, 7, 8, 9, 10, 11, 12, 13, 14, 15, 16, 17, 18, 19, 20, 21, 22, X, or Y, *e.g.*, in order to calibrate a diagnostic test or to screen for a trisomy in a fetus before the trisomy displays a lethal phenotype.

The ratio of the copy number of the first nucleotide sequence to the copy number of the second nucleotide sequence may vary from about 3:2, *e.g.*, for diagnosing an aneuploidy other than a trisomy or for calibrating a diagnostic test or assay. Thus, in some embodiments, the ratio of the copy number of the first nucleotide sequence to the copy number of the second nucleotide sequence may be about 1:1 or greater than 1:1, such as greater than about 11:10, greater than about 10:9, greater than about 9:8, greater than about 8:7, greater than about 7:6, greater than about 6:5, greater than about 5:4, greater than about 4:3, greater than about 3:2, or greater than about 2:1. For example, in some embodiments, the first nucleotide sequence may have sequence homology to chromosome Y, the second nucleotide sequence may have sequence homology with an autosome, and the ratio of the copy number of the first nucleotide sequence to the copy number of the second nucleotide sequence may be about 1:1. In some embodiments, the ratio of the copy number of the first nucleotide sequence to the copy number of the second nucleotide sequence is about 1:1 to about 2:1, such as about 11:10 to about 2:1, about 10:9 to about 2:1, about 9:8 to about 2:1, about 8:7 to about 2:1, about 7:6 to about 2:1, about 6:5 to about 2:1, about 5:4 to about 2:1, or about 4:3 to about 2:1. In some embodiments, the ratio of the copy number of the first nucleotide sequence to the copy number of the second nucleotide sequence is about 3:2.

The first mixture of nucleic acids may comprise a third nucleotide sequence, *e.g.*, for use in determining whether a fetus has Klinefelter syndrome. In this embodiment, the first nucleotide sequence may have sequence homology with human chromosome X; a second nucleotide sequence may have sequence homology with an autosome; a third nucleotide sequence may have sequence homology with chromosome Y; and the ratio of the copy numbers of the first, second, and third nucleotide sequences may be about 2:2:1.

In some embodiments, the first mixture of nucleic acids comprises a first plurality of nucleotide sequences and a second plurality of nucleotide sequences. The first plurality of nucleotide sequences may each have sequence homology with a genotype of interest, such as a chromosome associated with aneuploidy, a genotype associated with a hereditary disease, a genotype associated with a communicable disease, and/or a genotype associated with a neoplasm. The second plurality of nucleotide sequences may each have sequence homology to nucleotide sequences that are different from than the first plurality of nucleotide sequences. For example, the first plurality of nucleotide sequences may each have sequence homology with a first chromosome, the second plurality of nucleotide sequences may each have sequence homology with a second chromosome, and the ratio of the copy number of any nucleotide sequence of the first plurality to the copy number of any nucleotide sequence of the second plurality in the first mixture may be about 3:2, *e.g.*, for use with diagnostic tests that aims to determine whether the first chromosome is present in a sample as a trisomy. Thus, each nucleotide sequence of the first plurality may have sequence homology to any one of chromosomes 8, 9, 13, 18, 21, 22, or X, of which trisomy may result in a viable fetus, and each nucleotide sequence of the second plurality may have sequence homology with a different chromosome, *e.g.*, a different chromosome that is an autosome. Nevertheless, even though other trisomic chromosomes are not known to result in viable offspring, the nucleotide sequences of the first plurality may have sequence homology to any one of chromosomes 1, 2, 3, 4, 5, 6, 7, 8, 9, 10, 11, 12, 13, 14, 15, 16, 17, 18, 19, 20, 21, 22, X, or Y, *e.g.*, in order to calibrate a diagnostic test or to screen for a trisomy in a fetus before the trisomy displays a lethal phenotype.

The ratio of the copy number of any nucleotide sequence of the first plurality to the copy number of any nucleotide sequence of the second plurality may vary from about 3:2, *e.g.*, for diagnosing an aneuploidy other than a trisomy or for calibrating a diagnostic test or assay. Thus, in some embodiments, the ratio of the copy number of any nucleotide sequence of the first plurality to the copy number of any nucleotide sequence of the second plurality may be about 1:1 or greater than 1:1, such as greater than about 11:10, greater than about 10:9, greater than about 9:8, greater than about 8:7, greater than about 7:6, greater than about 6:5, greater than about 5:4, greater than about 4:3, greater than about 3:2, or greater than about 2:1. For example, in some embodiments, each nucleotide sequence of the first plurality may have sequence homology to chromosome Y, each nucleotide sequence of the second plurality may have sequence homology with an autosome, and the ratio of the copy number of any nucleotide sequence of the first plurality to the copy number of any nucleotide sequence of the second plurality may be about 1:1. In some embodiments, the ratio of the copy number of any nucleotide sequence of the first plurality to the copy number of any nucleotide sequence of the second plurality may be about 1:1 to about 2:1, such as about 11:10 to about 2:1, about 10:9 to about 2:1, about 9:8 to about 2:1, about 8:7 to about 2:1, about 7:6 to about 2:1, about 6:5 to about 2:1, about 5:4 to about 2:1, or about 4:3 to about 2:1. In some embodiments, the ratio of the copy number of any nucleotide sequence of the first plurality to the copy number of any nucleotide sequence of the second plurality is about 3:2.

The first mixture of nucleic acids may comprise nucleotide sequences that have sequence homology with the first chromosome that are not included in the first plurality of nucleotide sequences. Similarly, the first mixture of nucleic acids may comprise nucleotide sequences that have sequence homology with the second chromosome that are not included in the second plurality of nucleotide sequences.

The first mixture of nucleic acids may comprise a third plurality of nucleotide sequences, e.g., for use in determining whether a fetus has Klinefelter syndrome. In this embodiment, each nucleotide sequence of the first plurality may have sequence homology with human chromosome X; each nucleotide sequence of the second plurality may have sequence homology with an autosome; each nucleotide sequence of the third plurality may have sequence homology with chromosome Y; and the ratio of the copy numbers of any three nucleotide sequences selected from the first, second, and third pluralities may be about 2:2:1.

The first mixture of nucleic acids may comprise a first plurality of nucleotide sequences, a second plurality of nucleotide sequences, a third plurality of nucleotide sequences, and a fourth plurality of nucleotide sequences. Each nucleotide sequence of the first plurality of nucleotide sequences may have sequence homology to chromosome 13, each nucleotide sequence of the second plurality of nucleotide sequences may have sequence homology to chromosome 18, and each nucleotide sequence of the third plurality of nucleotide sequences may have sequence homology to chromosome 21. Each nucleotide sequence of the fourth plurality of nucleotide sequences may have sequence homology to chromosome 1, 2, 3, 4, 5, 6, 7, 8, 9, 10, 11, 12, 14, 15, 16, 17, 19, 20, or 22, preferably chromosome 1, 6, or 7. The ratio of the copy numbers of any nucleotide sequence selected from the first, second, and third plurality to any nucleotide sequence selected from the fourth plurality may be about 7:6.

The first mixture of nucleic acids may comprise a nucleotide sequence that encodes a genotype listed in the catalogue of somatic mutations in cancer ("COSMIC") database (see http://cancer.sanger.ac.uk/cosmic ), and/or the first mixture of nucleic acids may comprise a nucleotide sequence that comprises a wild type genotype corresponding to any one of the genotypes listed in the COSMIC database. The first mixture of nucleic acids may comprise a plurality of nucleotide sequences, wherein each nucleotide sequence of the plurality encodes a genotype listed in the COSMIC database. For example, the first mixture of nucleic acids may comprise a plurality of nucleotide sequences, wherein the plurality of nucleotide sequences encodes 1, 2, 3, 4, 5, 6, 7, 8, 9, 10, 11, 12, 13, 14, 15, 16, 17, 18, 19, 20, 1, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 200, 300, 400, 500, 600, 700, 800, 900, or 1000 genotypes listed in the COSMIC database (e.g., a plurality of genotypes listed in the COSMIC database). The first mixture of nucleic acids may comprise a plurality of nucleotide sequences, wherein the plurality of nucleotide sequences encodes at least 1, 2, 3, 4, 5, 6, 7, 8, 9, 10, 11, 12, 13, 14, 15, 16, 17, 18, 19, 20, 1, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 200, 300, 400, 500, 600, 700, 800, 900, or 1000 genotypes listed in the COSMIC database.

Sixty-six mutations (i.e., genotypes) listed in the COSMIC database are shown in Table 1. In some embodiments, the first mixture of nucleic acids comprises a nucleotide sequence encoding a genotype listed in Table 1. The first mixture of nucleic acids may comprise a plurality of nucleotide sequences, wherein each nucleotide sequence of the plurality encodes a genotype listed in the Table 1. For example, the first mixture of nucleic acids may comprise a plurality of nucleotide sequences, wherein the plurality of nucleotide sequences encodes 1, 2, 3, 4, 5, 6, 7, 8, 9, 10, 11, 12, 13, 14, 15, 16, 17, 18, 19, 20, 1, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, or 66 genotypes listed in Table 1. In some embodiments, the first mixture of nucleic acids comprises a nucleotide sequence encoding a portion of a gene comprising a mutation, wherein the gene is selected from MTOR, MPL, NRAS, PARP1, AKT3, DNMT3A, MSH2, IDH1, VHL, MLH1, MYD88, CTNNB1, ATR, PIK3CA, FGFR3, PDGFRA, KIT, FBXW7, APC, GABRG2, NPM1, EGFR, MET, BRAF, EZH2, JAK2, GNAQ, RET, PTEN, ATM, KRAS, PTPN11, FLT3, RB1, PARP2, ARHGAP5, AKT1, RAD51, IDH2, TP53, NF1, SMAD4, AKT2, ERCC1, and GNAS. In some embodiments, the first mixture of nucleic acids comprises a plurality of nucleotide sequences, wherein each nucleotide sequence of the plurality encodes a portion of a gene comprising a mutation, and the genes are selected from MTOR, MPL, NRAS, PARP1, AKT3, DNMT3A, MSH2, IDH1, VHL, MLH1, MYD88, CTNNB1, ATR, PIK3CA, FGFR3, PDGFRA, KIT, FBXW7, APC, GABRG2, NPM1, EGFR, MET, BRAF, EZH2, JAK2, GNAQ, RET, PTEN, ATM, KRAS, PTPN11, FLT3, RB1, PARP2, ARHGAP5, AKT1, RAD51, IDH2, TP53, NF1, SMAD4, AKT2, ERCC1, and GNAS. In some embodiments, the first mixture of nucleic acids comprises a plurality of nucleotide sequences, wherein each nucleotide sequence of the plurality encodes a portion of a gene comprising a mutation, the nucleotide sequences of the plurality encode portions of 1, 2, 3, 4, 5, 6, 7, 8, 9, 10, 11, 12, 13, 14, 15, 16, 17, 18, 19, 20, 1, 22, 23, 24, 25, 26, 27, 28, 29, 30, 31, 32, 33, 34, 35, 36, 37, 38, 39, 40, 41, 42, 43, or 44 different genes, and the genes are selected from MTOR, MPL, NRAS, PARP1, AKT3, DNMT3A, MSH2, IDH1, VHL, MLH1, MYD88, CTNNB1, ATR, PIK3CA, FGFR3, PDGFRA, KIT, FBXW7, APC, GABRG2, NPM1, EGFR, MET, BRAF, EZH2, JAK2, GNAQ, RET, PTEN, ATM, KRAS, PTPN11, FLT3, RB1, PARP2, ARHGAP5, AKT1, RAD51, IDH2, TP53, NF1, SMAD4, AKT2, ERCC1, and GNAS.

**Table 1**. Selected somatic mutations listed in the COSMIC database.

| Gene | Position | REF | ALT | Strand | CDS | AA | COSID |
|------|----------|-----|-----|--------|-----|-----|-------|
| MTOR | 11291097 | T | A | - | 2664 A>T | L888F | COSM94356 |
| MPL | 43815009 | G | T | + | 1544 G>T | W515L | COSM18918 |
| NRAS | 115256529 | T | C | - | 182 A>G | Q61R | COSM584 |
| PARP1 | 226551691 | TC | T | - | 2738 del G | G913fs*4 | COSM21691 |
| AKT3 | 243809253 | T | A | - | 371 A>T | Q124L | COSM48227 |
| DNMT3A | 25457243 | G | A | - | 2644 C>T | R882C | COSM53042 |
| MSH2 | 47705449 | TG | T | + | 2250 del G | G751fs*12 | COSM111644 |
| MSH2 | 47705558 | ACT | A | + | 2359_2360 del CT | L787fs*11 | COSM26122 |
| IDH1 | 209113113 | G | A | - | 394C>T | R132C | COSM28747 |
| VHL | 10188282 | TTGAC | T | + | 426_429 del TGAC | G144fs*14 | COSM18578 |
| MLH1 | 37067240 | T | A | + | 1151 T>A | V384D | COSM26085 |
| MYD88 | 38182641 | T | C | + | 794 T>C | L265P | COSM85940 |
| CTNNB1 | 41266124 | A | G | + | 121 A>G | T41A | COSM5664 |
| ATR | 142254972 | GCTTTTAT | G | - | 3790_3796 del ATAAAAG | I1264fs*24 | COSM20627 |
| PIK3CA | 178936091 | G | A | + | 1633 G>A | E545K | COSM763 |
| PIK3CA | 178952085 | A | G | + | 3140 A>G | H1047R | COSM775 |
| PIK3CA | 178952149 | C | CA | + | 3204_3205 ins A | N1068fs*4 | COSM12464 |
| FGFR3 | 1803568 | C | G | + | 746 C>G | S249C | COSM715 |
| PDGFRA | 55141048 | T | TA | + | 1694_1695 ins A | S566fs*6 | COSM28053 |
| PDGFRA | 55152093 | A | T | + | 2525 A>T | D842V | COSM736 |
| KIT | 55599321 | A | T | + | 2447 A>T | D816V | COSM1314 |
| FBXW7 | 153249384 | C | T | - | 1394 G>A | R465H | COSM22965 |
| APC | 112175538 | GC | G | + | 4248 del C | I1417fs*2 | COSM18584 |
| APC | 112175639 | C | T | + | 4348 C>T | R1450* | COSM13127 |

(continued)

| Gene | Position | REF | ALT | Strand | CDS | AA | COSID |
|---|---|---|---|---|---|---|---|
| APC | 112175957 | A | AA | + | 4666_4667 ins A | T1556fs*3 | COSM18561 |
| GABRA6 | 161117296 | G | C | + | 763 G>C | V255L | COSM70853 |
| GABRG2 | 161580301 | A | G | + | 1355 A>G | Y452C | COSM74722 |
| NPM1 | 170837547 | G | GTCTG | + | 863_864 ins TCTG | W288fs*12 | COSM17559 |
| EGFR | 55242465 | GGAATTAAG AGAAGCA | G | + | 2236_2250 del 15 | E746_A750 del ELREA | COSM6225 |
| EGFR | 55249012 | C | CGGT | + | 2310_2311 ins GGT | D770_N771 ins G | COSM12378 |
| EGFR | 55249071 | C | T | + | 2369 C>T | T790M | COSM6240 |
| EGFR | 55259515 | T | G | + | 2573 T>G | L858R | COSM6224 |
| MET | 116423428 | T | G | + | 3757 T>G | Y1253D | COSM700 |
| BRAF | 140453136 | A | T | - | 1799 T>A | V600E | COSM476 |
| EZH2 | 148508727 | T | A | - | 1937 A>T | Y646F | COSM37028 |
| JAK2 | 5073770 | G | T | + | 1849 G>T | V617F | COSM12600 |
| GNAQ | 80409488 | T | G | - | 626 A>C | Q209P | COSM28758 |
| RET | 43617416 | T | C | + | 2753 T>C | M918T | COSM965 |
| PTEN | 89692904 | C | T | + | 388 C>T | R130* | COSM5152 |
| PTEN | 89717716 | A | AA | + | 741_742 ins A | P248fs*5 | COSM4986 |
| PTEN | 89717774 | AA | A | + | 800 del A | K267fs*9 | COSM5809 |
| ATM | 108117846 | TGT | T | + | 1058_1059 del GT | C353fs*5 | COSM21924 |
| ATM | 108175462 | G | A | + | 5557 G>A | D1853N | COSM41596 |
| KRAS | 25398284 | C | T | - | 35 G>A | G12D | COSM521 |
| PTPN11 | 112888210 | G | A | + | 226 G>A | E76K | COSM13000 |
| FLT3 | 28592642 | C | A | - | 2503 G>T | D835Y | COSM783 |
| RB1 | 48941648 | C | T | + | 958 C>T | R320* | COSM891 |
| PARP2 | 20820412 | A | C | + | 398 A>C | D133A | COSM75849 |
| ARHGAP5 | 32561739 | G | A | + | 1864 G>A | E622K | COSM88502 |
| AKT1 | 105246455 | C | T | - | 145 G>A | E49K | COSM36918 |
| AKT1 | 105246551 | C | T | - | 49 G>A | E17K | COSM33765 |
| RAD51 | 41001312 | C | T | + | 433 C>T | Q145* | COSM117943 |
| IDH2 | 90631838 | C | T | - | 515 G>A | R172K | COSM33733 |
| IDH2 | 90631934 | C | T | - | 419 G>A | R140Q | COSM41590 |
| TP53 | 7577120 | C | T | - | 818 G>A | R273H | COSM10660 |
| TP53 | 7577538 | C | T | - | 743 G>A | R248Q | COSM10662 |
| TP53 | 7577557 | AG | A | - | 723 del C | C242fs*5 | COSM6530 |

(continued)

| Gene | Position | REF | ALT | Strand | CDS | AA | COSID |
|------|----------|-----|-----|--------|-----|-----|-------|
| TP53 | 7578406 | C | T | - | 524 G>A | R175H | COSM10648 |
| TP53 | 7579423 | GG | G | - | 263 del C | S90fs*33 | COSM18610 |
| NF1 | 29556989 | T | TAC | + | 2987_2988 ins AC | R997fs*16 | COSM41820 |
| NF1 | 29576111 | C | T | + | 4084 C>T | R1362* | COSM24443 |
| NF1 | 29679317 | TG | T | + | 7501 del G | E2501fs*22 | COSM24468 |
| SMAD4 | 48603093 | T | TT | + | 1394_1395 ins T | A466fs*28 | COSM14105 |
| AKT2 | 40761084 | C | A | - | 268 G>T | V90L | COSM93894 |
| ERCC1 | 45924470 | G | T | - | 287 C>A | A96E | COSM140843 |
| GNAS | 57484420 | C | T | + | 601 C>T | R201C | COSM27887 |

In some embodiments, the genotype is a mutation to a gene selected from the group consisting of MTOR, MPL, NRAS, PARP1, AKT3, DNMT3A, MSH2, IDH1, VHL, MLH1, MYD88, CTNNB1, ATR, PIK3CA, FGFR3, PDGFRA, KIT, FBXW7, APC, GABRG2, NPM1, EGFR, MET, BRAF, EZH2, JAK2, GNAQ, RET, PTEN, ATM, KRAS, PTPN11, FLT3, RB1, PARP2, ARHGAP5, AKT1, RAD51, IDH2, TP53, NF1, SMAD4, AKT2, ERCC1, and GNAS. In some embodiments, each genotype of the plurality of genotypes consists of a mutation to a gene selected from the group consisting of MTOR, MPL, NRAS, PARP1, AKT3, DNMT3A, MSH2, IDH1, VHL, MLH1, MYD88, CTNNB1, ATR, PIK3CA, FGFR3, PDGFRA, KIT, FBXW7, APC, GABRG2, NPM1, EGFR, MET, BRAF, EZH2, JAK2, GNAQ, RET, PTEN, ATM, KRAS, PTPN11, FLT3, RB1, PARP2, ARHGAP5, AKT1, RAD51, IDH2, TP53, NF1, SMAD4, AKT2, ERCC1, and GNAS.

In addition to the COSMIC database, specific mutations have been identified as somatic mutations that frequently occur in various cancers. For example, Boland *et al.* identified 26 different genes that are frequently mutated in various cancer types (*see* Boland, G. M., et al. Oncotarget, 5 (2015)). Accordingly, in some embodiments, the first mixture of nucleic acids comprises a nucleotide sequence encoding a portion of a gene comprising a mutation, wherein the gene is selected from AKT1, ATM, BRAF, CDKN2A, CSF1R, EGFR, ERBB2 ("HER2"), ERBB4 ("HER4"), FGFR1, FGFR2, FGFR3, GNA11, HRAS, JAK2, JAK3, KDR, KIT, KRAS, MET, NOTCH1, NRAS, PDGFRA, PIK3CA, PTEN, RET, and STK11. In some embodiments, the first mixture of nucleic acids comprises a plurality of nucleotide sequences, wherein each nucleotide sequence of the plurality encodes a portion of a gene comprising a mutation, and the genes are selected from AKT1, ATM, BRAF, CDKN2A, CSF1R, EGFR, ERBB2 ("HER2"), ERBB4 ("HER4"), FGFR1, FGFR2, FGFR3, GNA11, HRAS, JAK2, JAK3, KDR, KIT, KRAS, MET, NOTCH1, NRAS, PDGFRA, PIK3CA, PTEN, RET, and STK11. In some embodiments, the first mixture of nucleic acids comprises a plurality of nucleotide sequences, wherein each nucleotide sequence of the plurality encodes a portion of a gene comprising a mutation, the nucleotide sequences of the plurality encode portions of 1, 2, 3, 4, 5, 6, 7, 8, 9, 10, 11, 12, 13, 14, 15, 16, 17, 18, 19, 20, 1, 22, 23, 24, 25, or 26 different genes, and the genes are selected from AKT1, ATM, BRAF, CDKN2A, CSF1R, EGFR, ERBB2 ("HER2"), ERBB4 ("HER4"), FGFR1, FGFR2, FGFR3, GNA11, HRAS, JAK2, JAK3, KDR, KIT, KRAS, MET, NOTCH1, NRAS, PDGFRA, PIK3CA, PTEN, RET, and STK11.

In some embodiments, the genotype is a mutation to a gene selected from the group consisting of AKT1, ATM, BRAF, CDKN2A, CSF1R, EGFR, ERBB2 ("HER2"), ERBB4 ("HER4"), FGFR1, FGFR2, FGFR3, GNA11, HRAS, JAK2, JAK3, KDR, KIT, KRAS, MET, NOTCH1, NRAS, PDGFRA, PIK3CA, PTEN, RET, and STK11. In some embodiments, each genotype of the plurality of genotypes consists of a mutation to a gene selected from the group consisting of AKT1, ATM, BRAF, CDKN2A, CSF1R, EGFR, ERBB2 ("HER2"), ERBB4 ("HER4"), FGFR1, FGFR2, FGFR3, GNA11, HRAS, JAK2, JAK3, KDR, KIT, KRAS, MET, NOTCH1, NRAS, PDGFRA, PIK3CA, PTEN, RET, and STK11.

In some embodiments, the first mixture of nucleic acids comprises a nucleotide sequence encoding a portion of a gene comprising a mutation, wherein the gene is selected from ABL1, AKT1, ALK, APC, AR, AR1D1A, ARAF, ATM, BCL2, BCR, BRAF, BRC42, BRCA1, BRCA2, BRIP1, CCND1, CCND2, CCNE1, CDH1, CDK4, CDK6, CDKN2A, CDKN2B, CSF1R, CTNNB1, DDR2, EGFR, ERBB2, ERBB3, ERBB4, ESR1, ETV1, ETV4, ETV6, EWSR1, EZH2, FANCA, FANCC, FANCD2, FANCE, FANCF, FANCG, FANCL, FBXW7, FGFR1, FGFR2, FGFR3, FLT3, FOXL2, GATA3, GNA11, GNAQ, GNAS, HER/ERBB2, HNF1A, HPAS, HRAS, IDH1, IDH2, IHD2, JAK2, JAK3, KDR, KIT, KRAS, MAP2K1, MAP2K2, MET, MLH1, MLL, MPL, MSH2, MSH6, MTOR, MYC, MYCN, NF1, NF2, NFE2L2, NOTCH1, NPM1, NRAS, NTRK1, PALB2, PDGFRA, PDGFRB, PIK3CA, PMS2, PTCH1, PTEN, PTPN11, RAFT, RARA, RB1, RET, RHEB, RHOA, RIT1, ROS1, SMAD4, SMARCB1, SMO, SRC, STK11, TERT, TMPRSS2, TP53, TSC1, TSC2, and VHL. In some embodiments, the first mixture of nucleic acids comprises a plurality of nucleotide sequences, wherein each nucleotide sequence of the plurality encodes a portion of a gene comprising a mutation, and the genes are selected from ABL1, AKT1, ALK, APC, AR, AR1D1A, ARAF, ATM, BCL2, BCR, BRAF, BRC42, BRCA1, BRCA2, BRIP1, CCND1, CCND2, CCNE1, CDH1, CDK4, CDK6, CDKN2A, CDKN2B, CSF1R, CTNNB1, DDR2, EGFR, ERBB2, ERBB3, ERBB4, ESR1, ETV1, ETV4, ETV6, EWSR1, EZH2, FANCA, FANCC, FANCD2, FANCE, FANCF, FANCG, FANCL, FBXW7, FGFR1, FGFR2, FGFR3, FLT3, FOXL2, GATA3, GNA11, GNAQ, GNAS, HER/ERBB2, HNF1A, HPAS, HRAS, IDH1, IDH2, IHD2, JAK2, JAK3, KDR, KIT, KRAS, MAP2K1, MAP2K2, MET, MLH1, MLL, MPL, MSH2, MSH6, MTOR, MYC, MYCN, NF1, NF2, NFE2L2, NOTCH1, NPM1, NRAS, NTRK1, PALB2, PDGFRA, PDGFRB, PIK3CA, PMS2, PTCH1, PTEN, PTPN11, RAFT, RARA, RB1, RET, RHEB, RHOA, RIT1, ROS1, SMAD4, SMARCB1, SMO, SRC, STK11, TERT, TMPRSS2, TP53, TSC1, TSC2, and VHL. In some embodiments, the first mixture of nucleic acids comprises a plurality of nucleotide sequences, wherein each nucleotide sequence of the plurality encodes a portion of a gene comprising a mutation, the nucleotide sequences of the plurality encode portions of 1, 2, 3, 4, 5, 6, 7, 8, 9, 10, 11, 12, 13, 14, 15, 16, 17, 18, 19, 20, 1, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 different genes, and the genes are selected from ABL1, AKT1, ALK, APC, AR, AR1D1A, ARAF, ATM, BCL2, BCR, BRAF, BRC42, BRCA1, BRCA2, BRIP1, CCND1, CCND2, CCNE1, CDH1, CDK4, CDK6, CDKN2A, CDKN2B, CSF1R, CTNNB1, DDR2, EGFR, ERBB2, ERBB3, ERBB4, ESR1, ETV1, ETV4, ETV6, EWSR1, EZH2, FANCA, FANCC, FANCD2, FANCE, FANCF, FANCG, FANCL, FBXW7, FGFR1, FGFR2, FGFR3, FLT3, FOXL2, GATA3, GNA11, GNAQ, GNAS, HER/ERBB2, HNF1A, HPAS, HRAS, IDH1, IDH2, IHD2, JAK2, JAK3, KDR, KIT, KRAS, MAP2K1, MAP2K2, MET, MLH1, MLL, MPL, MSH2, MSH6, MTOR, MYC, MYCN, NF1, NF2, NFE2L2, NOTCH1, NPM1, NRAS, NTRK1, PALB2, PDGFRA, PDGFRB, PIK3CA, PMS2, PTCH1, PTEN, PTPN11, RAFT, RARA, RB1, RET, RHEB, RHOA, RIT1, ROS1, SMAD4, SMARCB1, SMO, SRC, STK11, TERT, TMPRSS2, TP53, TSC1, TSC2, and VHL.

In some embodiments, the genotype is a mutation to a gene selected from the group consisting of ABL1, AKT1, ALK, APC, AR, AR1D1A, ARAF, ATM, BCL2, BCR, BRAF, BRC42, BRCA1, BRCA2, BRIP1, CCND1, CCND2, CCNE1, CDH1, CDK4, CDK6, CDKN2A, CDKN2B, CSF1R, CTNNB1, DDR2, EGFR, ERBB2, ERBB3, ERBB4, ESR1, ETV1, ETV4, ETV6, EWSR1, EZH2, FANCA, FANCC, FANCD2, FANCE, FANCF, FANCG, FANCL, FBXW7, FGFR1, FGFR2, FGFR3, FLT3, FOXL2, GATA3, GNA11, GNAQ, GNAS, HER/ERBB2, HNF1A, HPAS, HRAS, IDH1, IDH2, IHD2, JAK2, JAK3, KDR, KIT, KRAS, MAP2K1, MAP2K2, MET, MLH1, MLL, MPL, MSH2, MSH6, MTOR, MYC, MYCN, NF1, NF2, NFE2L2, NOTCH1, NPM1, NRAS, NTRK1, PALB2, PDGFRA, PDGFRB, PIK3CA, PMS2, PTCH1, PTEN, PTPN11, RAFT, RARA, RB1, RET, RHEB, RHOA, RIT1, ROS1, SMAD4, SMARCB1, SMO, SRC, STK11, TERT, TMPRSS2, TP53, TSC1, TSC2, and VHL. In some embodiments, each genotype of the plurality of genotypes consists of a mutation to a gene selected from the group consisting of ABL1, AKT1, ALK, APC, AR, AR1D1A, ARAF, ATM, BCL2, BCR, BRAF, BRC42, BRCA1, BRCA2, BRIP1, CCND1, CCND2, CCNE1, CDH1, CDK4, CDK6, CDKN2A, CDKN2B, CSF1R, CTNNB1, DDR2, EGFR, ERBB2, ERBB3, ERBB4, ESR1, ETV1, ETV4, ETV6, EWSR1, EZH2, FANCA, FANCC, FANCD2, FANCE, FANCF, FANCG, FANCL, FBXW7, FGFR1, FGFR2, FGFR3, FLT3, FOXL2, GATA3, GNA11, GNAQ, GNAS, HER/ERBB2, HNF1A, HPAS, HRAS, IDH1, IDH2, IHD2, JAK2, JAK3, KDR, KIT, KRAS, MAP2K1, MAP2K2, MET, MLH1, MLL, MPL, MSH2, MSH6, MTOR, MYC, MYCN, NF1, NF2, NFE2L2, NOTCH1, NPM1, NRAS, NTRK1, PALB2, PDGFRA, PDGFRB, PIK3CA, PMS2, PTCH1, PTEN, PTPN11, RAFT, RARA, RB1, RET, RHEB, RHOA, RIT1, ROS1, SMAD4, SMARCB1, SMO, SRC, STK11, TERT, TMPRSS2, TP53, TSC1, TSC2, and VHL.

In some embodiments, the first mixture of nucleic acids comprises a nucleotide sequence encoding a portion of a gene comprising a mutation, wherein the gene is BRAF and the mutation is V600E, the gene is EGFR and the mutation is T790M, the gene is EFGR and the mutation is delL747-P753insS, the gene is ERBB2 and the mutation is A775_G776insYVMA, or the gene is KRAS and the mutation is G12D. In some embodiments, the first mixture of nucleic acids comprises a plurality of nucleotide sequences, wherein each nucleotide sequence of the plurality encodes a portion of a gene comprising a mutation, and the genes are selected from BRAF, EGFR, ERBB2, and KRAS and the mutations are selected from V600E (BRAF), T790M (EGFR), delL747-P753insS, (EGFR), A775_G776insYVMA (ERBB2), and G12D (KRAS) . In some embodiments, the first mixture of nucleic acids comprises a plurality of nucleotide sequences, wherein each nucleotide sequence of the plurality encodes a portion of a gene comprising a mutation, the nucleotide sequences of the plurality encode portions of 1, 2, 3, or 4 different genes, and the genes are selected from BRAF, EGFR, ERBB2, and KRAS.

In some embodiments, the genotype is a mutation to a gene selected from the group consisting of BRAF, EGFR, ERBB2, and KRAS. In some embodiments, each genotype of the plurality of genotypes consists of a mutation to a gene selected from the group consisting of BRAF, EGFR, ERBB2, and KRAS.

*A second mixture of nucleic acids*

The second mixture of nucleic acids may encode a second genotype that is different from the first genotype. In certain embodiments, the second mixture of nucleic acids encodes "normal" genotypes (*i.e.*, genotypes that are not associated with disease) relative to the genotype(s) of interest. Thus, in some embodiments, the second mixture of nucleic acids does not encode an aneuploidy, a genotype associated with a hereditary disease, a genotype associated with a communicable disease, or a genotype associated with a neoplasm. Nevertheless, the second mixture of nucleic acids may encode an aneuploidy, a genotype associated with a hereditary disease, a genotype associated with a communicable disease, or a genotype associated with a neoplasm, so long as the genotype does not mask the genotype of interest associated with the first mixture of nucleic acids or otherwise confound the use of the control.

The second mixture of nucleic acids may comprise one or more pluralities of nucleotide sequences, which may encode one or more genotypes, *e.g.*, one plurality of nucleotide sequences may encode one or more genotypes.

In some embodiments, the second mixture of nucleic acids comprises nucleotide sequences encoding substantially all of the genome of a cell, plurality of cells, cell line, or subject. For example, the cell line may be an immortalized lymphocyte cell line genome, a fibroblast cell line genome, or a cytotrophoblast cell line genome. In certain embodiments, the second mixture of nucleic acids comprises nucleotide sequences encoding substantially all of the genome of a human cell, human cell line, or human subject. The second mixture of nucleic acids may be obtained from a cell, plurality of cells, cell line, or donor, *e.g.*, a cell, plurality of cells, cell line, or donor that does not carry an aneuploidy, hereditary disease, provirus, or cancer mutation. For example, the second mixture of nucleic acids may be obtained from a human donor, *e.g.*, from cells or bodily fluids of the human donor. The second mixture of nucleic acids may be obtained from peripheral blood mononuclear cells (PBMCs), lymphocytes, fibroblasts, placenta, and/or adipocytes of a human donor. In certain preferred embodiments, the second mixture of nucleic acids is obtained from PBMCs. The second mixture of nucleic acids may be obtained from the placenta of a human donor. The second mixture of nucleic acids may comprise cell free DNA obtained from a donor (*e.g.*, human donor). The cell free DNA may be obtained from blood plasma or blood serum. The cell free DNA may be obtained from urine. In certain embodiments, the human donor may be male or female. In certain embodiments, the donor is female.

The second mixture of nucleic acids need not comprise nucleotide sequences that encode an entire genome. For example, a mixture of nucleic acids derived from a cell may encode substantially all of the genome of the cell even though some nucleotide sequences may have been lost during processing steps, such as during isolation and/or fragmentation steps. Similarly, the second mixture of nucleic acids may be enriched or depleted of various nucleotide sequences, *e.g.*, for use in testing the robustness of an assay or diagnostic test. Alternatively, the second mixture of nucleic acids may originate from one or more non-human sources, such as a host cell comprising one or more nucleotide sequences sufficient to calibrate an assay or diagnostic test or to assess its performance. In some embodiments, the second mixture of nucleic acids encodes substantially all of the genome of a cell, cell line, or subject, *e.g.*, a human cell, human cell line, or human subject. In other embodiments, the second mixture of nucleic acids does not encode the genome of a cell, cell line, or subject. The second mixture of nucleic acids may also comprise nucleotide sequences from human pathogens, *e.g.*, the second mixture of nucleic acids may comprise viral, bacterial, protist, or fungal nucleotide sequences, wherein the virus, bacterium, protist, or fungus is a human pathogen.

The second mixture of nucleic acids may be substantially free of chromatin, nucleosomes, and/or histones, *e.g.*, the second mixture of nucleic acids may comprise human nucleotide sequences that are substantially free of chromatin, nucleosomes, and histones. The second mixture of nucleic acids may be free of chromatin, nucleosomes, and/or histones. In some embodiments, the second mixture of nucleic acids comprises chromatin, nucleosomes, and/or histones. The second mixture of nucleic acids may comprise methylated nucleic acids or the second mixture of nucleic acids may be substantially free of methylated nucleic acids.

The second mixture of nucleic acids may comprise mitochondrial nucleotide sequences, or the second mixture of nucleic acids may be substantially free of mitochondrial nucleotide sequences.

The second mixture of nucleic acids may comprise DNA and/or RNA. In some embodiments, the second mixture of nucleic acids is substantially free of RNA.

In some embodiments, the second mixture of nucleic acids comprises a plurality of nucleotide sequences, *e.g.*, for embodiments in which the first mixture of nucleic acids comprises a plurality of nucleotide sequences. In certain embodiments, the second mixture of nucleic acids comprises a first nucleotide sequence that is related to the first nucleotide sequence of the first mixture of nucleic acids. For example, in embodiments in which the genotype of interest is aneuploidy, the first nucleotide sequence of the second mixture of nucleic acids may be identical to the first nucleotide sequence of the first mixture of nucleic acids. Similarly, in embodiments in which the genotype of interest is associated with a hereditary disease, the first nucleotide sequence of the second mixture of nucleic acids may encode a healthy or normal genotype, which is related to but varies from the first nucleotide sequence of the first mixture of nucleic acids, which encodes the disease genotype. Further, in embodiments in which the genotype of interest is associated with a neoplasm, the first nucleotide sequence of the second mixture of nucleic acids may encode a healthy or normal genotype, which is related to but varies from the first nucleotide sequence of the first mixture of nucleic acids, which may encode a disease genotype.

The second mixture of nucleic acids may comprise a second nucleotide sequence. In certain embodiments, the second nucleotide sequence is related to or identical to the second nucleotide sequence of the second mixture of nucleic acids. The second nucleotide sequence may have sequence homology to a different nucleotide sequence than the first nucleotide sequence. For example, the first nucleotide sequence may have sequence homology with a first chromosome, the second nucleotide sequence may have sequence homology with a second chromosome, and the ratio of the copy number of the first nucleotide sequence to the copy number of the second nucleotide sequence in the first mixture may be about 1:1, e.g., when the first mixture of nucleic acids comprises the first nucleotide sequence and the second nucleotide sequence in a different ratio for use as an aneuploidy control. Thus, the first nucleotide sequence may have sequence homology to any one of chromosomes 8, 9, 13, 18, 21, 22, or X, of which trisomy may result in a viable fetus, and the second nucleotide sequence may have sequence homology with a different chromosome, e.g., a different chromosome that is an autosome, such as chromosome 1, 6, or 7, which are commonly used as reference chromosomes. Nevertheless, even though other trisomic chromosomes are not known to result in viable offspring, the first nucleotide sequence may have sequence homology to any one of chromosomes 1, 2, 3, 4, 5, 6, 7, 8, 9, 10, 11, 12, 13, 14, 15, 16, 17, 18, 19, 20, 21, 22, X, or Y, e.g., in order to calibrate a diagnostic test or to screen for a trisomy in a fetus before the trisomy displays a lethal phenotype. Similarly, the ratio of the copy number of the first nucleotide sequence to the copy number of the second nucleotide sequence may vary from about 1:1, e.g., for use in determining the ploidy of a sex chromosome. For example, in some embodiments, the first nucleotide sequence may have sequence homology to chromosome Y, the second nucleotide sequence may have sequence homology with an autosome, and the ratio of the copy number of the first nucleotide sequence to the copy number of the second nucleotide sequence may be about 1:2.

The second mixture of nucleic acids may comprise a third nucleotide sequence, e.g., for use in determining whether a fetus has Klinefelter syndrome. In this embodiment, the first nucleotide sequence of the plurality may have sequence homology with human chromosome X; a second nucleotide sequence of the plurality may have sequence homology with an autosome; a third nucleotide sequence of the plurality may have sequence homology with chromosome Y; and the ratio of the copy numbers of the first, second, and third nucleotide sequences may be about 1:2:1, e.g., when the first mixture of nucleic acids comprises the first, second, and third nucleotide sequences in a ratio of about 2:2:1.

In some embodiments, the second mixture of nucleic acids comprises a first plurality of nucleotide sequences and a second plurality of nucleotide sequences, e.g., for embodiments in which the first mixture of nucleic acids comprises a first plurality of nucleotide sequences and a second plurality of nucleotide sequences. In certain embodiments, the first plurality of nucleotide sequences of the second mixture of nucleic acids is related to the first plurality of nucleotide sequences of the first mixture of nucleic acids. For example, in embodiments in which the genotype of interest is aneuploidy, the first plurality of nucleotide sequences of the second mixture may be identical to (or have sequence homology with) the first plurality of nucleotide sequences of the first mixture. Similarly, in embodiments in which the genotype of interest is associated with a hereditary disease, the first plurality of nucleotide sequences of the second mixture may comprise a nucleotide sequence that encodes a healthy or normal genotype, which is related to but varies from a nucleotide sequence of the first plurality of nucleotide sequences of the first mixture, which may encode a disease genotype from the same genetic locus as the nucleotide sequence of the second mixture. Further, in embodiments in which the genotype of interest is associated with a neoplasm, the first plurality of nucleotide sequences of the second mixture may comprise a nucleotide sequence that encodes a healthy or normal genotype, which is related to but varies from a nucleotide sequence of the first plurality of nucleotide sequences of the first mixture, which may encode a disease genotype from the same genetic locus as the nucleotide sequence of the second mixture.

In certain embodiments, the second plurality of nucleotide sequences of the second mixture of nucleic acids is related to or identical to the second plurality of nucleotide sequences of the first mixture of nucleic acids. The second plurality of nucleotide sequences of the second mixture may have sequence homology to different nucleotide sequences than the first plurality of nucleotide sequences of the second mixture. For example, the first plurality of nucleotide sequences may have sequence homology with a first chromosome, the second plurality of nucleotide sequences may have sequence homology with a second chromosome, and the ratio of the copy number of any nucleotide sequence of the first plurality to the copy number of any nucleotide sequence in the second plurality may be about 1:1, e.g., when the first mixture of nucleic acids comprises copy numbers for a first nucleotide sequence and second nucleotide sequence in a different ratio for use as an aneuploidy control. Thus, each nucleotide sequence of the first plurality may have sequence homology to any one of chromosomes 8, 9, 13, 18, 21, 22, or X, of which trisomy may result in a viable fetus, and each nucleotide sequence of the second plurality may have sequence homology with a different chromosome, e.g., a different chromosome that is an autosome. Nevertheless, even though other trisomic chromosomes are not known to result in viable offspring, each nucleotide sequence of the first plurality may have sequence homology to any one of chromosomes 1, 2, 3, 4, 5, 6, 7, 8, 9, 10, 11, 12, 13, 14, 15, 16, 17, 18, 19, 20, 21, 22, X, or Y, e.g., in order to calibrate a diagnostic test or to screen for a trisomy in a fetus before the trisomy displays a lethal phenotype. Similarly, the ratio of the copy number of any nucleotide sequence of the first plurality to the copy number of any nucleotide sequence of the second plurality may vary from about 1:1, *e.g.*, for use in determining the ploidy of a sex chromosome. For example, in some embodiments, each nucleotide sequence of the first plurality may have sequence homology to chromosome Y, each nucleotide sequence of the second plurality may have sequence homology with an autosome, and the ratio of the copy number of any nucleotide sequence of the first plurality to the copy number of any nucleotide sequence of the second plurality may be about 1:2.

The second mixture of nucleic acids may comprise nucleotide sequences that have sequence homology with the first chromosome that are not included in the first plurality of nucleotide sequences. Similarly, the second mixture of nucleic acids may comprise nucleotide sequences that have sequence homology with the second chromosome that are not included in the second plurality of nucleotide sequences.

The second mixture of nucleic acids may comprise a third plurality of nucleotide sequences, *e.g.*, for use in determining whether a fetus has Klinefelter syndrome. In this embodiment, each nucleotide sequence of the first plurality may have sequence homology with human chromosome X; each nucleotide sequence of the second plurality may have sequence homology with an autosome; each nucleotide sequence of the third plurality may have sequence homology with chromosome Y; and the ratio of the copy numbers of any three nucleotide sequences selected from the first, second, and third pluralities may be about 1:2:1.

The second mixture of nucleic acids may comprise a first plurality of nucleotide sequences, a second plurality of nucleotide sequences, a third plurality of nucleotide sequences, and a fourth plurality of nucleotide sequences, *e.g.*, when the first mixture of nucleic acids comprises a first plurality of nucleotide sequences, a second plurality of nucleotide sequences, a third plurality of nucleotide sequences, and a fourth plurality of nucleotide sequences. Each nucleotide sequence of the first plurality of nucleotide sequences may have sequence homology to chromosome 13, each nucleotide sequence of the second plurality of nucleotide sequences may have sequence homology to chromosome 18, and each nucleotide sequence of the third plurality of nucleotide sequences may have sequence homology to chromosome 21. Each nucleotide sequence of the fourth plurality of nucleotide sequences may have sequence homology to chromosome 1, 2, 3, 4, 5, 6, 7, 8, 9, 10, 11, 12, 14, 15, 16, 17, 19, 20, or 22, preferably chromosome 1, 6, or 7. The ratio of the copy number of any nucleotide sequence selected from the first, second, and third pluralities to the copy number of any nucleotide sequence selected from the fourth plurality may be about 1:1.

In some embodiments, the first mixture of nucleic acids comprises a nucleotide sequence that encodes a genotype listed in the COSMIC database, and the second mixture of nucleic acids comprises a nucleotide sequence that encodes a wild type genotype corresponding to the genotype listed in the COSMIC database. In some embodiments, the first mixture of nucleic acids comprises a first plurality of nucleotide sequences, wherein each nucleotide sequence of the first plurality encodes a genotype listed in the COSMIC database, and the second mixture of nucleic acids comprises a second plurality of nucleotide sequences encoding wild type genotypes corresponding to each genotype of the first plurality. For example, the first mixture of nucleic acids may comprise a first plurality of nucleotide sequences, wherein the first plurality of nucleotide sequences encodes 1, 2, 3, 4, 5, 6, 7, 8, 9, 10, 11, 12, 13, 14, 15, 16, 17, 18, 19, 20, 1, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 200, 300, 400, 500, 600, 700, 800, 900, or 1000 genotypes listed in the COSMIC database, and the second mixture of nucleic acids may comprise a second plurality of nucleotide sequences encoding wild type genotypes corresponding to each genotype in the first plurality. The first mixture of nucleic acids may comprise a first plurality of nucleotide sequences, wherein the first plurality of nucleotide sequences encodes at least 1, 2, 3, 4, 5, 6, 7, 8, 9, 10, 11, 12, 13, 14, 15, 16, 17, 18, 19, 20, 1, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 200, 300, 400, 500, 600, 700, 800, 900, or 1000 genotypes listed in the COSMIC database, and the second mixture of nucleic acids may comprise a second plurality of nucleotide sequences encoding wild type genotypes corresponding to each genotype in the first plurality.

Similarly, the first mixture of nucleic acids may comprise a first plurality of nucleotide sequences, wherein each nucleotide sequence of the first plurality encodes a genotype listed in the Table 1, and the second mixture of nucleic acids may comprise a second plurality of nucleotide sequences encoding wild type genotypes corresponding to each genotype in the first plurality. For example, the first mixture of nucleic acids may comprise a first plurality of nucleotide sequences, wherein the first plurality of nucleotide sequences encodes 1, 2, 3, 4, 5, 6, 7, 8, 9, 10, 11, 12, 13, 14, 15, 16, 17, 18, 19, 20, 1, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, or 66 genotypes listed in Table 1, and the second mixture of nucleic acids may comprise a second plurality of nucleotide sequences encoding wild type genotypes corresponding to each genotype in the first plurality. In some embodiments, the first mixture of nucleic acids comprises a first nucleotide sequence encoding a portion of a gene comprising a mutation, wherein the gene is selected from MTOR, MPL, NRAS, PARP1, AKT3, DNMT3A, MSH2, IDH1, VHL, MLH1, MYD88, CTNNB1, ATR, PIK3CA, FGFR3, PDGFRA, KIT, FBXW7, APC, GABRG2, NPM1, EGFR, MET, BRAF, EZH2, JAK2, GNAQ, RET, PTEN, ATM, KRAS, PTPN11, FLT3, RB1, PARP2, ARHGAP5, AKT1, RAD51, IDH2, TP53, NF1, SMAD4, AKT2, ERCC1, and GNAS, and the second mixture of nucleic acids comprises a second nucleotide sequence encoding the portion of the gene, but comprising a wild type sequence. In some embodiments, the first mixture of nucleic acids comprises a first plurality of nucleotide sequences, wherein each nucleotide sequence of the first plurality encodes a portion of a gene comprising a mutation, and the genes are selected from MTOR, MPL, NRAS, PARP1, AKT3, DNMT3A, MSH2, IDH1, VHL, MLH1, MYD88, CTNNB1, ATR, PIK3CA, FGFR3, PDGFRA, KIT, FBXW7, APC, GABRG2, NPM1, EGFR, MET, BRAF, EZH2, JAK2, GNAQ, RET, PTEN, ATM, KRAS, PTPN11, FLT3, RB1, PARP2, ARHGAP5, AKT1, RAD51, IDH2, TP53, NF1, SMAD4, AKT2, ERCC1, and GNAS, and the second mixture of nucleic acids comprises a second plurality of nucleotide sequences, wherein the second plurality of nucleotide sequences encodes the portion of each gene, but comprising a wild type sequence for each gene. In some embodiments, the first mixture of nucleic acids comprises a plurality of nucleotide sequences, wherein each nucleotide sequence of the plurality encodes a portion of a gene comprising a mutation, the nucleotide sequences of the plurality encode portions of 1, 2, 3, 4, 5, 6, 7, 8, 9, 10, 11, 12, 13, 14, 15, 16, 17, 18, 19, 20, 1, 22, 23, 24, 25, 26, 27, 28, 29, 30, 31, 32, 33, 34, 35, 36, 37, 38, 39, 40, 41, 42, 43, or 44 different genes, and the genes are selected from MTOR, MPL, NRAS, PARP1, AKT3, DNMT3A, MSH2, IDH1, VHL, MLH1, MYD88, CTNNB1, ATR, PIK3CA, FGFR3, PDGFRA, KIT, FBXW7, APC, GABRG2, NPM1, EGFR, MET, BRAF, EZH2, JAK2, GNAQ, RET, PTEN, ATM, KRAS, PTPN11, FLT3, RB1, PARP2, ARHGAP5, AKT1, RAD51, IDH2, TP53, NF1, SMAD4, AKT2, ERCC1, and GNAS, and the second mixture of nucleic acids comprises a second plurality of nucleotide sequences, wherein the second plurality of nucleotide sequences encodes the portion of each gene, but comprising a wild type sequence for each gene.

In some embodiments, the first mixture of nucleic acids comprises a first nucleotide sequence encoding a portion of a gene comprising a mutation, wherein the gene is selected from AKT1, ATM, BRAF, CDKN2A, CSF1R, EGFR, ERBB2 ("HER2"), ERBB4 ("HER4"), FGFR1, FGFR2, FGFR3, GNA11, HRAS, JAK2, JAK3, KDR, KIT, KRAS, MET, NOTCH1, NRAS, PDGFRA, PIK3CA, PTEN, RET, and STK11, and the second mixture of nucleic acids comprises a second nucleotide sequence comprising the portion of the gene, but comprising a wild type sequence. In some embodiments, the first mixture of nucleic acids comprises a first plurality of nucleotide sequences, wherein each nucleotide sequence of the first plurality encodes a portion of a gene comprising a mutation, and the genes are selected from AKT1, ATM, BRAF, CDKN2A, CSF1R, EGFR, ERBB2 ("HER2"), ERBB4 ("HER4"), FGFR1, FGFR2, FGFR3, GNA11, HRAS, JAK2, JAK3, KDR, KIT, KRAS, MET, NOTCH1, NRAS, PDGFRA, PIK3CA, PTEN, RET, and STK11, and the second mixture of nucleic acids comprises a second plurality of nucleotide sequences, wherein the second plurality of nucleotide sequences encodes the portion of each gene, but comprising a wild type sequence for each gene. In some embodiments, the first mixture of nucleic acids comprises a first plurality of nucleotide sequences, wherein each nucleotide sequence of the first plurality encodes a portion of a gene comprising a mutation, the nucleotide sequences of the plurality encode portions of 1, 2, 3, 4, 5, 6, 7, 8, 9, 10, 11, 12, 13, 14, 15, 16, 17, 18, 19, 20, 1, 22, 23, 24, 25, or 26 different genes, and the genes are selected from AKT1, ATM, BRAF, CDKN2A, CSF1R, EGFR, ERBB2 ("HER2"), ERBB4 ("HER4"), FGFR1, FGFR2, FGFR3, GNA11, HRAS, JAK2, JAK3, KDR, KIT, KRAS, MET, NOTCH1, NRAS, PDGFRA, PIK3CA, PTEN, RET, and STK11, and the second mixture of nucleic acids comprises a second plurality of nucleotide sequences, wherein the second plurality of nucleotide sequences encodes the portion of each gene, but comprising a wild type sequence for each gene.

*First and Second Mixtures of Nucleic Acids*

In some embodiments, the control comprises a first mixture of nucleic acids encoding a first genotype and a second mixture of nucleic acids encoding a second genotype, and the ratio of the copy number of each nucleotide sequence that encodes the first genotype to the copy number of each nucleotide sequence that encodes the second genotype is from about 1:1000 to 1000:1, such as from about 1:100 to about 100:1, about 1:50 to about 50:1, about 1:40 to about 40:1, about 1:30 to about 30:1, about 1:20 to about 20:1, about 1:15 to about 15:1, about 1:10 to about 10:1, about 1:9 to about 9:1, about 1:8 to about 8:1, about 1:7 to about 7:1, about 1:6 to about 6:1, about 1:5 to about 5:1, about 1:4, to about 4:1, about 1:3 to about 3:1, about 1:2 to about 2:1; about 1:1000 to 1:1, such as from about 1:100 to about 1:1, about 1:50 to about 1:1, about 1:40 to about 1:1, about 1:30 to about 1:1, about 1:20 to about 1:1, about 1:15 to about 1:1, about 1:10 to about 1:1, about 1:9 to about 1:1, about 1:8 to about 1:1, about 1:7 to about 1:1, about 1:6 to about 1:1, about 1:5 to about 1:1, about 1:4, to about 1:1, about 1:3 to about 1:1, or from about 1:2 to about 1:1. In some embodiments, the ratio of the copy number of each nucleotide sequence that encodes the first genotype to the copy number of each nucleotide sequence that encodes the second genotype is about 1:200 to about 1:2, such as about 1:200 to about 1:3, about 1:100 to about 1:2, about 1:100 to about 1:3, about 1:50 to about 1:2, about 1:50 to about 1:3, about 1:33 to about 1:2, about 1:33 to about 1:3, about 1:20 to about 1:2, or about 1:20 to about 1:3. In some embodiments, the ratio of the copy number of each nucleotide sequence that encodes the first genotype to the copy number of each nucleotide sequence that encodes the second genotype is about 1:1000, 1:100, 1:50, 1:40, 1:30, 1:20, 1:15, 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1, 15:1, 20:1, 30:1, 40:1, 50:1, 100:1, or 1000:1.

In some embodiments, the control comprises a first mixture of nucleic acids comprising a plurality of nucleotide sequences encoding a first genotype and a second mixture of nucleic acids comprising a plurality of nucleotide sequences encoding a second genotype, and the ratio of the copy number of each nucleotide sequence of the plurality encoding the first genotype to the copy number of each nucleotide sequence of the plurality encoding the second genotype is from about 1:1000 to 1000:1, such as from about 1:100 to about 100:1, about 1:50 to about 50:1, about 1:40 to about 40:1, about 1:30 to about 30:1, about 1:20 to about 20:1, about 1:15 to about 15:1, about 1:10 to about 10:1, about 1:9 to about 9:1, about 1:8 to about 8:1, about 1:7 to about 7:1, about 1:6 to about 6:1, about 1:5 to about 5:1, about 1:4, to about 4:1, about 1:3 to about 3:1, about 1:2 to about 2:1; about 1:1000 to 1:1, such as from about 1:100 to about 1:1, about 1:50 to about 1:1, about 1:40 to about 1:1, about 1:30 to about 1:1, about 1:20 to about 1:1, about 1:15 to about 1:1, about 1:10 to about 1:1, about 1:9 to about 1:1, about 1:8 to about 1:1, about 1:7 to about 1:1, about 1:6 to about 1:1, about 1:5 to about 1:1, about 1:4, to about 1:1, about 1:3 to about 1:1, or from about 1:2 to about 1:1. In some embodiments, the ratio of the copy number of each nucleotide sequences of the plurality encoding the first genotype to the copy number of each nucleotide sequence of the plurality encoding the second genotype is about 1:200 to about 1:2, such as about 1:200 to about 1:3, about 1:100 to about 1:2, about 1:100 to about 1:3, about 1:50 to about 1:2, about 1:50 to about 1:3, about 1:33 to about 1:2, about 1:33 to about 1:3, about 1:20 to about 1:2, or about 1:20 to about 1:3. In some embodiments, the ratio of the copy number of each nucleotide sequences of the plurality encoding the first genotype to the copy number of each nucleotide sequence of the plurality encoding the second genotype is about 1:1000, 1:100, 1:50, 1:40, 1:30, 1:20, 1:15, 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1, 15:1, 20:1, 30:1, 40:1, 50:1, 100:1, or 1000:1.

In some embodiments, the concentration of nucleic acids in the control is about 100 pg/mL to about 1 mg/mL, such as about 500 pg/mL to about 500 ng/mL, about 1 ng/mL to about 100 ng/mL, about 1 ng/mL to about 10 ng/mL, about 5 ng/mL to about 15 ng/mL, about 10 ng/mL to about 20 ng/mL, about 15 ng/mL to about 25 ng/mL, about 20 ng/mL to about 30 ng/mL, about 25 ng/mL to about 35 ng/mL, about 30 ng/mL to about 40 ng/mL, about 35 ng/mL to about 45 ng/mL, about 40 ng/mL to about 50 ng/mL, about 45 ng/mL to about 55 ng/mL, about 50 ng/mL to about 60 ng/mL, about 55 ng/mL to about 65 ng/mL, about 60 ng/mL to about 70 ng/mL, about 65 ng/mL to about 75 ng/mL, about 70 ng/mL to about 80 ng/mL, about 75 ng/mL to about 85 ng/mL, about 80 ng/mL to about 90 ng/mL, about 85 ng/mL to about 95 ng/mL, or about 90 ng/mL to about 100 ng/mL. In some embodiments, the concentration of nucleic acids in the control is about 5 ng/mL to about 50 ng/mL, such as about 5 ng/mL, about 10 ng/mL, about 15 ng/mL, about 20 ng/mL, about 25 ng/mL, about 30 ng/mL, about 35 ng/mL, about 40 ng/mL, about 45 ng/mL, or about 50 ng/mL. In some embodiments, the concentration of nucleic acids in the control is about 20 ng/mL to about 40 ng/mL.

In some embodiments, the nucleic acids in the first mixture make up about 0%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 50%, 55%, 60%, 65%, 70%, 75%, 80%, 83%, 85%, 88%, 90%, 91%, 92%, 93%, 94%, 95%, 96%, 97%, 98%, 99%, or 100% of the total concentration of nucleic acids in the control. In some embodiments, the nucleic acids in the first mixture make up from about 0% to about 10%, about 5% to about 15%, about 10% to about 20%, about 15% to about 25%, about 20% to about 30%, about 25% to about 35%, about 30% to about 40%, about 35% to about 45%, about 40% to about 50%, about 45% to about 55%, about 50% to about 60%, about 55% to about 65%, about 60% to about 70%, about 65% to about 75%, about 70% to about 80%, about 75% to about 85%, about 80% to about 90%, about 85% to about 95%, or about 90% to about 100% of the total concentration of nucleic acids in the control.

In some embodiments, the nucleic acids in the second mixture make up about 0%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 50%, 55%, 60%, 65%, 70%, 75%, 80%, 83%, 85%, 88%, 90%, 91%, 92%, 93%, 94%, 95%, 96%, 97%, 98%, 99%, or 100% of the total concentration of nucleic acids in the control. In some embodiments, the nucleic acids in the second mixture make up from about 0% to about 10%, about 5% to about 15%, about 10% to about 20%, about 15% to about 25%, about 20% to about 30%, about 25% to about 35%, about 30% to about 40%, about 35% to about 45%, about 40% to about 50%, about 45% to about 55%, about 50% to about 60%, about 55% to about 65%, about 60% to about 70%, about 65% to about 75%, about 70% to about 80%, about 75% to about 85%, about 80% to about 90%, about 85% to about 95%, or about 90% to about 100% of the total concentration of nucleic acids in the control.

In some embodiments, the average length of the nucleic acids in the control is about 20 base pairs to about 10,000 base pairs, such as about 35 base pairs to about 1000 base pairs, about 50 base pairs to about 900 base pairs, about 50 base pairs to about 800 base pairs, about 50 base pairs to about 700 base pairs, about 50 base pairs to about 600 base pairs, about 50 base pairs to about 500 base pairs, about 50 base pairs to about 400 base pairs, or about 50 base pairs to about 300 base pairs. In some embodiments, the average length of the nucleic acids in the control is about 50 base pairs to about 350 base pairs, such as about 100 base pairs to about 300 base pairs. The average length of the nucleic acids in the control may be about 100 base pairs, about 110 base pairs, about 120 base pairs, about 130 base pairs, about 140 base pairs, about 150 base pairs, about 160 base pairs, about 170 base pairs, about 180 base pairs, about 190 base pairs, about 200 base pairs, about 210 base pairs, about 220 base pairs, about 230 base pairs, about 240 base pairs, about 250 base pairs, about 260 base pairs, about 270 base pairs, about 280 base pairs, about 290 base pairs, or about 300 base pairs.

In some embodiments, the median length of the nucleic acids in the control is about 20 base pairs to about 10,000 base pairs, such as about 35 base pairs to about 1000 base pairs, about 50 base pairs to about 900 base pairs, about 50 base pairs to about 800 base pairs, about 50 base pairs to about 700 base pairs, about 50 base pairs to about 600 base pairs, about 50 base pairs to about 500 base pairs, about 50 base pairs to about 400 base pairs, or about 50 base pairs to about 300 base pairs. In some embodiments, the median length of the nucleic acids in the control is about 50 base pairs to about 350 base pairs, such as about 100 base pairs to about 300 base pairs. The median length of the nucleic acids in the control may be about 100 base pairs, about 110 base pairs, about 120 base pairs, about 130 base pairs, about 140 base pairs, about 150 base pairs, about 160 base pairs, about 170 base pairs, about 180 base pairs, about 190 base pairs, about 200 base pairs, about 210 base pairs, about 220 base pairs, about 230 base pairs, about 240 base pairs, about 250 base pairs, about 260 base pairs, about 270 base pairs, about 280 base pairs, about 290 base pairs, or about 300 base pairs.

In some embodiments, the average length of the nucleic acids in the control is about 20 nucleotides to about 10,000 nucleotides, such as about 35 nucleotides to about 1000 nucleotides, about 50 nucleotides to about 900 nucleotides, about 50 nucleotides to about 800 nucleotides, about 50 nucleotides to about 700 nucleotides, about 50 nucleotides to about 600 nucleotides, about 50 nucleotides to about 500 nucleotides, about 50 nucleotides to about 400 nucleotides, or about 50 nucleotides to about 300 nucleotides. In some embodiments, the average length of the nucleic acids in the control is about 50 nucleotides to about 350 nucleotides, such as about 100 nucleotides to about 300 nucleotides. The average length of the nucleic acids in the control may be about 100 nucleotides, about 110 nucleotides, about 120 nucleotides, about 130 nucleotides, about 140 nucleotides, about 150 nucleotides, about 160 nucleotides, about 170 nucleotides, about 180 nucleotides, about 190 nucleotides, about 200 nucleotides, about 210 nucleotides, about 220 nucleotides, about 230 nucleotides, about 240 nucleotides, about 250 nucleotides, about 260 nucleotides, about 270 nucleotides, about 280 nucleotides, about 290 nucleotides, or about 300 nucleotides.

In some embodiments, the median length of the nucleic acids in the control is about 20 nucleotides to about 10,000 nucleotides, such as about 35 nucleotides to about 1000 nucleotides, about 50 nucleotides to about 900 nucleotides, about 50 nucleotides to about 800 nucleotides, about 50 nucleotides to about 700 nucleotides, about 50 nucleotides to about 600 nucleotides, about 50 nucleotides to about 500 nucleotides, about 50 nucleotides to about 400 nucleotides, or about 50 nucleotides to about 300 nucleotides. In some embodiments, the median length of the nucleic acids in the control is about 50 nucleotides to about 350 nucleotides, such as about 100 nucleotides to about 300 nucleotides. The median length of the nucleic acids in the control may be about 100 nucleotides, about 110 nucleotides, about 120 nucleotides, about 130 nucleotides, about 140 nucleotides, about 150 nucleotides, about 160 nucleotides, about 170 nucleotides, about 180 nucleotides, about 190 nucleotides, about 200 nucleotides, about 210 nucleotides, about 220 nucleotides, about 230 nucleotides, about 240 nucleotides, about 250 nucleotides, about 260 nucleotides, about 270 nucleotides, about 280 nucleotides, about 290 nucleotides, or about 300 nucleotides.

In some embodiments, the average length of the nucleic acids in the first mixture of nucleic acids is about 20 base pairs to about 10,000 base pairs, such as about 35 base pairs to about 1000 base pairs, about 50 base pairs to about 900 base pairs, about 50 base pairs to about 800 base pairs, about 50 base pairs to about 700 base pairs, about 50 base pairs to about 600 base pairs, about 50 base pairs to about 500 base pairs, about 50 base pairs to about 400 base pairs, or about 50 base pairs to about 300 base pairs. In some embodiments, the average length of the nucleic acids in the first mixture of nucleic acids is about 50 base pairs to about 350 base pairs, such as about 100 base pairs to about 300 base pairs. The average length of the nucleic acids in the first mixture of nucleic acids may be about 100 base pairs, about 110 base pairs, about 120 base pairs, about 130 base pairs, about 140 base pairs, about 150 base pairs, about 160 base pairs, about 170 base pairs, about 180 base pairs, about 190 base pairs, about 200 base pairs, about 210 base pairs, about 220 base pairs, about 230 base pairs, about 240 base pairs, about 250 base pairs, about 260 base pairs, about 270 base pairs, about 280 base pairs, about 290 base pairs, or about 300 base pairs. In some embodiments, the average length of the nucleic acids in the first mixture of nucleic acids is about 8 base pairs to about 1000 base pairs, such as about 10 base pairs to about 800 base pairs, about 12 base pairs to about 600 base pairs, about 14 base pairs to about 400 base pairs, about 15 base pairs to about 500 base pairs, about 16 base pairs to about 400 base pairs, about 17 base pairs to about 300 base pairs, about 18 base pairs to about 200 base pairs, about 19 base pairs to about 100 base pairs, or about 20 base pairs to about 50 base pairs. The average length of the nucleic acids in the first mixture of nucleic acids may be about 10 base pairs, about 11 base pairs, about 12 base pairs, about 13 base pairs, about 14 base pairs, about 15 base pairs, about 16 base pairs, about 17 base pairs, about 18 base pairs, about 19 base pairs, about 20 base pairs, about 21 base pairs, about 22 base pairs, about 23 base pairs, about 24 base pairs, about 25 base pairs, about 26 base pairs, about 27 base pairs, about 28 base pairs, about 29 base pairs, or about 30 base pairs.

In some embodiments, the median length of the nucleic acids in the first mixture of nucleic acids is about 20 base pairs to about 10,000 base pairs, such as about 35 base pairs to about 1000 base pairs, about 50 base pairs to about 900 base pairs, about 50 base pairs to about 800 base pairs, about 50 base pairs to about 700 base pairs, about 50 base pairs to about 600 base pairs, about 50 base pairs to about 500 base pairs, about 50 base pairs to about 400 base pairs, or about 50 base pairs to about 300 base pairs. In some embodiments, the median length of the nucleic acids in the first mixture of nucleic acids is about 50 base pairs to about 350 base pairs, such as about 100 base pairs to about 300 base pairs. The median length of the nucleic acids in the first mixture of nucleic acids may be about 100 base pairs, about 110 base pairs, about 120 base pairs, about 130 base pairs, about 140 base pairs, about 150 base pairs, about 160 base pairs, about 170 base pairs, about 180 base pairs, about 190 base pairs, about 200 base pairs, about 210 base pairs, about 220 base pairs, about 230 base pairs, about 240 base pairs, about 250 base pairs, about 260 base pairs, about 270 base pairs, about 280 base pairs, about 290 base pairs, or about 300 base pairs. In some embodiments, the median length of the nucleic acids in the first mixture of nucleic acids is about 8 base pairs to about 1000 base pairs, such as about 10 base pairs to about 800 base pairs, about 12 base pairs to about 600 base pairs, about 14 base pairs to about 400 base pairs, about 15 base pairs to about 500 base pairs, about 16 base pairs to about 400 base pairs, about 17 base pairs to about 300 base pairs, about 18 base pairs to about 200 base pairs, about 19 base pairs to about 100 base pairs, or about 20 base pairs to about 50 base pairs. The median length of the nucleic acids in the first mixture of nucleic acids may be about 10 base pairs, about 11 base pairs, about 12 base pairs, about 13 base pairs, about 14 base pairs, about 15 base pairs, about 16 base pairs, about 17 base pairs, about 18 base pairs, about 19 base pairs, about 20 base pairs, about 21 base pairs, about 22 base pairs, about 23 base pairs, about 24 base pairs, about 25 base pairs, about 26 base pairs, about 27 base pairs, about 28 base pairs, about 29 base pairs, or about 30 base pairs.

In some embodiments, the average length of the nucleic acids in the first mixture of nucleic acids is about 20 nucleotides to about 10,000 nucleotides, such as about 35 nucleotides to about 1000 nucleotides, about 50 nucleotides to about 900 nucleotides, about 50 nucleotides to about 800 nucleotides, about 50 nucleotides to about 700 nucleotides, about 50 nucleotides to about 600 nucleotides, about 50 nucleotides to about 500 nucleotides, about 50 nucleotides to about 400 nucleotides, or about 50 nucleotides to about 300 nucleotides. In some embodiments, the average length of the nucleic acids in the first mixture of nucleic acids is about 50 nucleotides to about 350 nucleotides, such as about 100 nucleotides to about 300 nucleotides. The average length of the nucleic acids in the first mixture of nucleic acids may be about 100 nucleotides, about 110 nucleotides, about 120 nucleotides, about 130 nucleotides, about 140 nucleotides, about 150 nucleotides, about 160 nucleotides, about 170 nucleotides, about 180 nucleotides, about 190 nucleotides, about 200 nucleotides, about 210 nucleotides, about 220 nucleotides, about 230 nucleotides, about 240 nucleotides, about 250 nucleotides, about 260 nucleotides, about 270 nucleotides, about 280 nucleotides, about 290 nucleotides, or about 300 nucleotides. In some embodiments, the average length of the nucleic acids in the first mixture of nucleic acids is about 8 nucleotides to about 1000 nucleotides, such as about 10 nucleotides to about 800 nucleotides, about 12 nucleotides to about 600 nucleotides, about 14 nucleotides to about 400 nucleotides, about 15 nucleotides to about 500 nucleotides, about 16 nucleotides to about 400 nucleotides, about 17 nucleotides to about 300 nucleotides, about 18 nucleotides to about 200 nucleotides, about 19 nucleotides to about 100 nucleotides, or about 20 nucleotides to about 50 nucleotides. The average length of the nucleic acids in the first mixture of nucleic acids may be about 10 nucleotides, about 11 nucleotides, about 12 nucleotides, about 13 nucleotides, about 14 nucleotides, about 15 nucleotides, about 16 nucleotides, about 17 nucleotides, about 18 nucleotides, about 19 nucleotides, about 20 nucleotides, about 21 nucleotides, about 22 nucleotides, about 23 nucleotides, about 24 nucleotides, about 25 nucleotides, about 26 nucleotides, about 27 nucleotides, about 28 nucleotides, about 29 nucleotides, or about 30 nucleotides.

In some embodiments, the median length of the nucleic acids in the first mixture of nucleic acids is about 20 nucleotides to about 10,000 nucleotides, such as about 35 nucleotides to about 1000 nucleotides, about 50 nucleotides to about 900 nucleotides, about 50 nucleotides to about 800 nucleotides, about 50 nucleotides to about 700 nucleotides, about 50 nucleotides to about 600 nucleotides, about 50 nucleotides to about 500 nucleotides, about 50 nucleotides to about 400 nucleotides, or about 50 nucleotides to about 300 nucleotides. In some embodiments, the median length of the nucleic acids in the first mixture of nucleic acids is about 50 nucleotides to about 350 nucleotides, such as about 100 nucleotides to about 300 nucleotides. The median length of the nucleic acids in the first mixture of nucleic acids may be about 100 nucleotides, about 110 nucleotides, about 120 nucleotides, about 130 nucleotides, about 140 nucleotides, about 150 nucleotides, about 160 nucleotides, about 170 nucleotides, about 180 nucleotides, about 190 nucleotides, about 200 nucleotides, about 210 nucleotides, about 220 nucleotides, about 230 nucleotides, about 240 nucleotides, about 250 nucleotides, about 260 nucleotides, about 270 nucleotides, about 280 nucleotides, about 290 nucleotides, or about 300 nucleotides. In some embodiments, the median length of the nucleic acids in the first mixture of nucleic acids is about 8 nucleotides to about 1000 nucleotides, such as about 10 nucleotides to about 800 nucleotides, about 12 nucleotides to about 600 nucleotides, about 14 nucleotides to about 400 nucleotides, about 15 nucleotides to about 500 nucleotides, about 16 nucleotides to about 400 nucleotides, about 17 nucleotides to about 300 nucleotides, about 18 nucleotides to about 200 nucleotides, about 19 nucleotides to about 100 nucleotides, or about 20 nucleotides to about 50 nucleotides. The median length of the nucleic acids in the first mixture of nucleic acids may be about 10 nucleotides, about 11 nucleotides, about 12 nucleotides, about 13 nucleotides, about 14 nucleotides, about 15 nucleotides, about 16 nucleotides, about 17 nucleotides, about 18 nucleotides, about 19 nucleotides, about 20 nucleotides, about 21 nucleotides, about 22 nucleotides, about 23 nucleotides, about 24 nucleotides, about 25 nucleotides, about 26 nucleotides, about 27 nucleotides, about 28 nucleotides, about 29 nucleotides, or about 30 nucleotides.

In some embodiments, the average length of the nucleic acids in the second mixture of nucleic acids is about 20 base pairs to about 10,000 base pairs, such as about 35 base pairs to about 1000 base pairs, about 50 base pairs to about 900 base pairs, about 50 base pairs to about 800 base pairs, about 50 base pairs to about 700 base pairs, about 50 base pairs to about 600 base pairs, about 50 base pairs to about 500 base pairs, about 50 base pairs to about 400 base pairs, or about 50 base pairs to about 300 base pairs. In some embodiments, the average length of the nucleic acids in the second mixture of nucleic acids is about 50 base pairs to about 350 base pairs, such as about 100 base pairs to about 300 base pairs. The average length of the nucleic acids in the second mixture of nucleic acids may be about 100 base pairs, about 110 base pairs, about 120 base pairs, about 130 base pairs, about 140 base pairs, about 150 base pairs, about 160 base pairs, about 170 base pairs, about 180 base pairs, about 190 base pairs, about 200 base pairs, about 210 base pairs, about 220 base pairs, about 230 base pairs, about 240 base pairs, about 250 base pairs, about 260 base pairs, about 270 base pairs, about 280 base pairs, about 290 base pairs, or about 300 base pairs.

In some embodiments, the median length of the nucleic acids in the second mixture of nucleic acids is about 20 base pairs to about 10,000 base pairs, such as about 35 base pairs to about 1000 base pairs, about 50 base pairs to about 900 base pairs, about 50 base pairs to about 800 base pairs, about 50 base pairs to about 700 base pairs, about 50 base pairs to about 600 base pairs, about 50 base pairs to about 500 base pairs, about 50 base pairs to about 400 base pairs, or about 50 base pairs to about 300 base pairs. In some embodiments, the median length of the nucleic acids in the second mixture of nucleic acids is about 50 base pairs to about 350 base pairs, such as about 100 base pairs to about 300 base pairs. The median length of the nucleic acids in the second mixture of nucleic acids may be about 100 base pairs, about 110 base pairs, about 120 base pairs, about 130 base pairs, about 140 base pairs, about 150 base pairs, about 160 base pairs, about 170 base pairs, about 180 base pairs, about 190 base pairs, about 200 base pairs, about 210 base pairs, about 220 base pairs, about 230 base pairs, about 240 base pairs, about 250 base pairs, about 260 base pairs, about 270 base pairs, about 280 base pairs, about 290 base pairs, or about 300 base pairs.

In some embodiments, the average length of the nucleic acids in the second mixture of nucleic acids is about 20 nucleotides to about 10,000 nucleotides, such as about 35 nucleotides to about 1000 nucleotides, about 50 nucleotides to about 900 nucleotides, about 50 nucleotides to about 800 nucleotides, about 50 nucleotides to about 700 nucleotides, about 50 nucleotides to about 600 nucleotides, about 50 nucleotides to about 500 nucleotides, about 50 nucleotides to about 400 nucleotides, or about 50 nucleotides to about 300 nucleotides. In some embodiments, the average length of the nucleic acids in the second mixture of nucleic acids is about 50 nucleotides to about 350 nucleotides, such as about 100 nucleotides to about 300 nucleotides. The average length of the nucleic acids in the second mixture of nucleic acids may be about 100 nucleotides, about 110 nucleotides, about 120 nucleotides, about 130 nucleotides, about 140 nucleotides, about 150 nucleotides, about 160 nucleotides, about 170 nucleotides, about 180 nucleotides, about 190 nucleotides, about 200 nucleotides, about 210 nucleotides, about 220 nucleotides, about 230 nucleotides, about 240 nucleotides, about 250 nucleotides, about 260 nucleotides, about 270 nucleotides, about 280 nucleotides, about 290 nucleotides, or about 300 nucleotides.

In some embodiments, the median length of the nucleic acids in the second mixture of nucleic acids is about 20 nucleotides to about 10,000 nucleotides, such as about 35 nucleotides to about 1000 nucleotides, about 50 nucleotides to about 900 nucleotides, about 50 nucleotides to about 800 nucleotides, about 50 nucleotides to about 700 nucleotides, about 50 nucleotides to about 600 nucleotides, about 50 nucleotides to about 500 nucleotides, about 50 nucleotides to about 400 nucleotides, or about 50 nucleotides to about 300 nucleotides. In some embodiments, the median length of the nucleic acids in the second mixture of nucleic acids is about 50 nucleotides to about 350 nucleotides, such as about 100 nucleotides to about 300 nucleotides. The median length of the nucleic acids in the second mixture of nucleic acids may be about 100 nucleotides, about 110 nucleotides, about 120 nucleotides, about 130 nucleotides, about 140 nucleotides, about 150 nucleotides, about 160 nucleotides, about 170 nucleotides, about 180 nucleotides, about 190 nucleotides, about 200 nucleotides, about 210 nucleotides, about 220 nucleotides, about 230 nucleotides, about 240 nucleotides, about 250 nucleotides, about 260 nucleotides, about 270 nucleotides, about 280 nucleotides, about 290 nucleotides, or about 300 nucleotides.

In some embodiments, the nucleic acids of a control comprising cfDNA are more stable than nucleic acids of a control that does not comprise cfDNA, *i.e.,* a control with a similar concentration of nucleic acids of the same origin in a similar buffer. Stability may refer to a reduced propensity to aggregate. In some embodiments, the nucleic acids of a control comprising cfDNA are less likely to aggregate than nucleic acids of a control that does not comprise cfDNA. Aggregation may be determined, for example, by measuring the apparent length of nucleic acids in a composition, for example, by using a Bioanalyzer (Agilent). The nucleic acids of a control according to various embodiments of the disclosure have not aggregated if most nucleic acids of the control fall within an observed size range of about 35 base pairs to about 1000 base pairs, *e.g.*, as observed using a Bioanalyzer. The nucleic acids of a control have aggregated if most nucleic acids of the control are observed to be more than 1000 base pairs, *e.g.*, as observed using a Bioanalyzer.

In some embodiments, the nucleic acids of a control are stable when stored at a temperature of about 0°C to about 100°C, such as about 4°C to about 45°C, about 15°C to about 45°C, about 4°C to about 25°C, or about 15°C to about 25°C, *e.g.,* most of the nucleic acids of the control fall within an observed size range of about 35 base pairs to about 1000 base pairs as analyzed by a Bioanalyzer after storage. In some embodiments, the nucleic acids of a control are stable at a temperature of about 0°C to about 100°C for a period of time from about 1 day to about 5 years. In some embodiments, the nucleic acids of a control are stable at a temperature of about 4°C to about 45°C for a period of time from about 1 day to about 5 years, such as about 1 week to about 2 years, about 1 month to about 18 months, or about 2 months to about 12 months. In some embodiments, the nucleic acids of a control are stable at a temperature of about 4°C to about 25°C for a period of time from about 1 day to about 5 years, such as about 1 week to about 2 years, about 1 month to about 18 months, or about 2 months to about 12 months. In some embodiments, the nucleic acids of a control are stable at a temperature of about 15°C to about 25°C for a period of time from about 1 day to about 5 years, such as about 1 week to about 2 years, about 1 month to about 18 months, or about 2 months to about 12 months.

In some embodiments, the nucleic acids of a control do not form aggregates when stored at a temperature of about 0°C to about 100°C, such as about 4°C to about 45°C, about 15°C to about 45°C, about 4°C to about 25°C, or about 15°C to about 25°C, *e.g.,* most of the nucleic acids of the control fall within an observed size range of about 35 base pairs to about 1000 base pairs as analyzed by a Bioanalyzer after storage. In some embodiments, the nucleic acids of a control do not form aggregates when stored at a temperature of about 0°C to about 100°C for a period of time from about 1 day to about 5 years. In some embodiments, the nucleic acids of a control do not form aggregates when stored at a temperature of about 4°C to about 45°C for a period of time from about 1 day to about 5 years, such as about 1 week to about 2 years, about 1 month to about 18 months, or about 2 months to about 12 months. In some embodiments, the nucleic acids of a control do not form aggregates when stored at a temperature of about 4°C to about 25°C for a period of time from about 1 day to about 5 years, such as about 1 week to about 2 years, about 1 month to about 18 months, or about 2 months to about 12 months. In some embodiments, the nucleic acids of a control do not form aggregates when stored at a temperature of about 15°C to about 25°C for a period of time from about 1 day to about 5 years, such as about 1 week to about 2 years, about 1 month to about 18 months, or about 2 months to about 12 months.

The period of time may be at least 1 day, at least 1 week, at least 1 month, at least 2 months, at least 3 months, at least 4 months, at least 5 months, at least 6 months, or at least 1 year. The period of time may be about 1 week to about 5 years, about 1 month to about 5 years, about 2 months to about 5 years, about 6 months to about 5 years, about 1 year to about 5 years, about 1 week to about 2 years, about 1 month to about 2 years, about 2 months to about 2 years, or about 6 months to about 2 years. The period of time may be about 1 day, about 1 week, about 1 month, about 2 months, about 3 months, about 4 months, about 5 months, about 6 months, or about 1 year.

## II. METHODS FOR ANALYZING CFDNA

In some embodiments, disclosed herein are methods for quantitative analysis of cell-free DNA (cfDNA) comprising, (a) treating the 5' end or 3' end or both of cfDNA with one or more end-repair enzymes to generate end-repaired cfDNA; (b) ligating one or more adaptors, wherein the adaptors comprise a restriction enzyme site and a sequence from a respiratory syncytial virus (RSV) (*i.e.,* the adaptors comprise a restriction enzyme site and sequences that would not be expected in cfDNA such as those from respiratory syncytial virus (RSV)), to each end of the end-repaired cfDNA to generate a cfDNA library; (c) amplifying the cfDNA library to generate a cfDNA library clone; (d) digesting the cfDNA library clone with a restriction enzyme to generate a modified cfDNA clone library; wherein digesting the cfDNA library clones with the restriction enzyme removes the one or more adaptors; (e) purifying the modified cfDNA clone library; and (f) performing quantitative analysis of one or more target loci in the modified cfDNA library clone. In some embodiments, amplifying the cfDNA library is stopped prior to the amplification reaching a plateau. In some embodiments, at least 50% of the sequences in the modified cfDNA library clone have between 150 and 200 bp of the same cfDNA sequence as the end-repaired cfDNA. The methods may further comprise isolating cfDNA from a biological sample of a subject (*e.g.,* amniotic fluid, blood, plasma, serum, semen, lymphatic fluid, cerebral spinal fluid, ocular fluid, urine, saliva, stool, mucous, and sweat). The methods may comprise sonication DNA in order to generate shorter molecules of DNA. Or, the methods may not involve sonicating the isolated cfDNA.

In some embodiments, the treating of the 5' end and 3' end of cfDNA with one or more end-repair enzymes further consists of adding a dA base to the 3' end of the cfDNA. The 5' and 3' ends of the end-repaired cfDNA may be blunt ends, and the one or more adaptors comprise a plurality of adaptor species. The one or more adaptors may comprise a primer binding site or primer binding sites for amplification of the cfDNA library. In some embodiments, the adaptor comprises two single stranded DNA molecules, such as SEQ ID NO:1 and SEQ ID NO:2. In some embodiments, the adaptor comprises two single stranded DNA molecules that have been annealed.

In some embodiments, the restriction enzyme to generate a modified cfDNA clone library is an enzyme that cuts the DNA sequence outside of the enzyme's recognition site (*e.g.,* SapI). The restriction enzyme used by the methods disclosed herein may be expected to change the size of about 1 in 40 amplified molecules by cutting within the amplified molecules when an applicable restriction site is found within the cfDNA sequence. Since cfDNA is rare, the methods disclosed herein may be used to prepare sufficient amplified cfDNA for multiple analyses and on multiple platforms as is needed for controls, reference materials and proficiency testing samples. Additionally, the amplified cfDNA should be directly compatible with NGS-based assays that sequence the ends of cfDNA, although an additional end repair step may be necessary if not already performed as part of those assays.

The control or reference material used to generate the cfDNA library may contain a set of longer than desired DNA molecules, such as those that have a sequence length that is greater than 600 base pairs. The longer DNA may be removed from the cfDNA library by addition of an appropriate amount of AMPure XP beads or through other methods, such as agarose gel purification. In some embodiments, the, majority of the cfDNA library is at or below 600 base pairs.

Complexity is a measure of sequence diversity in the sample. Obtaining and maintaining a sufficiently high complexity is critical for amplified ccfDNA. The minimum complexity is also dependent on the targeted assay. For example, plasma contains about 5 ng/mL of ccfDNA. With an average length of ~160 bp, this leads to potentially ~28.5 billion different molecules per mL of plasma, of which Verinata and Sequenom assays only sequence 10-20 million. If all ccfDNA molecules could be sequenced, then a microliter of plasma would be sufficient. However, these assays use amplification in order to obtain sufficient concentrations of library for hybridization to flow cells, which makes it possible to sequence the same molecule more than once. In fact, if one microliter were analyzed and all ccfDNA could be converted into a library, then only about half the NGS reads would be unique, which would manifest as a very high percentage of duplicate reads. However, with 0.5 ng of ccfDNA represented in the library, about 99.3% of reads would occur only once.

At the same time, a SNP-based assay or a ddPCR-based assay would require a higher complexity. Given that a human genome has a mass of ~3.5 pg, 5 ng only represents about 1,429 copies. For heterozygous SNPs, the observed variant frequencies can be modeled using binomial distributions. Because not all of the input material will amplify and because amplified material has the potential to be sequenced twice, the distributions observed after sequencing will be even broader. Thus, SNP-based NIPT assays require a higher complexity in the input material than those that sequence the ends of ccfDNA.

For ctDNA assays, it has been reported that the median observed variant frequency in patient samples is now around 0.6 %. Thus, half the samples have variant frequencies below this and ctDNA assays likely have to function at 0.1% - if not 0.01% for resistance mutations like EGFR T790M.

This also affects commutability since an assay that can analyze 5 ng is expected to detect a variant at 0.6 % the vast majority of times. However, if only 1 ng of the 5 ng can be analyzed (e.g., due to poor adapter ligation), then a variant at 0.6 % may get missed 20% of the time and could suggest poor assay performance even though it may be due to poor reference material performance.

In order to maintain sufficient "complexity" in the amplified cfDNA library, a sufficient amount of input cfDNA library should be amplified such that DNA target regions are represented at least 10 times - preferably significantly more times. In some embodiments, multiple cfDNA libraries may be prepared, amplified, and their products pooled in order to obtain sufficient complexity in the output material. In some embodiments, one large cfDNA library may be prepared. In some embodiments, the cfDNA library or cfDNA libraries are distributed into multiple PCR reactions. In some embodiments, those reactions are carried out in parallel. In some embodiments, those reactions are not carried out in parallel. In some embodiments, the products from multiple PCR reactions are pooled. In some embodiments, the pooling occurs at a later step, for example, after digestion with restriction enzymes.

Polymerase chain reaction (PCR) may be performed to amplify the cfDNA library. The input into a reaction can vary but the output of all subsequently pooled reactions should be of sufficient complexity. PCR should not be carried out beyond the point where amplification is still exponential, which occurs when about 8 ng/$\mu$L of amplified library is present in a reaction. However, this amount can vary depending on reaction conditions. In some embodiments, less than 8 ng/$\mu$l are produced. The components of the PCR comprise primers that have a 5' end and a 3'end, and a proofreading thermostable polymerase. In some embodiments, the thermostable polymerase may be a "hotstart" polymerase so that PCR reaction components may be mixed at room temperature. The primers may be modified at the 5' end of the primer (*e.g.*, biotinylation), or the modification at the 5' end of the primer comprises phosphorothioation with at least 1, 2, 3, or 4 nucleotides. The primers may be SEQ ID NO:3 and SEQ ID NO:4.

Amplified ccfDNA involves generating a library from DNA input material. The efficiency with which the library is generated depends on the input material. With sonicated DNA, only about 4-5 % of the input material is converted into amplifiable library. With ccfDNA, a significantly higher percentage of the input material can be converted into amplifiable library.

For a SNP-based NIPT assay, the amount of ccfDNA from a (pregnant) female donor should be at least 25 ng - if not higher - in order to allow for a library incorporation efficiency of ~20 %.

For an MPSS NIPT assay, the amount of ccfDNA can be lower, but should still be around 25 ng in order to allow for compatibility with SNP-based assays.

For a ctDNA assay that typically analyzes 50 ng of ccfDNA, the complexity of the library should be higher than 50 ng. This can be attained by starting with at least 10 $\mu$g of sonicated input DNA. With a library incorporation efficiency of 5 %, this leads to ~500 ng of amplifiable material. After size selection, ~100 ng of sized amplifiable library can be recovered. Overall, with 100 ng of amplifiable material, variants starting at 0.6 % should be recovered close to 0.6 %. However, with lower amounts of amplifiable material, such variants would deviate more from 0.6 %. The impact on 0.1 % variants would be even greater and - at 10 ng - there is a chance that a 0.1 % variant will not end up in the library.

In some embodiments, the entire library is amplified in order to maintain the complexity of the library. If only 10 % of the library is amplified, then the complexity of the amplified material will be 10-fold lower than what was found in the library. This is unlikely to be acceptable for ctDNA.

In some embodiments, an amplified library is re-amplified. This causes complexity to be reduced because a given aliquot will not have an even amount of all molecules. In order to minimize the loss in complexity, the amount of re-amplified library is significantly higher than the original amount of amplifiable library. For example, with ctDNA, about 500 ng of amplified library is used for re-amplification of 50 ng.

In some embodiments, a Test Amplification is performed prior to the Production Amplification, which is used to determine the amount of amplifiable library as well as the optimal number of cycles that should be used to amplify the library. By dividing the amount of amplifiable library that is used for the Production Amplification by 3.5 pg/copy, it is possible to estimate the complexity of the library for SNP-based NIPT assays and ctDNA assays (=input).

In some embodiments, the amplified library (=output) can then be used for another round of re-amplification. There will be a loss in complexity because - due to underlying Poisson distributions - not all sequences from the original amplified material may be present, some sequences from the original material may be present multiple times, and sequences that are present multiple times will not all be present the same number of times.

The complexity of material derived from re-amplification (=new input) can be estimated as follows:

$$new\ input = \frac{input \times output}{input + output}$$

Thus, if 50 ng of amplifiable library (=input) is amplified, and 500 ng of amplified library (=output) is used for re-amplification, then the re-amplified material should have a complexity of ~45.5 ng. On the other hand, if only 50 ng of amplified library is re-amplified, then the re-amplified material is expected to have a complexity of 25 ng, which would be a 50% loss compared to what is found in the original amplified library, which would result in noticeably wider variant frequency distributions. It should be noted that 500 ng of amplified library is equal to the PCR product of about 2 wells.

Overall, the relative complexity of re-amplified material can be estimated from the relative amount of amplified material that is used compared to the original amplifiable material as follows:

$$relative\ complexity = \frac{relative\ amount}{1 + relative\ amount}$$

Thus, if 10 times the amount of amplified material is re-amplified compared to the amount of amplifiable material that was used to make the amplified material, then the relative complexity is expected to be ~91% in the re-amplified material compared to the amplified material.

Similarly, the relative amount that is needed in order to obtain a given relative complexity can be estimated as follows:

$$relative\ amount = \frac{relative\ complexity}{1 - relative\ complexity}$$

Thus, if a 90% relative complexity is desired in the output of re-amplified material, then 9-fold more amplified material should be used for re-amplification than was used to make the amplified material. If a 95% relative complexity is desired, then the relative amount is 19-fold. If a 99% relative complexity is desired, then the relative amount if 99-fold, which is no longer feasible. With ~4 ng/μl of recoverable PCR output, an initial amplification of a 96 well plate can result in around 20,000 ng of material. This can be used as-is in order to obtain ~10,000 ng of output. A remaining 500 ng could be re-amplified and the subsequent output would have a -10% loss in complexity. Each time this is repeated, there would be another ~10% loss in complexity.

On the other hand, assuming that ~50 ng of amplifiable library was used in order to obtain the ~20,000 ng, those 20,000 ng could be used to prepare 40 aliquots of 500 ng, which would be used for re-amplification. The product itself would be result from the re-amplified material, and would consistently have ~90% the complexity of the amplifiable library (until those 40 aliquots are used up).

In some embodiments, cfDNA is maintained in 50 mM Na⁺ containing TE buffer or similar. In some embodiments, PCR amplification is performed only up to the point where amplification is no longer -exponential. In some embodiments, the optimal number of cycles for amplification is determined accurately for each library

In some embodiments, the quantitative analysis is performed on a plurality of loci in the cfDNA library clones, or the quantitative analysis is performed on a plurality of loci in a plurality of cfDNA clone libraries.

The quantitative analysis may comprise hybridizing one or more capture probes to a target locus to form capture probe-cfDNA clone complexes, isolating the capture probe-cfDNA clone complexes, and amplification of the cfDNA clone sequence in the isolated hybridized capture probe-cfDNA clone complexes.

In some embodiments, the quantitative analysis comprises DNA sequencing to generate a plurality of sequencing reads. Bioinformatic analysis plurality of sequencing reads may be used (a) to quantify the number of genome equivalents analyzed in the cfDNA clone library; (b) to detect genetic variants in a target genetic locus; (c) to detect mutations within a target genetic locus; (d) to detect genetic fusions within a target genetic locus; and (e) to measure copy number fluctuations within a target genetic locus.

In some embodiments, the subject has been diagnosed with a genetic disease and quantitative genetic analysis is used to identify or detect one or more genetic lesions that cause or are associated with the genetic disease. The genetic lesion may comprise a nucleotide transition or transversion, a nucleotide insertion or deletion, a genomic rearrangement, a change in copy number, or a gene fusion. The genetic disease may be a cancer. In some examples, the subject is pregnant or a transplant recipient. When the subject is pregnant, quantitative analysis may be used to identify or detect one or more genetic variants or genetic lesions of one or more target genetic loci in fetal cfDNA. The quantitative analysis may also be used to identify or detect donor cfDNA in the subject.

In some embodiments, disclosed herein are methods of predicting, diagnosing, or monitoring a genetic disease in a subject comprising, (a) isolating or obtaining a sample of cfDNA from a subject; (b) treating the 5' end or 3' end or both of cfDNA with one or more end-repair enzymes to generate end-repaired cfDNA; (c) ligating one or more adaptors, wherein the adaptors comprise a restriction enzyme site and a sequence from a respiratory syncytial virus (RSV) (*i.e.,* the adaptors comprise a restriction enzyme site and sequences that would not be expected in cfDNA such as those from respiratory syncytial virus (RSV)), to each end of the end-repaired cfDNA to generate a cfDNA library; (d) amplifying the cfDNA library to generate a cfDNA library clone; (e) digesting the cfDNA library clone with a restriction enzyme to generate a modified cfDNA clone library; wherein the digesting the cfDNA library clone with the restriction enzyme removes the one or more adaptors; (f) purifying the modified cfDNA clone library; and (g) performing a quantitative analysis of one or more target loci in the modified cfDNA library clone, wherein the detection of one or more genetic lesions in the one or more target genetic loci is prognostic for, diagnostic of, or monitors the progression of the genetic disease. In some examples, the amplification of the cfDNA library is stopped prior to the amplification reaching a plateau. In some embodiments, at least 50% of the sequences in the modified cfDNA library clone have between 150 and 200 bp of the same cfDNA sequence as the end-repaired cfDNA. The cfDNA may isolated from a biological sample (*e.g.*, amniotic fluid, blood, plasma, serum, semen, lymphatic fluid, cerebral spinal fluid, ocular fluid, urine, saliva, stool, mucous, and sweat). The genetic lesion may comprise a nucleotide transition or transversion, a nucleotide insertion or deletion, a genomic rearrangement, a change in copy number, or a gene fusion.

In some embodiments, disclosed herein are methods of predicting, diagnosing, or monitoring a cancer in a subject comprising: (a) isolating or obtaining a sample of circulating tumor DNA ("ctDNA") from a subject; (b) treating the 5' end or 3' end or both of ctDNA with one or more end-repair enzymes to generate end-repaired ctDNA; (c) ligating one or more adaptors, wherein the adaptors comprise a restriction enzyme site and a sequence from a respiratory syncytial virus (RSV) (*i.e.,* the adaptors comprise a restriction enzyme site and sequences that would not be expected in cfDNA such as those from respiratory syncytial virus (RSV)), to each end of the end-repaired ctDNA to generate a ctDNA library; (d) amplifying the ctDNA library to generate a ctDNA library clone; (e) digesting the ctDNA library clone with a restriction enzyme to generate a modified ctDNA clone library; wherein the digesting the ctDNA library clone with the restriction enzyme removes the one or more adaptors; (f) purifying the modified ctDNA clone library; and (h) performing a quantitative analysis of one or more target loci in the modified ctDNA library clone, wherein the identification or detection of one or more somatic alterations in the one or more target loci is prognostic for, diagnostic of, or monitors the progression of the cancer. In some examples, the amplification of the cfDNA library is stopped prior to the amplification reaching a plateau. In some embodiments, at least 50% of the sequences in the modified cfDNA library clone have between 150 and 200 bp of the same cfDNA sequence as the end-repaired cfDNA. The cfDNA may isolated from a biological sample (*e.g.*, amniotic fluid, blood, plasma, serum, semen, lymphatic fluid, cerebral spinal fluid, ocular fluid, urine, saliva, stool, mucous, and sweat). The somatic alteration may be a genomic alteration, epigenomic alteration, point mutation, rearranged genomic sequence, copy number variant (CNV), microsatellite instability (MSI), loss of heterozygosity (LOH), or DNA methylation. In some embodiments, disclosed herein are methods of validating a diagnostic test for analysis of a sample of circulating cell-free DNA, comprising performing the diagnostic test on a control or a reference material disclosed herein, wherein the diagnostic test is validated if it correctly identifies the genotype of the control or the reference material. The sample of circulating cell-free DNA may be cell-free circulating tumor DNA ("ctDNA").

In some embodiments, disclosed herein are methods wherein the control or reference material is not mixed.

In some embodiments, the control or reference material is derived from a subject that is a donor (*e.g.,* a donor who is pregnant, or has a cancer).

In some embodiments, disclosed herein are methods related to generating a cfDNA control or reference material, comprising obtaining cfDNA from a subject, amplifying the cfDNA by PCR, and digesting the amplified cfDNA with a restriction enzyme (*e.g.*, SapI). The methods may further comprise isolating the cfDNA from a biological sample of the subject. The biological sample may be selected from: amniotic fluid, blood, plasma, serum, semen, lymphatic fluid, cerebral spinal fluid, ocular fluid, urine, saliva, stool, mucous, sweat, or cell supernatant of cultured cells. In some embodiments, the subject has been diagnosed with a genetic disease and quantitative genetic analysis is used to identify or detect one or more genetic lesions that cause or are associated with the genetic disease. The genetic lesion may comprise a nucleotide transition or transversion, a nucleotide insertion or deletion, a genomic rearrangement, a change in copy number, or a gene fusion. In some embodiments, the genetic disease is cancer. The subject may be a fetus, or the subject may be pregnant. The pregnant subject may have a cancer. Quantitative analysis may be used to identify or detect one or more genetic variants or genetic lesions of one or more target genetic loci in fetal cfDNA. In some embodiments, the size distribution and genomic representation biases in the cfDNA from the subject are represented in an output sample. In some embodiments, the generated cfDNA control or reference material is used in a biological assay. The periodicity of the cfDNA or ctDNA peaks may be the same as the original sample. In some embodiments, the periodicity of the DNA peaks will be maintained at about 166 bps, 146 bps, 136 bps, 126 bps, and/or 116 bps.

In some embodiments, disclosed herein are methods, the methods further comprise encapsulating the purified modified cfDNA clone library in a liposome, lipid, or a protein. Such methods may be used to generate a commutable control or reference material.

In some embodiments, disclosed herein are methods of determining whether a sample comprises a genotype comprising, performing a diagnostic test on the sample; and performing the diagnostic test on a control disclosed herein, wherein the control comprises the genotype; wherein: the sample is found to comprise the genotype if the diagnostic test indicates that both the sample and the control comprise the genotype; the sample is found to not comprise the genotype if the diagnostic test indicates that the sample does not comprise the genotype but that the control comprises the genotype; and the diagnostic test is found to be inconclusive if the test indicates that the control does not comprise the genotype.

**EXEMPLIFICATION**

*Example 1: End Polishing and dA-Tailing*

Starting with an isolated cfDNA sample, or other DNA fragments (*e.g.*, sonicated DNA), the first step involves end polishing, which may also be referred to as end repair, as well as a second step of dA-tailing (Figure 1B). Both steps, end polishing and dA-tailing, may be performed simultaneously. dA-tailing is the addition of a dA base at the 3' end. End polishing and dA-tailing takes the starting sample and allows some amount to become usable for ligation to adapter molecules. End polishing and dA-tailing are common in NGS methods used for whole genome sequencing (WGS) and hybrid/capture approaches. Depending on the adapters that are used, dA-tailing may not be required.

During end polishing, the ends of the input material are made blunt. 5' overhangs are commonly filled in with a polymerase. 3' overhangs are commonly removed with a 3' to 5' exonuclease (*e.g.*, an enzyme with activity that is also present in proofreading polymerases.) End polishing may be omitted, but the efficiency of downstream steps may be lower.

During dA-tailing, a dA base is added to the 3' end. This step is performed in order to increase the efficiency of downstream adapter ligation when the adapter(s) has a dT overhang. Additionally, this step is performed in order to increase the specificity of downstream adapter ligation by inhibiting the ligation of two input molecules to each other. dA-tailing may be omitted, but the efficiency of downstream steps may be lower.

*Example 2: Adapter Ligation*

After dA-tailing, an adapter comprising a SapI enzyme recognition site as well, as a 3' dT base overhang, is ligated to the sample with a ligase or similar enzyme (Figure 1B). Such an overhanging base is not limited to a dT base, but may be any base or similar molecule that is compatible with the base added during dA-tailing and with downstream polymerase chain reaction (PCR) amplification steps. The recessed end on the other strand has a 5' phosphate in order to allow for ligation (Figure 1B).

One particular distinction of the methods disclosed herein is the dT overhang, since it occurs where the SapI enzyme will cleave. Thus, the dT base and the dA base that was added during dA-tailing are later removed. Consequently, a SapI digested sample consists essentially of no bases that originate from the adapter and also does not lose any of the bases that remain after end polishing, thereby preserving the same starting input cfDNA sample. The SapI restriction enzyme may be replaced by a different enzyme. In such cases, it is necessary to adjust the sequence of the adapter accordingly so that the sample consists of the same starting cfDNA sample. If adapter ligation does not include dA-tailing, then it is still necessary to obtain a 1 base offset between the SapI site and the cfDNA so that none of the cfDNA bases are lost during subsequent SapI cleavage.

The adapter itself may contain additional sequences that enable PCR amplification. The adaptor may comprise sequences form a Y-style adapter wherein the sequences are not 100% complementary (Figure 1C). Other forms of adapters may also be used (*e.g.*, hairpin). The additional sequences are chosen so that the complementary sequences starting with the SapI site are sufficiently long in order to keep the part that is ligating in a double stranded conformation. The remaining sequences of the adaptor are selected to allow for PCR amplification. Since the amplified cfDNA may be used in DNA sequencing assays, such as where the ends of cfDNA are used to assign each cfDNA molecule to a chromosome (*e.g.*, in noninvasive prenatal screening), additional sequences comprising non-human nucleotides are selected. In some embodiments, the additional adapter sequence is derived in part from non-integrating RNA virus (respiratory syncytial virus (RSV)) sequences that would not be expected in assays of DNA. The adapter may be comprised of the following two single stranded DNA molecules:

Table 1: Adaptor Sequences

| Sequence Identification Number | Sequence (5'to 3') |
|---|---|
| SEQ ID NO: 1 | GAAGAGCCCTATATGGGATGATCTGACCGTCGT |
| SEQ ID NO:2 | TGATTTTGCCTGGCGTGTTGTATAGGGCTCTTCT |

The 5' end of the sequence may be modified (*e.g.*, the 5' end may be phosphorylated) (Figure 1C). Such single stranded DNA molecules may be purified in order to select those sequences that are likely to be of full length.

After ligation, the cfDNA (or other DNA) is now present in a library for amplification. This library may contain the same starting DNA that was used for input or a subset of this DNA.

Adapters may be added in molar excess to the expected number of cfDNA ends in order to ensure that there is sufficient adapter present to ligate to both ends of the input DNA. The library is then purified in order to remove smaller DNA molecules, such as unincorporated adapters. Presently, purification conditions may use glass fiber spin columns and guanidine thiocyanate for binding. Other purification conditions may also be used.

*Example 3: Removal of Large DNA molecules*

During the initial isolation of cfDNA, there may be contamination with genomic DNA that is released by lysed cells (*e.g.,* as a result of sample collection) or with longer than desired DNA molecules. This may later manifest itself as a population of larger than expected DNA molecules. The library may then be processed in order to remove large molecules. For example, the population of larger than expected DNA molecules may be removed by the addition of AMPure XP beads (*e.g.*, added at 0.5x volume to the purified library) (Figure 2A). The optimum amount of AMPure XP beads may be different from lot to lot, and must be established for the (chosen) method. Agarose gel purification may also be performed in order to select DNA molecules of specific lengths.

For example, Figure 2A shows an initial titration with DNA ladder performed in order to determine the appropriate concentration and remove larger molecules while retaining smaller molecules. Desired ccfDNA have lengths of below 500 bp. With the added adapters, this leads to a desired recovery of molecules below about 570 bp in the library. This was observed with AMPure XP beads added at approximately 0.5x the volume of DNA. Figures 2B and 2C show Bioanalyzer traces of two different samples of cfDNA. Figure 2B shows a Bioanalyzer trace of a library that was not processed to remove large DNA molecules. Figure 2C shows a Bioanalyzer trace of a library that was processed to remove large DNA molecules.

*Example 4: Library Amplification*

PCR is used to amplify the library (Figure 3A). Ideally, the majority of the library is amplified at once using a proofreading thermostable polymerase in order to preserve the complexity found in the library. Proofreading is essential in order to minimize the likelihood of introducing sequence errors during amplification. Especially in NGS assays that are used in Oncology, sequence errors may be misinterpreted as mutations found in cancer. For the experiments in Figures 4A and 4B, Agilent PfuUltra II hotstart master mix was used as the proofreading thermostable polymerase.

Initially, using an aliquot of the library, an optimum number of PCR cycles for amplification is established that considers the desired amount of product, such as where additional product requires that the library be split into more reactions, and the available amount of input sample. Using too few PCR cycles results in reduced yield. Using too many PCR cycles results in a sample that may not be suitable for SapI digestion and downstream analyses.

The optimum number of PCR cycles for library amplification may be established using a 2-fold serial dilution of the library because each additional PCR cycle is expected to lead to about a 2-fold amplification of PCR product during the exponential amplification phase. A fixed number of PCR cycles was performed and the concentration of double-stranded DNA in each reaction is determined. The dilution where the amplification efficiency starts to go down is used to determine the optimum number of cycles. Typically, a reduction in amplification efficiency occurs when the concentration of PCR products is such that the complementary adapter-derived ends anneal more quickly than the PCR primers (Figure 3B) and was observed to occur when there is more than about 8 ng/$\mu$l of PCR product in a reaction - although, this is dependent on reaction conditions. This may lead to the formation of partially double-stranded annealed amplified DNA molecules where the ends are complementary but the sequences between them are not.

Such mixed molecules may not be suitable for SapI digestion due to a lack of complementarity where the enzyme normally cuts (Figure 3B). Even if SapI digestion is successful, the released single-stranded DNA molecules would not be compatible with the NGS-based assays that rely on adapter ligation to double-stranded DNA molecules.

In Figures 4A and 4B, 12 cycles of PCR were performed for library amplification. Each PCR reaction consisted of a PfuUltra II master mix (at 1x) and each primer had a concentration of 500 nM. At the highest concentration, 1 $\mu$l of a given library was present in a 50 $\mu$l reaction. After PCR, the concentration of a 5 $\mu$l (of 50 $\mu$l) sample was determined using a Qubit dsDNA BR assay. The optimal amount of product per 50 $\mu$l reaction is approximately 100 to 400 ng. Above this range, the amplification efficiency becomes lower. For example, in the case of the T13 and T18 samples, the amount of DNA above 1 is no longer -twice the value above 0.5. (See Figures 4A and 4B)

Once the optimum number of cycles is determined, the library is amplified using PCR. In order to facilitate the later removal of adapters after SapI digestions, the PCR primers are labeled with a tag, such as biotin, that enables the removal of adapters. Other tags besides biotin may be used. Additionally, tags are not necessary and DNA may be purified by size or other methods after SapI digestion.

The primers that are used for PCR may be purified in order to ensure that they are full length and contain any desired tag. Additionally, the 5' ends of the primers may be modified in order to limit their ability to be ligated to other DNA or RNA molecules. In Figures 4A and 4B, the primers were not biotinylated at their 5' ends, as this is not necessary to establish the optimal number of cycles for amplification. The primers may be phosphorothioated between the four bases at the 5' end in order to limit 5' to 3' nuclease activity. The following two primer sequences are compatible with the described methods:

Table 2: PCR Primers

| Sequence Identification Number | Sequence (5'to 3') |
|---|---|
| SEQ ID NO:3 | ACGACGGTCAGATCATCCCA |
| SEQ ID NO:4 | TGATTTTGCCTGGCGTGTTG |

The 5' end of SEQ ID NO:3 and SEQ ID NO:4 may undergo biotinylation. SEQ ID NO:3 and SEQ ID NO:4 may be phosphorothioated. For example, SEQ ID NO:3 may be the following, where "/5Biosg/" specifies the 5' biotin and "*" specify the locations where the primers are phosphorothioated:

Table 3: Modified PCR Primers

| Sequence Identification Number | Sequence (5'to 3') |
|---|---|
| SEQ ID NO:5 | -/5Biosg/A*C*G*ACGGTCAGATCATCCCA- |
| SEQ ID NO:6 | -/5Biosg/T*G*A*TTTTGCCTGGCGTGTTG- |

Other suitable primers may also be designed for the methods disclosed herein.

After PCR, the library is purified in order to remove the polymerase and any unincorporated input materials. Additionally, purification conditions are chosen in order to minimize the recovery of smaller DNA molecules such as primer dimers. Such purification may not recover all of the desired output and/or may not remove all of the undesired species. Such purification may be performed with glass fiber filters and guanidine thiocyanate chemistry. Other suitable purification methods may also be used.

Some of the amplified library is retained in order to allow for future re-amplification. Ideally, a larger amount (mole basis) of amplified library should be retained than was used to make the library (i.e., if a library was made from 100 ng of amplifiable cfDNA, then more than 100 ng of amplified material should be retained). Such a step is performed to preserve, as much as possible, the complexity located in the library. For this reason, any re-amplification of the library should, if possible, amplify all of the retained library. The retained library may be split into many separate PCR reactions and pooled afterwards.

*Example 5: SapI Digestion*

Purified library PCR products are next quantified and subjected to a restriction digest using an appropriate amount of SapI enzyme (Figure 5). Other enzymes may be appropriate for the adapters and appropriate reaction conditions. Purification of the PCR library allows for unwanted ends to dissociate from the amplified material.

Approximately 1 in 8192 random bases will contain a SapI restriction site. SapI has a 7 base recognition sequence (GCTCTTC), and the presence of a single SapI recognition sequence is expected to occur once in every $4^7 = 16,384$ bases on a given strand of DNA. Since this recognition sequence is not a palindrome, it may also be encountered on the other strand of DNA. For a random DNA sequence, a SapI site is expected to be found about once every 8,192 bases. Thus, since cfDNA has a reported length of approximately 180 base pairs, SapI is expected in the sequence in about one every 40 to 50 cfDNA molecules, so there will be minimum internal cutting of the amplified material. Depending on the enzyme, enzymatic digestion of the purified PCR library results in a overhang for the amplified material or a blunt end. For example, cutting with the SapI enzyme may result in a 3 base 5' overhang. A 3' overhang may result in the amplified material having at least one foreign base added to the amplified material. The foreign base may be removed in downstream assays. By using an enzyme with a rarer recognition sequence, than is the case for SapI, the likelihood for this may be reduced. After the restriction digest, the enzyme may be heat inactivated at 65°C , or inactivated or removed by any one of a number of standard alternative methods, such as guanidine thiocyanate purification or the addition of proteinase K or another proteinase. The DNA at this stage may be purified; for example, to remove smaller molecules such as dissociated ends. Additionally, methods other than SapI may be used to remove adapters.

*Example 6: Final Purification*

In order to remove the dissociated ends, the SapI digest library may be exposed to streptavidin-coated magnetic beads since the dissociated ends were previously labeled with biotinylated primers. A sufficient amount of beads should be used to ensure that essentially all biotinylated molecules are removed.

Such purification is also likely to remove PCR products not digested with SapI enzyme. This situation may occur due to random reannealing of the ends of PCR products.

Based on Qubit dsDNA BR assay measurements, the amount of recovered product is approximately 50% of the amount of purified PCR product. A theoretical recovery of approximately 67% would be expected, suggesting that SapI digestion does not proceed to completion and suggesting that some molecules may not be digested (i.e., due to the presence of random reannealed PCR products).

Figure 6A show gels and the difference in size between amplified libraries and SapI digests (left = T13, T18 and T21 libraries where the input was sonicated cell line DNA; right = amplified cfDNA; "PCR" is the PCR reaction before purification; "purified" = the PCR reaction after guanidine thiocyanate purification; "SapI" is the SapI digest of the purified PCR reaction after adapter removal).

The final purified SapI digests are now ready for quantitative assays that analyze cfDNA.
For example, the sample may undergo additional end polishing as the first step in many hybrid/capture-based NGS assays where the full sequence of the cfDNA is restored (Figure 6B). Additionally, this end polishing step depicted in Figure 6B could be performed as part of manufacture of amplified cfDNA.

*Example 7: An Example of Quantitative Sample Analysis*

An initial set of two amplified ccfDNA samples was sent to a testing lab for analysis with the testing lab's NIPT assay (Figure 7). One sample was composed of amplified ccfDNA from a non-pregnant woman. Such a sample should appear as a normal female fetus using the assay. The other sample was similar but contained additional sonicated genomic DNA from a male aneuploid cell line at ~10% molar amount. This sample should appear as a male aneuploid fetus using the assay. These samples (blue and orange dots) in Figure 7 were analyzed along with other samples that included the testing lab's own samples (grey dots) and reference materials that had been produced using sonicated genomic DNA (green dots). The results show that, as has been observed previously, cell-derived sonicated DNA exhibits anomalies such as lower than expected values for sequences from the X chromosome (NCV X; NCV ≈ Z-score), which can make it appear as if the simulated fetus has Turner syndrome. On the other hand, the amplified ccfDNA samples were normal in regard to the amount of X and Y chromosome and were called correctly as female and male fetuses (Figure 7).

Interestingly, the amount of added sonicated material was not sufficient to have the aneuploidy detected (Figure 8). The efficiency with which sonicated material is incorporated into libraries is significantly lower than the efficiency with which ccfDNA or enzymatically-digested material is incorporated into libraries, as reflected in the results in Figure 8. Therefore, any material that is mixed with amplified ccfDNA should be subjected to similar processes, such as amplification and SapI digestion, in order to have similar efficiencies of detection with some assays. As shown in Figure 8 in this particular assay, one of the amplified ccfDNA samples (red circle; left) was nearly above an NCV value of 3 for chromosome 21 and has a higher NCV value for chromosome 21 than the sample without the sonicated aneuploid DNA (red circle; right). The other NCV values for chromosomes 13 and 18 (thin red circles) are lower. The other samples that were submitted to the testing lab are on the left of the chart (sonicated genomic DNA-derived) and the testing samples follow those and illustrate a typical spread of NCV values.

## Example 8A: Using Sonication Prior to Library Preparation

A mixture was obtained of approximately 150 ng/$\mu$l GM24385-derived genomic DNA and plasmids that should yield variant frequencies of approximately 0.625% for BRAF V600E, EGFR E746_A750delELREA, EGFR D770_N771insG, EGFR T790M, KIT D816V, KRAS G12D, NRAS Q61R, PIK3CA H1047R and PIK3CA N1068fs*4. In order to generate fragments of this material, two 50 $\mu$L aliquots of this mixture were sonicated for 250 seconds each with a Covaris M220 sonicator and screw-cap microTUBEs using settings of 50W peak incident power, 20% duty factor, 200 cycles per burst and 20 °C temperature.

While cfDNA is generally only present at relatively low amounts (5 ng per mL of plasma) and analyzed at low amounts, a large amount of starting material was needed (10 $\mu$g) in order to have sufficient complexity in the amplified library. This is because of binomial distributions - where the variance of variant frequencies is proportional to the number of copies - and the need to maintain variant frequencies at close to their starting variant frequencies. For example, assuming 3.5 pg of DNA per haploid human genome, 10 ng of DNA would contain about 2,857 haploid human genomes. Therefore, in a 10 ng aliquot of the GM24385/plasmid mix, a 0.625% variant is expected to be present between 0.350% and 0.945%, 95% of the time as calculated in Excel using =BINOM.INV(2857,0.00625,0.025)/2857 and =BINOM.INV(2857,0.00625,0.975)/2857, respectively. Thus, with 10 ng of starting material, two 0.625% variants could end up differing from one another by more than a factor of 2. Given that only 5% of sonicated DNA may be converted into a library for amplification, a 0.625% variant in 10 ng of starting material could be present at 0% (absent) or 2% in the amplified library. Additional losses of the library are expected due to size selection of the library. With 10 $\mu$g of starting material, 5% library incorporation efficiency and 10% recovery after size selection, a 0.625% variant is expected to be present between 0.5% and 0.76%, 95% of the time. Similarly, a 0.1% variant is expected to be present between 0.05% and 0.15%, 95% of the time.

## Example 8B: Library Synthesis from the Sonicated Material

67 $\mu$L (~10 $\mu$g) of the sonicated DNA was subjected to end repair and dA-tailing by adding 70 $\mu$l of NEBNext Ultra II End Prep Reaction Buffer, 433 $\mu$L of water and 30 $\mu$L of NEBNext Ultra II End Prep Enzyme Mix and incubating the ~600 $\mu$L at ~20 °C for 30 minutes. The enzymes in the mixture were heat inactivated by placing the mixture at ~65 °C for 40 minutes. After cooling the mixture, 80 $\mu$L of 200 $\mu$M adapters, 10 $\mu$L of NEBNext Ligation Enhancer and 300 $\mu$L of NEBNext Ultra II Ligation Master Mix were added. The reaction was incubated at ~20 °C for 20 minutes and stored frozen until DNA was extracted using guanidine thiocyanate-based DNA extraction.

## Example 8C: Size-Selection of the Library

The cfDNA library was then separated on an agarose gel (Figure 9) and adapter-containing cfDNA was subsequently excised (Figure 10). Approximately 835 ng of DNA were recovered at approximately 8.35 ng/$\mu$l as measured by Nanodrop. Assuming 5% library incorporation efficiency, 8.35 ng/$\mu$l should correspond to about 0.42 ng/$\mu$L of amplifiable library.

## Example 8D: Determining the Optimal Amount of Amplification

Using approximately 0.4 $\mu$L of library per 20 $\mu$l PCR reaction (0.167 ng/$\mu$l), and 2-fold serial dilutions thereof so that 8 different starting concentrations were obtained (the lowest concentration was analyzed in duplicate), 14 cycles of PCR were performed. About 0.4 $\mu$l of library is expected to result in a starting concentration of ~8.4 pg/$\mu$l of amplifiable library that, when amplified 2^14 = 16,384-fold could result in up to 138 ng/$\mu$l of product. This exceeds by far the maximum expected yield, and only the 5th 2-fold dilution is expected to result in < 10 ng/$\mu$l. However, because library incorporation efficiency may be < 5%, but may also be > 5%, a range of starting concentrations was used in order to determine the optimal number of cycles for amplification.

Qubit BR dsDNA analyses of the PCR reactions are shown in Figures 11A and 11B. Exponential amplification was observed for the 5th dilution - which resulted in 7.0 ng/μL - through the 8th dilution. At 100% amplification efficiency, this corresponds to a library incorporation efficiency of ~4%. The amplified material was also analyzed on a 2% agarose gel (between ladders; NTC is to the right of the left ladder) and compared to amplified cfDNA (leftmost lane). The characteristic lower 2 bands of cfDNA were observed in the amplified ctDNA.

*Example 8E: Library Amplification*

In order to maintain the complexity of the library and the frequency of the variants therein (Figure 3C), it is essential in this method to amplify a large amount of the library. Having determined the concentration of amplifiable library, a mixture was prepared using 91 μL of the library, 12 μL of each 100 μM biotinylated primer and 1,085 μL of water and the 1,200 μl mixture was split into two 600 μl aliquots. Into each aliquot, 600 μl of Agilent PfuUltra II hotstart master mix were added. Thus 91 μL * 8.35 ng/μl * 4% = 30.4 ng of starting amplifiable library was present in 2.4 mL. In order to obtain 8 ng/μL of PCR product, 8 ng/μl * 2,400 μl = 19,200 ng of PCR product were needed from 30.4 ng of input. This 632-fold amplification was expected to require ln(632)/ln(2) = 9.3 cycles of PCR. In order to not exceed 8 ng/μl, 9 cycles of PCR were then used to amplify the library. The expected yield was 30.4 * 512 = 15,565 ng.

The following PCR protocol was carried out with the 2,400 μL split into 48 separate 50 μl reactions: 2 min 95 °C, 9 cycles of 20 sec 95 °C, 30 sec 50 °C, 45 sec 72 °C, followed by 3 min 72 °C. After PCR, a DNA concentration of 6.40 ng/μL was obtained by Qubit BR dsDNA assay. This corresponded to a yield of 6.40 ng/μL * 50 μL * 48 = 15,360 ng, which was very close to the expected yield.

The amplified library was extracted using guanidine thiocyanate-based DNA extraction. A final DNA elution volume of ~375 μL resulted in a dsDNA concentration of 21.8 ng/μl by Qubit BR dsDNA assay, which corresponded to an amount of 375 μl * 21.8 ng/μl = 8,175 ng.

*Example 8F: SapI Digestion*

In order to remove the primers that were used to amplify the library, 92 μl of amplified library (~2 μg) were combined with 11.3 μL of 10x CutSmart buffer and 10 μL of 10 U/μl SapI restriction enzyme. The reaction was incubated for 4 hours at 37 °C, heat inactivated for 20 minutes at 65 °C and placed at 4 °C. Subsequently, exposed the digested material to hydrophilic magnetic streptavidin beads in order to remove biotinylated molecules (residual primers, ends of amplified molecules that were released by the digests, undigested amplified molecules, etc.). After recovering the unbound material (~105 μl), the volume was adjusted to 200 μl and a concentration of 4.40 ng/μl was obtained by Qubit BR dsDNA assay. These -0.88 μg corresponded to a recovery of ~44% of the 2 μg input material.

*Example 8G: External Testing*

Some purified SapI digested samples were shipped to a testing lab for analysis. Some samples sent for testing were a mixture of amplified ccfDNA from a single non-pregnant female donor (majority of the DNA in each sample) and amplified sonicated DNA from three different trisomic cell lines (minority of the DNA in each sample; the "fetal fraction"). Four amplified libraries were prepared and each of the four amplified libraries (1x ccfDNA from donor; 3x sonicated DNA from trisomic cell lines) were digested separately with SapI in order to separate the flanking biotinylated adapters from the amplified sequences and then purified separately in order to reduce the concentration of the freed biotinylated adapters and DNA molecules that may not have been digested fully (and still contain biotinylated adapters) in the mixtures. The resulting four mixtures were quantified for DNA concentration and mixed. The presence of the trisomic cell line-derived sequences was detected within the background of donor sequences.

In addition, seven purified SapI digested samples were shipped to a testing lab for analysis, and all seven variants that were expected to be detected as a variant were actually detected as a variant at or near the expected variant frequency of 0.625%. See Table 1.

Table 1

| Description | GEs | Avg Depth | Mutation | Position | Frequency |
|---|---|---|---|---|---|
| proto1 | 5280 | 4691 | BRAF V600E | chr7:140453136 | 0.008 |
| | | | EGFR ELREA746- | chr7:55242465 | 0.007 |
| | | | EGFR N771GN | chr7:55249012 | 0.008 |
| | | | EGFR T790M | chr7:55249071 | 0.005 |
| | | | KRAS G12D | chr12:25398284 | 0.007 |

(continued)

| Description | GEs | Avg Depth | Mutation | Position | Frequency |
|---|---|---|---|---|---|
| proto1 | 5280 | 4691 | BRAF V600E | chr7:140453136 | 0.008 |
| | | | NRAS Q61R | chr1:115256529 | 0.008 |
| | | | PIK3CA H1047R | chr3:178952085 | 0.006 |
| | | | | | |
| proto2 | 5890 | 4794 | BRAF V600E | chr7:140453136 | 0.007 |
| | | | EGFR ELREA746- | chr7:55242465 | 0.006 |
| | | | EGFR N771GN | chr7:55249012 | 0.006 |
| | | | EGFR T790M | chr7:55249071 | 0.007 |
| | | | KRAS G12D | chr12:25398284 | 0.009 |
| | | | NRAS Q61R | chr1:115256529 | 0.007 |
| | | | PIK3CA H1047R | chr3:178952085 | 0.006 |

*Applications*

cfDNA may be analyzed in the area of oncology to determine whether any mutations are present within a subject's cfDNA sequence that are predictive of cancer, or cfDNA sequences that may be used to select an appropriate treatment or prognosis. Since it is well established that cfDNA is limited, cfDNA isolated from blood may be insufficient for multiple tests. Additionally, cfDNA isolated from blood may be insufficient to allow for a broad comparison of tests, such as the case during proficiency testing. The amplification of cfDNA, as disclosed herein, allows for the amplification of cfDNA from a limited amount of representative starting sample ("input DNA"), and analyze the library PCR product using a multitude of different assays. As disclosed herein, in order to reduce the likelihood of introducing potential mutations through PCR amplification of cfDNA, a proofreading polymerase should be incorporated into the PCR.

The amplified cfDNA library product may be analyzed in noninvasive prenatal tests (NIPT) or screens (NIPS) to assess the genetic makeup of the fetus since fetal DNA may often be found in the maternal cfDNA after about 9 weeks of gestation. Given the recognized premise that pregnant women are protected subjects and any medical procedures should be a non-significant risk to the mother and fetus, it is therefore only possible to obtain limited volumes of blood, such as 1,2,3, 4 or 5- 10 mL tubes worth. As a result, using current methods, it is only possible to obtain limited amounts of cfDNA.

Current methods of preparing cfDNA-like material typically sonicate input DNA. As mentioned earlier, sonication may introduce several artifacts that may cause some assays to fail. For example, the fetal component of cfDNA may have a different relative genomic representation than that of maternal cfDNA or sonicated genomic DNA. The fetal component of cfDNA may also have a different size distribution than that of maternal cfDNA or sonicated genomic DNA. In those instances, sonication may cause a different representation of the maternal and fetal cfDNA than would otherwise be present in a typical input sample. Therefore, as disclosed herein, amplified cfDNA presents a solution that allows a limited amount of cfDNA to be analyzed on multiple platforms and allows amplification from a limited amount of donor input cfDNA to generate large quantities for reference materials and proficiency testing samples. The methods disclosed herein may be used with sonication or without sonication. For example, after giving birth, the mother is no longer a protected subject and is generally free to donate larger volumes of blood. At this point, additional blood, which is now free of fetal-derived cfDNA, may be collected to obtain cfDNA and reduce the apparent fetal fraction in previously-collected cfDNA. This blood may also be used to obtain cells that are used by some assays (*e.g.*, SNP-based assays) to determine the genetic profile of the mother. cfDNA may also be analyzed from grafts in the circulation of transplant recipients as potential biomarker of rejection.

**INCORPORATION BY REFERENCE**

All of the patents, patent application publications, and other references cited herein are hereby incorporated by reference.

**EQUIVALENTS**

Those skilled in the art will recognize, or be able to ascertain using no more than routine experimentation, many equivalents to the specific embodiments of the invention described herein. Such equivalents are intended to be encompassed by the following claims. Aspects of the invention:

1. A control for use in identifying a genotype, comprising amplified cell-free DNA (cfDNA), and a first mixture of nucleic acids; wherein the cfDNA has a base pair (bp) length of about 75 bps to about 600 bps, the first mixture of nucleic acids comprises a nucleotide sequence that encodes the genotype, and the first mixture of nucleic acids constitutes at least 50% of double stranded DNA of about 75 bps to about 600 bps in length.

2. The control of aspect 1, wherein the first mixture of nucleic acids encodes substantially all of a human genome.

3. The control of aspect 1 or 2, further comprising a second mixture of nucleic acids comprising a nucleotide sequence that encodes a second genotype, wherein the genotype and the second genotype are alternate genotypes that occur at the same genetic locus.

4. The control of aspect 3, wherein the second mixture of nucleic acids encodes substantially all of a second human genome.

5. The control of aspect 3 or 4, wherein the ratio of the copy number of the nucleotide sequence that encodes the genotype to the copy number of the nucleotide sequence that encodes the second genotype is about 1:1000 to about 1000:1.

6. The control of any one of aspects 1-5, wherein the genotype is associated with a neoplasm.

7. The control of any one of aspects 1-5, wherein the genotype is associated with a provirus.

8. The control of any one of aspects 1-5, wherein the genotype is associated with a hereditary disease.

9. The control of any one of aspects 1-5, wherein the genotype is associated with a somatic mutation.

10. The control of aspect 9, wherein the genotype is a mutation to a gene selected from the group consisting of MTOR, MPL, NRAS, PARP1, AKT3, DNMT3A, MSH2, IDH1, VHL, MLH1, MYD88, CTNNB1, ATR, PIK3CA, FGFR3, PDGFRA, KIT, FBXW7, APC, GABRG2, NPM1, EGFR, MET, BRAF, EZH2, JAK2, GNAQ, RET, PTEN, ATM, KRAS, PTPN11, FLT3, RB1, PARP2, ARHGAP5, AKT1, RAD51, IDH2, TP53, NF1, SMAD4, AKT2, ERCC1, and GNAS.

11. The control of aspect 9, wherein the genotype is a mutation to a gene selected from the group consisting of AKT1, ATM, BRAF, CDKN2A, CSF1R, EGFR, ERBB2, ERBB4, FGFR1, FGFR2, FGFR3, GNA11, HRAS, JAK2, JAK3, KDR, KIT, KRAS, MET, NOTCH1, NRAS, PDGFRA, PIK3CA, PTEN, RET, and STK11.

12. The control of any one of aspects 1-5, wherein the genotype is associated with a virus or bacteria.

13. The control of any one of aspects 1-5, wherein the genotype is associated with a graft rejection.

14. The control of any one of aspects 1-5, wherein the second genotype is not associated with a disease.

15. The control of any one of aspects 1-9, wherein the genotype is a single nucleotide polymorphism, point mutation, premature stop codon, trinucleotide repeat, translocation, somatic rearrangement, allelomorph, single nucleotide variant, coding insertion or deletion ("indel"), splice variant, regulatory variant, copy number variant, or gene fusion.

16. A reference material for use in identifying a genotype, comprising amplified cell-free DNA (cfDNA), and a first mixture of nucleic acids; wherein the cfDNA has a base pair (bp) length of about 75 bps to about 600 bps, the first mixture of nucleic acids comprises a nucleotide sequence that encodes the genotype, and the first mixture of nucleic acids constitutes at least 50% of double stranded DNA of about 75 bps to about 600 bps in length.

17. The reference material of aspect 16, wherein the first mixture of nucleic acids encodes substantially all of a human genome.

18. The reference material of aspect 16 or 17, further comprising a second mixture of nucleic acids comprising a nucleotide sequence that encodes a second genotype, wherein the genotype and the second genotype are alternate genotypes that occur at the same genetic locus.

19. The reference material of aspect 18, wherein the second mixture of nucleic acids encodes substantially all of a second human genome.

20. The reference material of aspect 18 or 19, wherein the ratio of the copy number of the nucleotide sequence that encodes the genotype to the copy number of the nucleotide sequence that encodes the second genotype is about 1:1000 to about 1000:1.

21. The reference material of any one of aspects 16-20, wherein the genotype is associated with a neoplasm.

22. The reference material of any one of aspects 16-20, wherein the genotype is associated with a provirus.

23. The reference material of any one of aspects 16-20, wherein the genotype is associated with a hereditary disease.

24. The reference material of any one of aspects 16-20, wherein the genotype is associated with a somatic mutation.

25. The reference material of aspect 24, wherein the genotype is a mutation to a gene selected from the group consisting of MTOR, MPL, NRAS, PARP1, AKT3, DNMT3A, MSH2, IDH1, VHL, MLH1, MYD88, CTNNB1, ATR, PIK3CA, FGFR3, PDGFRA, KIT, FBXW7, APC, GABRG2, NPM1, EGFR, MET, BRAF, EZH2, JAK2, GNAQ, RET, PTEN, ATM, KRAS, PTPN11, FLT3, RB1, PARP2, ARHGAP5, AKT1, RAD51, IDH2, TP53, NF1, SMAD4, AKT2, ERCC1, and GNAS.

26. The reference material of aspect 24, wherein the genotype is a mutation to a gene selected from the group consisting of AKT1, ATM, BRAF, CDKN2A, CSF1R, EGFR, ERBB2, ERBB4, FGFR1, FGFR2, FGFR3, GNA11, HRAS, JAK2, JAK3, KDR, KIT, KRAS, MET, NOTCH1, NRAS, PDGFRA, PIK3CA, PTEN, RET, and STK11.

27. The reference material of any one of aspects 16-20, wherein the genotype is associated with a virus or bacteria.

28. The reference material of any one of aspects 16-20, wherein the genotype is associated with a graft rejection.

29. The reference material of any one of aspects 16-20, wherein the second genotype is not associated with a disease.

30. The reference material of any one of aspects 16-24, wherein the genotype is a single nucleotide polymorphism, point mutation, premature stop codon, trinucleotide repeat, translocation, somatic rearrangement, allelomorph, single nucleotide variant, coding insertion or deletion ("indel"), splice variant, regulatory variant, copy number variant, or gene fusion.

31. A control for use in identifying a plurality of genotypes, comprising a sample of amplified cfDNA, wherein the amplified cfDNA has a base pair (bp) length of about 75 bps to about 600 bps, and a first mixture of nucleic acids, wherein the first mixture of nucleic acids comprises a first plurality of nucleotide sequences, each nucleotide sequence of the first plurality encodes a genotype of the plurality of genotypes, and the first mixture of nucleic acids constitutes at least 50% of double stranded DNA of about 75 bps to about 600 bps in length.

32. The control of aspect 31, wherein each genotype of the plurality of genotypes consists of a mutation to a gene selected from the group consisting of MTOR, MPL, NRAS, PARP1, AKT3, DNMT3A, MSH2, IDH1, VHL, MLH1, MYD88, CTNNB1, ATR, PIK3CA, FGFR3, PDGFRA, KIT, FBXW7, APC, GABRG2, NPM1, EGFR, MET, BRAF, EZH2, JAK2, GNAQ, RET, PTEN, ATM, KRAS, PTPN11, FLT3, RB1, PARP2, ARHGAP5, AKT1, RAD51, IDH2, TP53, NF1, SMAD4, AKT2, ERCC1, and GNAS.

33. The control of aspect 31, wherein each genotype of the plurality of genotypes consists of a mutation to a gene selected from the group consisting of AKT1, ATM, BRAF, CDKN2A, CSF1R, EGFR, ERBB2 ("HER2"), ERBB4 ("HER4"), FGFR1, FGFR2, FGFR3, GNA11, HRAS, JAK2, JAK3, KDR, KIT, KRAS, MET, NOTCH1, NRAS, PDGFRA, PIK3CA, PTEN, RET, and STK11.

34. The control of aspect 31, wherein each genotype of the plurality of genotypes consists of a mutation to a gene selected from the group consisting of ABL1, AKT1, ALK, APC, AR, AR1D1A, ARAF, ATM, BCL2, BCR, BRAF, BRC42, BRCA1, BRCA2, BRIP1, CCND1, CCND2, CCNE1, CDH1, CDK4, CDK6, CDKN2A, CDKN2B, CSF1R, CTNNB1, DDR2, EGFR, ERBB2, ERBB3, ERBB4, ESR1, ETV1, ETV4, ETV6, EWSR1, EZH2, FANCA, FANCC, FANCD2, FANCE, FANCF, FANCG, FANCL, FBXW7, FGFR1, FGFR2, FGFR3, FLT3, FOXL2, GATA3, GNA11, GNAQ, GNAS, HER/ERBB2, HNF1A, HPAS, HRAS, IDH1, IDH2, IHD2, JAK2, JAK3, KDR, KIT, KRAS, MAP2K1, MAP2K2, MET, MLH1, MLL, MPL, MSH2, MSH6, MTOR, MYC, MYCN, NF1, NF2, NFE2L2, NOTCH1, NPM1, NRAS, NTRK1, PALB2, PDGFRA, PDGFRB, PIK3CA, PMS2, PTCH1, PTEN, PTPN11, RAFT, RARA, RB1, RET, RHEB, RHOA, RIT1, ROS1, SMAD4, SMARCB1, SMO, SRC, STK11, TERT, TMPRSS2, TP53, TSC1, TSC2, and VHL.

35. The control of aspect 31, wherein each genotype of the plurality of genotypes consists of a mutation to a gene selected from the group consisting of BRAF, EGFR, ERBB2, and KRAS.

36. The control of aspect 32, wherein each genotype of the plurality of genotypes consists of a mutation to a gene selected from the group consisting of mutation V600E to gene BRAF, mutation T790M to gene EGFR, mutation delL747-P753insS to gene EGFR, mutation A775_G776insYVMA to gene ERBB2, and mutation G12D to gene KRAS.

37. The control of aspect 31, wherein the plurality of genotypes comprises mutation V600E to gene BRAF, mutation T790M to gene EGFR, mutation delL747-P753insS to gene EGFR, mutation A775_G776insYVMA to gene ERBB2, and mutation G12D to gene KRAS.

38. The control of any one of aspects 31-37, further comprising a second mixture of nucleic acids, wherein the second mixture of nucleic acids comprises a second plurality of nucleotide sequences, and each nucleotide sequence of the second plurality is an alternate genotype that occurs at the same genetic locus as a nucleotide sequence of the first plurality.

39. A reference material for use in identifying a plurality of genotypes, comprising a sample of amplified cfDNA, wherein the amplified cfDNA has a base pair (bp) length of about 75 bps to about 600 bps, and a first mixture of nucleic acids, wherein the first mixture of nucleic acids comprises a first plurality of nucleotide sequences, each nucleotide sequence of the first plurality encodes a genotype of the plurality of genotypes, and the first mixture of nucleic acids constitutes at least 50% of double stranded DNA of about 75 bps to about 600 bps in length.

40. The reference material of aspect 39, wherein each genotype of the plurality of genotypes consists of a mutation to a gene selected from the group consisting of MTOR, MPL, NRAS, PARP1, AKT3, DNMT3A, MSH2, IDH1, VHL, MLH1, MYD88, CTNNB1, ATR, PIK3CA, FGFR3, PDGFRA, KIT, FBXW7, APC, GABRG2, NPM1, EGFR, MET, BRAF, EZH2, JAK2, GNAQ, RET, PTEN, ATM, KRAS, PTPN11, FLT3, RB1, PARP2, ARHGAP5, AKT1, RAD51, IDH2, TP53, NF1, SMAD4, AKT2, ERCC1, and GNAS.

41. The reference material of aspect 39, wherein each genotype of the plurality of genotypes consists of a mutation to a gene selected from the group consisting of AKT1, ATM, BRAF, CDKN2A, CSF1R, EGFR, ERBB2 ("HER2"), ERBB4 ("HER4"), FGFR1, FGFR2, FGFR3, GNA11, HRAS, JAK2, JAK3, KDR, KIT, KRAS, MET, NOTCH1, NRAS, PDGFRA, PIK3CA, PTEN, RET, and STK11.

42. The reference material of aspect 39, wherein each genotype of the plurality of genotypes consists of a mutation to a gene selected from the group consisting of ABL1, AKT1, ALK, APC, AR, AR1D1A, ARAF, ATM, BCL2, BCR, BRAF, BRC42, BRCA1, BRCA2, BRIP1, CCND1, CCND2, CCNE1, CDH1, CDK4, CDK6, CDKN2A, CDKN2B, CSF1R, CTNNB1, DDR2, EGFR, ERBB2, ERBB3, ERBB4, ESR1, ETV1, ETV4, ETV6, EWSR1, EZH2, FANCA, FANCC, FANCD2, FANCE, FANCF, FANCG, FANCL, FBXW7, FGFR1, FGFR2, FGFR3, FLT3, FOXL2, GATA3, GNA11, GNAQ, GNAS, HER/ERBB2, HNF1A, HPAS, HRAS, IDH1, IDH2, IHD2, JAK2, JAK3, KDR, KIT, KRAS, MAP2K1, MAP2K2, MET, MLH1, MLL, MPL, MSH2, MSH6, MTOR, MYC, MYCN, NF1, NF2, NFE2L2, NOTCH1, NPM1, NRAS, NTRK1, PALB2, PDGFRA, PDGFRB, PIK3CA, PMS2, PTCH1, PTEN, PTPN11, RAFT, RARA, RB1, RET, RHEB, RHOA, RIT1, ROS1, SMAD4, SMARCB1, SMO, SRC, STK11, TERT, TMPRSS2, TP53, TSC1, TSC2, and VHL.

43. The reference material of aspect 39, wherein each genotype of the plurality of genotypes consists of a mutation to a gene selected from the group consisting of BRAF, EGFR, ERBB2, and KRAS.

44. The reference material of aspect 43, wherein each genotype of the plurality of genotypes consists of a mutation to a gene selected from the group consisting of mutation V600E to gene BRAF, mutation T790M to gene EGFR, mutation delL747-P753insS to gene EGFR, mutation A775_G776insYVMA to gene ERBB2, and mutation G12D to gene KRAS.

45. The reference material of aspect 44, wherein the plurality of genotypes comprises mutation V600E to gene BRAF, mutation T790M to gene EGFR, mutation delL747-P753insS to gene EGFR, mutation A775_G776insYVMA to gene ERBB2, and mutation G12D to gene KRAS.

46. The reference material of aspect 39, wherein the plurality of genotypes comprises mutation V600E to gene BRAF, mutation T790M to gene EGFR, mutation delL747-P753insS to gene EGFR, mutation A775_G776insYVMA to gene ERBB2, and mutation G12D to gene KRAS.

47. The reference material of any one of aspects 39-46, further comprising a second mixture of nucleic acids, wherein the second mixture of nucleic acids comprises a second plurality of nucleotide sequences, and each nucleotide sequence of the second plurality is an alternate genotype that occurs at the same genetic locus as a nucleotide sequence of the first plurality.

48. A control for use in determining the ploidy of a chromosome in a fetus, comprising:

a first mixture of nucleic acids comprising a first nucleotide sequence and a second nucleotide sequence, wherein the first nucleotide sequence has sequence homology with the chromosome; the second nucleotide sequence has sequence homology with a different chromosome; and the ratio of the copy number of the first nucleotide sequence to the copy number of the second nucleotide sequence is greater than 1: 1;
a second mixture of nucleic acids comprising the first nucleotide sequence and the second nucleotide sequence, wherein the ratio of the copy number of the first nucleotide sequence to the copy number of the second nucleotide sequence is about 1:1; and
a sample of cfDNA, wherein the cfDNA has a base pair (bp) length of about 75 bps to about 600 bps, wherein the first mixture of nucleic acids constitutes at least 50% of double stranded DNA of about 75 bps to about 600 bps in length.

49. The control of aspect 48, wherein the different chromosome is human chromosome 1, 2, 3, 4, 5, 6, 7, 8, 9, 10, 11, 12, 13, 14, 15, 16, 17, 18, 19, 20, 21, 22, X, or Y.

50. The control of aspect 49, wherein the different chromosome is human chromosome 1, 6, or 7.

51. A reference material for use in determining the ploidy of a chromosome in a fetus, comprising:

a first mixture of nucleic acids comprising a first nucleotide sequence and a second nucleotide sequence, wherein the first nucleotide sequence has sequence homology with the chromosome; the second nucleotide sequence has sequence homology with a different chromosome; and the ratio of the copy number of the first nucleotide sequence to the copy number of the second nucleotide sequence is greater than 1:1;
a second mixture of nucleic acids comprising the first nucleotide sequence and the second nucleotide sequence, wherein the ratio of the copy number of the first nucleotide sequence to the copy number of the second nucleotide sequence is about 1:1; and
a sample of cfDNA, wherein the cfDNA has a base pair (bp) length of about 75 bps to about 600 bps, wherein the first mixture of nucleic acids constitutes at least 50% of double stranded DNA of about 75 bps to about 600 bps in length.

52. The reference material of aspect 51, wherein the different chromosome is human chromosome 1, 2, 3, 4, 5, 6, 7, 8, 9, 10, 11, 12, 13, 14, 15, 16, 17, 18, 19, 20, 21, 22, X, or Y.

53. The reference material of aspect 52, wherein the different chromosome is human chromosome 1, 6, or 7.

54. A control for use in determining the ploidy of a chromosome in a fetus, comprising:

a first mixture of nucleic acids comprising a first plurality of nucleotide sequences and a second plurality of nucleotide sequences, wherein the first plurality of nucleotide sequences has sequence homology with the chromosome; the second plurality of nucleotide sequences has sequence homology with at least one autosome, wherein the at least one autosome does not comprise the chromosome; and the ratio of the copy number for any nucleotide sequence in the first plurality to the copy number for any nucleotide sequence in the second plurality is about 3:2;

a second mixture of nucleic acids comprising the first plurality of nucleotide sequences and the second plurality of nucleotide sequences, wherein the ratio of the copy number for any nucleotide sequence in the first plurality to the copy number for any nucleotide sequence in the second plurality is about 1:1; and

a sample of cfDNA, wherein the cfDNA has a base pair (bp) length of about 75 bps to about 600 bps, wherein the first mixture of nucleic acids constitutes at least 50% of double stranded DNA of about 75 bps to about 600 bps in length.

55. The control of aspect 54, wherein each of the at least one autosome is independently selected from the group consisting of human chromosome 1, 2, 3, 4, 5, 6, 7, 8, 9, 10, 11, 12, 13, 14, 15, 16, 17, 18, 19, 20, 21, and 22.

56. The control of aspect 55, wherein each of the at least one autosome is independently selected from the group consisting of human chromosome 1, 6, and 7.

57. The control of any one of aspects 54-56, wherein the chromosome is human chromosome 1, 2, 3, 4, 5, 6, 7, 8, 9, 10, 11, 12, 13, 14, 15, 16, 17, 18, 19, 20, 21, 22, X, or Y.

58. The control of aspect 57, wherein the chromosome is human chromosome 8, 9, 13, 18, 21, 22, or X.

59. The control of any one of aspects 48-50, and 54-58, wherein the first mixture of nucleic acids or the second mixture of nucleic acids encodes substantially all of a human genome.

60. The control of any one of aspects 48-50 and 54-58, wherein the first mixture of nucleic acids and the second mixture of nucleic acids encodes substantially all of a human genome.

61. The control of aspect 59, wherein the human genome is an immortalized lymphocyte cell line genome, a fibroblast cell line genome, or a cytotrophoblast cell line genome.

62. The control of aspect 59, wherein the human genome is a peripheral blood mononuclear cell genome.

63. The control of aspect 59, wherein the human genome is a lymphocyte, fibroblast, placenta, or adipocyte genome.

64. The control of any one of aspects 48-50 and 54-60 , wherein the first mixture of nucleic acids or the second mixture of nucleic acids comprise mitochondrial nucleotide sequences.

65. The control of any one of aspects 48-50 and 54-60, wherein the first mixture of nucleic acids and the second mixture of nucleic acids are free of mitochondrial nucleotide sequences.

66. The control of any one of aspects 48-50 and 54-62, wherein the first mixture of nucleic acids or the second mixture of nucleic acids comprise methylated DNA.

67. The control of any one of aspects 48-50 and 54-62, wherein the first mixture of nucleic acids and the second mixture of nucleic acids are free of methylated DNA.

68. The control of any one of aspects 48-50 and 54-64, wherein the first mixture of nucleic acids or the second mixture of nucleic acids are derived from a plasmid.

69. The control of any one of aspects 48-50 and 54-65, wherein the ratio of the concentration of nucleic acids in the first mixture to the concentration of nucleic acids in the second mixture is about 1:1000 to about 1:1.

70. The control of aspect 66, wherein the ratio of the concentration of nucleic acids in the first mixture to the concentration of nucleic acids in the second mixture is about 1:200 to about 1:3.

71. The control of any one of aspects 1-15, 31-38, and 48-70, wherein the nucleic acids in the first mixture make up about 5% to about 15% of the total concentration of nucleic acids in the control.

72. The control of any one of aspects 1-15, 31-38, and 48-71, wherein the median length of the nucleic acids in the control is about 35 base pairs to about 1000 base pairs.

73. The control of aspect 72, wherein the median length of the nucleic acids in the control is about 50 base pairs to about 500 base pairs.

74. The control of any one of aspects 1-15, 31-38, and 48-73, wherein the cfDNA is isolated from a biological sample selected from: amniotic fluid, blood, plasma, serum, semen, lymphatic fluid, cerebral spinal fluid, ocular fluid, urine, saliva, stool, mucous, sweat, or cell supernatant of cultured cells.

75. The control of any one of aspects 1-15, 31-38, and 48-74, wherein the sample of cfDNA is cell-free circulating tumor DNA ("ctDNA").

76. The control of any one of aspects 1-15, 31-38, and 48-75, wherein the nucleic acids of the control do not form aggregates when stored at a temperature of about 0°C to about 100°C.

77. The control of any one of aspects 1-15, 31-38, and 48-76, wherein the nucleic acids of the control do not form aggregates when stored for a period of time from about 1 day to about 5 years.

78. The control of any one of aspects 1-15, 31-38, and 48-77, wherein the concentration of nucleic acids in the control is about 1 ng/mL to about 100 ng/mL.

79. The control of any one of aspects 1-15, 31-38, and 48-78, wherein the concentration of nucleic acids in the control is greater than 100 ng/mL.

80. The control of any one of aspects 1-15, 31-38, and 48-79, wherein the control is encapsulated by a liposome, lipid or protein to generate a commutable control.

81. The commutable control of aspect 80, wherein the control has at least 80% sequence similarity to a natural analyte.

82. A reference material for use in determining the ploidy of a chromosome in a fetus, comprising:

a first mixture of nucleic acids comprising a first plurality of nucleotide sequences and a second plurality of nucleotide sequences, wherein the first plurality of nucleotide sequences has sequence homology with the chromosome; the second plurality of nucleotide sequences has sequence homology with at least one autosome, wherein the at least one autosome does not comprise the chromosome; and the ratio of the copy number for any nucleotide sequence in the first plurality to the copy number for any nucleotide sequence in the second plurality is about 3:2;
a second mixture of nucleic acids comprising the first plurality of nucleotide sequences and the second plurality of nucleotide sequences, wherein the ratio of the copy number for any nucleotide sequence in the first plurality to the copy number for any nucleotide sequence in the second plurality is about 1:1; and
a sample of cfDNA, wherein the cfDNA has a base pair (bp) length of about 75 bps to about 600 bps,
wherein the first mixture of nucleic acids constitutes at least 50% of double stranded DNA of about 75 bps to about 600 bps in length.

83. The reference material of aspect 82, wherein each of the at least one autosome is independently selected from the group consisting of human chromosome 1, 2, 3, 4, 5, 6, 7, 8, 9, 10, 11, 12, 13, 14, 15, 16, 17, 18, 19, 20, 21, and 22.

84. The reference material of aspect 83, wherein each of the at least one autosome is independently selected from the group consisting of human chromosome 1, 6, and 7.

85. The reference material of any one of aspects 82-84, wherein the chromosome is human chromosome 1, 2, 3, 4, 5, 6, 7, 8, 9, 10, 11, 12, 13, 14, 15, 16, 17, 18, 19, 20, 21, 22, X, or Y.

86. The reference material of aspect 85, wherein the chromosome is human chromosome 8, 9, 13, 18, 21, 22, or X.

87. The reference material of any one of aspects 16-30, 39-47, 51-53 and 82-86, wherein the first mixture of nucleic acids or the second mixture of nucleic acids encodes substantially all of a human genome.

88. The reference material of any one of aspects 16-30, 39-47, 51-53 and 82-86, wherein the first mixture of nucleic acids and the second mixture of nucleic acids encodes substantially all of a human genome.

89. The reference material of aspect 87 or 88, wherein the human genome is an immortalized lymphocyte cell line genome, a fibroblast cell line genome, or a cytotrophoblast cell line genome.

90. The reference material of aspect 87 or 88, wherein the human genome is a peripheral blood mononuclear cell genome.

91. The reference material of aspect 87 or 88, wherein the human genome is a lymphocyte, fibroblast, placenta, or adipocyte genome.

92. The reference material of any one of aspects 16-30, 39-47, 51-53 and 82-91, wherein the first mixture of nucleic acids or the second mixture of nucleic acids comprise mitochondrial nucleotide sequences.

93. The reference material of any one of aspects 16-30, 39-47, 51-53 and 82-91, wherein the first mixture of nucleic acids and the second mixture of nucleic acids are free of mitochondrial nucleotide sequences.

94. The reference material of any one of aspects 16-30, 39-47, 51-53 and 82-93, wherein the first mixture of nucleic acids or the second mixture of nucleic acids comprise methylated DNA.

95. The reference material of any one of aspects 16-30, 39-47, 51-53 and 82-93, wherein the first mixture of nucleic acids and the second mixture of nucleic acids are free of methylated DNA.

96. The reference material of any one of aspects 16-30, 39-47, 51-53 and 82-96, wherein the first mixture of nucleic acids or the second mixture of nucleic acids are derived from a plasmid.

97. The reference material of any one of aspects 16-30, 39-47, 51-53 and 82-97, wherein the ratio of the concentration of nucleic acids in the first mixture to the concentration of nucleic acids in the second mixture is about 1: 1000 to about 1:1.

98. The reference material of aspect 97, wherein the ratio of the concentration of nucleic acids in the first mixture to the concentration of nucleic acids in the second mixture is about 1:200 to about 1:3.

99. The reference material of any one of aspects 16-30, 39-47, 51-53 and 82-99, wherein the nucleic acids in the first mixture make up about 5% to about 15% of the total concentration of nucleic acids in the reference material.

100. The reference material of any one of aspects 16-30, 39-47, 51-53 and 82-100, wherein the median length of the nucleic acids in the reference material is about 35 base pairs to about 1000 base pairs.

101. The reference material of aspect 100, wherein the median length of the nucleic acids in the reference material is about 50 base pairs to about 500 base pairs.

102. The reference material of any one of aspects 16-30, 39-47, 51-53 and 82-101, wherein the cfDNA is isolated from a biological sample selected from: amniotic fluid, blood, plasma, serum, semen, lymphatic fluid, cerebral spinal fluid, ocular fluid, urine, saliva, stool, mucous, sweat, or cell supernatant of cultured cells.

103. The reference material of any one of aspects 16-30, 39-47, 51-53 and 82-102, wherein the sample of cfDNA is cell-free circulating tumor DNA ("ctDNA").

104. The reference material of any one of aspects 16-30, 39-47, 51-53 and 82-103, wherein the nucleic acids of the reference material do not form aggregates when stored at a temperature of about 0°C to about 100°C.

105. The reference material of any one of aspects 16-30, 39-47, 51-53 and 82-104, wherein the nucleic acids of the reference material do not form aggregates when stored for a period of time from about 1 day to about 5 years.

106. The reference material of any one of aspects 16-30, 39-47, 51-53 and 82-105, wherein the concentration of nucleic acids in the reference material is about 1 ng/mL to about 100 ng/mL.

107. The reference material of any one of aspects 16-30, 39-47, 51-53 and 82-106, wherein the concentration of nucleic acids in the reference material is greater than 100 ng/mL.

108. The reference material of any one of aspects 16-30, 39-47, 51-53 and 82-107, wherein the reference material is encapsulated by a lipids or protein to generate a commutable reference material.

109. The commutable reference material of aspect 108, wherein the reference material has at least 80% sequence similarity to a natural analyte.

110. A method for quantitative analysis of cell-free DNA (cfDNA), comprising:

(a) treating the 5' end or 3' end or both of cfDNA with one or more end-repair enzymes to generate end-repaired cfDNA;
(b) ligating one or more adaptors, wherein the adaptors comprise a restriction enzyme site and a sequence from a respiratory syncytial virus (RSV), to each end of the end-repaired cfDNA to generate a cfDNA library;
(c) amplifying the cfDNA library to generate a cfDNA library clone;
(d) digesting the cfDNA library clone with a restriction enzyme to generate a modified cfDNA clone library; wherein digesting the cfDNA library clones with the restriction enzyme removes the one or more adaptors; and
(e) purifying the modified cfDNA clone library.

111. The method of aspect 110, wherein the amplified cfDNA is a control.

112. The method of aspect 110, wherein the amplified cfDNA is a reference material.

113. The method of any one of aspects 110 to 112, wherein the RSV sequence comprises a modified sequence.

114. The method of aspect 110, wherein the amplifying the cfDNA library is stopped prior to the amplification reaching a plateau.

115. The method of aspect 110, wherein at least about 50% of the 5' end and 3' end of the modified cfDNA clone library comprise the same cfDNA sequence as the 5' end and 3' end of the end-repaired cfDNA.

116. The method of aspect 110, further comprising isolating cfDNA from a biological sample of a subject.

117. The method of any one of aspects 116, wherein the cfDNA is isolated from a biological sample selected from: amniotic fluid, blood, plasma, serum, semen, lymphatic fluid, cerebral spinal fluid, ocular fluid, urine, saliva, stool, mucous, sweat, or cell supernatant of cultured cells.

118. The method of any one of aspects 110-117, wherein the treating of the 5' end and 3' end of cfDNA with one or more end-repair enzymes further consists of adding a dA base to the 3' end of the cfDNA.

119. The method of any one of aspects 110-118, wherein the 5' and 3' ends of the end-repaired cfDNA are blunt ends.

120. The method of any one of aspects 110-119, wherein the one or more adaptors comprise a plurality of adaptor species.

121. The method of any one of aspects 110-120, wherein the one or more adaptors comprise a primer binding site for amplification of the cfDNA library.

122. The method of any one of aspects 110-120, wherein the one or more adaptors comprise at least two primer binding sites for amplification of the cfDNA library

123. The method of aspect 121 or 122, wherein the adaptor comprises two single stranded DNA molecules.

124. The method of aspect 123, wherein the two single stranded DNA molecules are annealed.

125. The method of aspect 123 or 124, wherein the adaptor is SEQ ID NO:1 and SEQ ID NO:2.

126. The method of any one of aspects 110-125, wherein the one or more adaptors comprise one or more unique read codes.

127. The method of any one of aspects 110-126, wherein the one or more adaptors comprise one or more sample codes for sample multiplexing.

128. The method of any one of aspects 110-125, wherein the one or more adaptors comprise one or more sequences for DNA sequencing.

129. The method of any one of aspects 110 to 128, wherein the restriction enzyme is SapI.

130. The method of any one of aspects 110 to 129, wherein the cfDNA library comprises genomic DNA.

131. The method of aspect 130, wherein the genomic DNA has a sequence length greater than 600 base pairs.

132. The method of aspect 131, wherein the genomic DNA is removed from the cfDNA library by addition of AMPure XP beads.

133. The method of aspect 131, wherein the genomic DNA is removed from the cfDNA library by size selection.

134. The method of aspect 130, wherein size selection is agarose gel electrophoresis.

135. The method of any one of aspects 110 to 134, wherein the cfDNA library is at or below 600 base pairs.

136. The method of any one of aspects 110 to 135, wherein a PCR reaction is performed to amplify the cfDNA library.

137. The method of aspect 136, wherein the PCR reaction comprises a proofreading thermostable polymerase.

138. The method of aspect 137, wherein the primers comprise a modification at the 5' end of the primer.

139. The method of aspect 138, wherein the modification at the 5' end of the primer is biotinylation.

140. The method of aspect 137 or 138, wherein the modification at the 5' end of the primer comprises phosphorothioation with at least 1, 2, 3, or 4 nucleotides.

141. The method of any one of aspects 136 to 140, wherein the primers are SEQ ID NO:3 and SEQ ID NO:4.

142. The method of aspect 136, wherein the concentration of amplified cfDNA library produced per PCR reaction is at least 0.1 ng/$\mu$l.

143. The method of any one of aspects 110 to 142, wherein the amplified cfDNA library comprises a sufficient complexity.

144. The method of aspect 143, wherein the sufficient complexity is an amplified input cfDNA library represented in at least 10 copies of a genomic location.

145. The method of aspect 143, wherein the sufficient complexity comprises multiple cfDNA libraries.

146. The method of aspect 145, wherein the amplified products of the multiple cfDNA libraries are pooled.

147. The method of any one of aspects 110 to 146, wherein at least two cfDNA libraries are distributed into multiple PCR reactions.

148. The method of aspect 147, wherein the reactions are performed in parallel.

149. The method of aspect 147, wherein the reactions are not performed in parallel.

150. The method of aspect 147, wherein the products from multiple PCR reactions are pooled together.

151. The method of aspect 150, wherein the pooling of reactions is after the digestion with the restriction enzyme.

152. The method of any one of aspects 110 to 151, further comprising a quantitative analysis performed on a plurality of loci in the cfDNA library clones.

153. The method of aspect 152, wherein the quantitative analysis is performed on a plurality of loci in a plurality of cfDNA clone libraries.

154. The method of aspect 153, wherein the quantitative analysis comprises hybridizing one or more capture probes to a target locus to form capture probe-cfDNA clone complexes.

155. The method of aspect 154, wherein the quantitative analysis comprises isolating the capture probe-cfDNA clone complexes.

156. The method of aspect 155, wherein the quantitative analysis comprises amplification of the cfDNA clone sequence in the isolated hybridized capture probe-cfDNA clone complexes.

157. The method of any one of aspects 152 to 156, wherein the quantitative analysis comprises DNA sequencing to generate a plurality of sequencing reads.

158. The method of aspect 157, further comprising bioinformatic analysis of the plurality of sequencing reads.

159. The method of aspect 158, wherein bioinformatics analysis is used:

(a) to quantify the number of genome equivalents analyzed in the cfDNA clone library;
(b) to detect genetic variants in a target genetic locus;
(c) to detect mutations within a target genetic locus;
(d) to detect genetic fusions within a target genetic locus; and
(e) to measure copy number fluctuations within a target genetic locus.

160. The method of any one of aspects 110 to 159, wherein the subject has been diagnosed with a genetic disease.

161. The method of aspect 160, wherein the quantitative genetic analysis is used to identify or detect one or more genetic lesions that cause or are associated with the genetic disease.

162. The method of aspect 161, wherein the genetic lesion comprises a nucleotide transition or transversion, a nucleotide insertion or deletion, a genomic rearrangement, a change in copy number, or a gene fusion.

163. The method of any one of aspects 160-162, wherein the genetic disease is cancer.

164. The method of any one of aspects 116-163, wherein the subject is a fetus.

165. The method of any one of aspects 116-163, wherein the subject is pregnant.

166. The method of any one of aspects 116-163, wherein the subject is pregnant and has a cancer.

167. The method of any one of aspects 164 to 167, wherein the quantitative analysis is used to identify or detect one or more genetic variants or genetic lesions of one or more target genetic loci in fetal cfDNA.

168. The method of aspect 152, wherein the quantitative analysis is used to identify or detect fetal cfDNA in the subject.

169. The method of any one of aspects 116-163, wherein the subject is a transplant recipient.

170. The method of aspect 169, wherein the quantitative analysis is used to identify or detect donor cfDNA in the subject.

171. A method of predicting, diagnosing, or monitoring a genetic disease in a subject comprising:

(a) isolating or obtaining a sample of cfDNA from a subject;

(b) treating the 5' end or 3' end or both of cfDNA with one or more end-repair enzymes to generate end-repaired cfDNA;

(c) ligating one or more adaptors, wherein the adaptors comprise a restriction enzyme site and a sequence from a respiratory syncytial virus (RSV), to each end of the end-repaired cfDNA to generate a cfDNA library;

(d) amplifying the cfDNA library to generate a cfDNA library clone;

(e) digesting the cfDNA library clone with a restriction enzyme to generate a modified cfDNA clone library; wherein the digesting the cfDNA library clone with the restriction enzyme removes the one or more adaptors; and

(f) purifying the modified cfDNA clone library.

172. The method of aspect 171, wherein the amplifying the cfDNA library is stopped prior to the amplification reaching a plateau.

173. The method of aspect 171, wherein the RSV sequence comprises a modified sequence.

174. The method of aspect 171, wherein at least about 50% of the 5' end and 3' end of the modified cfDNA clone library comprise the same cfDNA sequence as the 5' end and 3' end of the end-repaired cfDNA.

175. The method of aspect 171, wherein the cfDNA is isolated from a biological sample selected from: amniotic fluid, blood, plasma, serum, semen, lymphatic fluid, cerebral spinal fluid, ocular fluid, urine, saliva, stool, mucous, sweat, or cell supernatant of cultured cells.

176. The method of aspect 171, wherein the genetic lesion comprises a nucleotide transition or transversion, a nucleotide insertion or deletion, a genomic rearrangement, a change in copy number, or a gene fusion.

177. A method of predicting, diagnosing, or monitoring a cancer in a subject comprising:

(a) isolating or obtaining a sample of circulating tumor DNA ("ctDNA") from a subject;

(b) treating the 5' end or 3' end or both of ctDNA with one or more end-repair enzymes to generate end-repaired ctDNA;

(c) ligating one or more adaptors, wherein the adaptors comprise a restriction enzyme site and a sequence from a respiratory syncytial virus (RSV), to each end of the end-repaired ctDNA to generate a ctDNA library;

(d) amplifying the ctDNA library to generate a ctDNA library clone;

(e) digesting the ctDNA library clone with a restriction enzyme to generate a modified ctDNA clone library; wherein the digesting the ctDNA library clone with the restriction enzyme removes the one or more adaptors; and

(f) purifying the modified ctDNA clone library.

178. The method of aspect 177, wherein the amplifying the ctDNA library is stopped prior to the amplification reaching a plateau.

179. The method of aspect 177, wherein the RSV sequence comprises a modified sequence.

180. The method of aspect 177, wherein at least about 50% of the 5' end and 3' end of the modified ctDNA clone library comprise the same cfDNA sequence as the 5' end and 3' end of the end-repaired cfDNA.

181. The method of aspect 177, wherein the ctDNA is isolated from a biological sample selected from: amniotic fluid, blood, plasma, serum, semen, lymphatic fluid, cerebral spinal fluid, ocular fluid, urine, saliva, stool, mucous, sweat, or cell supernatant of cultured cells.

182. The method of aspect 177, wherein the somatic alteration is genomic alteration, epigenomic alteration, point mutation, rearranged genomic sequence, copy number variant (CNV), microsatellite instability (MSI), loss of heterozygosity (LOH), or DNA methylation.

183. A method for validating a diagnostic test for analysis of a sample of circulating cell-free DNA, comprising performing the diagnostic test on the control of any one of aspects 1-15, 31-38, and 48-81 or the reference material of any one of aspects 16-30, 39-47, 51-53 and 82-107, wherein the diagnostic test is validated if it correctly identifies the genotype of the control.

184. The method of aspect 183, wherein the sample of circulating cell-free DNA is cell-free circulating tumor DNA ("ctDNA").

185. A method for determining whether a sample comprises a genotype, comprising:

performing a diagnostic test on the sample; and
performing the diagnostic test on a control of any one of aspects 1-15, 31-38, and 48-81, or a reference material of any one of aspects 16-30, 39-47, 51-53 and 82-107, wherein the control comprises the genotype; wherein:

the sample is found to comprise the genotype if the diagnostic test indicates that both the sample and the control comprise the genotype;
the sample is found to not comprise the genotype if the diagnostic test indicates that the sample does not comprise the genotype but that the control comprises the genotype; and
the diagnostic test is found to be inconclusive if the test indicates that the control does not comprise the genotype.

186. The method of any one of aspects 110 to 185, wherein the cfDNA or ctDNA, prior to amplification, is a bisulfite converted sample of DNA.

187. A method for quantitative analysis of cell-free DNA (cfDNA) comprising:

(a) converting the cfDNA to a bisulfite converted sample of cfDNA;
(b) treating the 5' end or 3' end or both of the converted cfDNA with one or more end-repair enzymes to generate end-repaired cfDNA;
(c) ligating one or more adaptors, wherein the adaptors comprise a restriction enzyme site and a sequence from a respiratory syncytial virus (RSV), to each end of the end-repaired cfDNA to generate a cfDNA library;
(d) amplifying the cfDNA library to generate a cfDNA library clone;
(e) digesting the cfDNA library clone with a restriction enzyme to generate a modified cfDNA clone library; wherein digesting the cfDNA library clones with the restriction enzyme removes the one or more adaptors; and
(f) purifying the modified cfDNA clone library.

188. A method of predicting, diagnosing, or monitoring a genetic disease in a subject comprising:

(a) isolating or obtaining a sample of cfDNA from a subject;
(b) converting the cfDNA to a bisulfite converted sample of cfDNA;
(c) treating the 5' end or 3' end or both of the converted cfDNA with one or more end-repair enzymes to generate end-repaired cfDNA;
(d) ligating one or more adaptors, wherein the adaptors comprise a restriction enzyme site and a sequence from a respiratory syncytial virus (RSV), to each end of the end-repaired cfDNA to generate a cfDNA library;
(e) amplifying the cfDNA library to generate a cfDNA library clone;
(f) digesting the cfDNA library clone with a restriction enzyme to generate a modified cfDNA clone library; wherein the digesting the cfDNA library clone with the restriction enzyme removes the one or more adaptors; and
(g) purifying the modified cfDNA clone library.

189. A method of predicting, diagnosing, or monitoring a cancer in a subject comprising:

(a) isolating or obtaining a sample of circulating tumor DNA ("ctDNA") from a subject;
(b) converting the ctDNA to a bisulfite converted sample of ctDNA;
(c) treating the 5' end or 3' end or both of the converted ctDNA with one or more end-repair enzymes to generate end-repaired ctDNA;
(d) ligating one or more adaptors, wherein the adaptors comprise a restriction enzyme site and a sequence from a respiratory syncytial virus (RSV), to each end of the end-repaired ctDNA to generate a ctDNA library;
(e) amplifying the ctDNA library to generate a ctDNA library clone;
(f) digesting the ctDNA library clone with a restriction enzyme to generate a modified ctDNA clone library; wherein the digesting the ctDNA library clone with the restriction enzyme removes the one or more adaptors; and
(g) purifying the modified ctDNA clone library.

190. The method of any one of aspects 110 to 189, wherein the amplified cfDNA or cfDNA is used as a control.

191. The method of aspect 190, wherein the control is a reference material.

192. The method of aspect 190 or 191, wherein the control or reference material is not mixed.

193. The method of any one of aspects 190-192, wherein the control or reference material is derived from a subject that is a donor.

194. The method of aspect 193, wherein the donor is pregnant or has a cancer.

195. The method of any one of aspects 110 to 195, wherein the ctDNA is initially sonicated and has a broad distribution.

196. The method of aspect 195, wherein the broad distribution is different from cfDNA.

197. The method of aspect 195, wherein the ctDNA is subsequently converted into a ctDNA library.

198. The method of aspect 197, wherein the library is separated by size using gel electrophoresis.

199. The method of aspect 198, wherein the library is extracted from excised agarose and subsequently amplified.

200. The method of aspect 199, wherein the amplified library is subsequently digested with a restriction enzyme.

201. The method of aspect 200, wherein the restriction enzyme is SapI.

202. The method of aspect 200, wherein the amplified library is subsequently purified to remove biotinylated molecules.

203. The method of aspect 202, wherein the purified library is a reference material or a control.

204. A method of generating a cfDNA control or reference material, comprising obtaining cfDNA from a subject, amplifying the cfDNA by PCR, and digesting the amplified cfDNA with a restriction enzyme.

205. The method of aspect 204, further comprising isolating cfDNA from a biological sample of the subject.

206. The method of aspect 204 or 205, wherein the cfDNA is isolated from a biological sample selected from: amniotic fluid, blood, plasma, serum, semen, lymphatic fluid, cerebral spinal fluid, ocular fluid, urine, saliva, stool, mucous, sweat, or cell supernatant of cultured cells.

207. The method of any one of aspects 204 to 206, wherein the subject has been diagnosed with a genetic disease.

208. The method of aspect 207, wherein the quantitative genetic analysis is used to identify or detect one or more genetic lesions that cause or are associated with the genetic disease.

209. The method of aspect 208, wherein the genetic lesion comprises a nucleotide transition or transversion, a nucleotide insertion or deletion, a genomic rearrangement, a change in copy number, or a gene fusion.

210. The method of any one of aspects 207-209, wherein the genetic disease is cancer.

211. The method of any one of aspects 204-210, wherein the subject is a fetus.

212. The method of any one of aspects 204-210, wherein the subject is pregnant.

213. The method of any one of aspects 204-210, wherein the subject is pregnant and has a cancer.

214. The method of any one of aspects 204 to 213, wherein the quantitative analysis is used to identify or detect one or more genetic variants or genetic lesions of one or more target genetic loci in fetal cfDNA.

215. The method of any one of aspects 204-214, wherein the restriction enzyme is Sapl.

216. The method of any one of aspects 204-215, wherein the size distribution and genomic representation biases in the cfDNA from the subject are represented in an output sample.

217. The method of any one of aspects 204-216, wherein the generated cfDNA control or reference material is used in a biological assay.

218. The method of any one of aspects 110 to 217, wherein the periodicity of the cfDNA or ctDNA peaks is the same as the original sample.

219. The method of aspect 218, wherein the periodicity of the DNA peaks will be maintained at about 166 bps, 146 bps, 136 bps, 126 bps, and/or 116 bps.

220. The method of any one of aspects 110 to 218, further comprising encapsulating the purified modified cfDNA clone library in a liposome, lipid, or a protein.

## Claims

1. A control for use in identifying a genotype, comprising a first mixture of nucleic acids and a second mixture of nucleic acids;

   wherein the first mixture of nucleic acids comprises a first plurality of nucleotide sequences, each nucleotide sequence of the plurality encodes a genotype of a first plurality of genotypes, and each genotype of the first plurality of genotypes is associated with a neoplasm;
   the second mixture of nucleic acids comprises a second plurality of nucleotide sequences, and each nucleotide sequence of the second plurality of nucleotide sequences encodes a genotype of a second plurality of genotypes, wherein the second plurality of genotypes comprises genotypes that are not associated with a disease; and
   the nucleic acids of the first mixture of nucleic acids or the nucleic acids of the second mixture have an average base pair (bp) length of about 50 bps to about 600 bps.

2. The control of claim 1, wherein:

   (i) the first mixture of nucleic acids or second mixture of nucleic acids encodes substantially all of a human genome;
   (ii) the ratio of the copy number of a nucleotide sequence that encodes a genotype of the first plurality of genotypes to the copy number of a nucleotide sequence that encodes a genotype of the second plurality of genotypes is 1:1000 to 1:1; and/or
   (iii) wherein:

      a) each genotype of the second plurality of genotypes is an alternate genotype that occurs at the same genetic locus as a genotype of the first plurality of genotypes; or
      b) each genotype of the second plurality of genotypes is not associated with a disease.

3. The control of claim 1 or 2, wherein:

   (i) each genotype of the first plurality of genotypes is a single nucleotide polymorphism, point mutation, premature stop codon, trinucleotide repeat, translocation, somatic rearrangement, allelomorph, single nucleotide variant, coding insertion or deletion ("indel"), splice variant, regulatory variant, copy number variant, or gene fusion;
   (ii) at least one genotype of the first plurality of genotypes consists of a mutation to a gene selected from the group consisting of MTOR, MPL, NRAS, PARP1, AKT3, DNMT3A, MSH2, IDH1, VHL, MLH1, MYD88, CTNNB1, ATR, PIK3CA, FGFR3, PDGFRA, KIT, FBXW7, APC, GABRG2, NPM1, EGFR, MET, BRAF, EZH2, JAK2, GNAQ, RET, PTEN, ATM, KRAS, PTPN11, FLT3, RB1, PARP2, ARHGAP5, AKT1, RAD51, IDH2, TP53, NF1, SMAD4, AKT2, ERCC1, and GNAS;
   (iii) at least one genotype of the first plurality of genotypes consists of a mutation to a gene selected from the group consisting of AKT1, ATM, BRAF, CDKN2A, CSF1R, EGFR, ERBB2 ("HER2"), ERBB4 ("HER4"), FGFR1, FGFR2, FGFR3, GNA11, HRAS, JAK2, JAK3, KDR, KIT, KRAS, MET, NOTCH1, NRAS, PDGFRA, PIK3CA, PTEN, RET, and STK11;

(iv) at least one genotype of the first plurality of genotypes consists of a mutation to a gene selected from the group consisting of ABL1, AKT1, ALK, APC, AR, ARID1A, ARAF, ATM, BCL2, BCR, BRAF, BRC42, BRCA1, BRCA2, BRIP1, CCND1, CCND2, CCNE1, CDH1, CDK4, CDK6, CDKN2A, CDKN2B, CSF1R, CTNNB1, DDR2, EGFR, ERBB2, ERBB3, ERBB4, ESR1, ETV1, ETV4, ETV6, EWSR1, EZH2, FANCA, FANCC, FANCD2, FANCE, FANCF, FANCG, FANCL, FBXW7, FGFR1, FGFR2, FGFR3, FLT3, FOXL2, GATA3, GNA11, GNAQ, GNAS, HER2/ERBB2, HNF1A, HPAS, HRAS, IDH1, IDH2, IHD2, JAK2, JAK3, KDR, KIT, KRAS, MAP2K1, MAP2K2, MET, MLH1, MLL, MPL, MSH2, MSH6, MTOR, MYC, MYCN, NF1, NF2, NFE2L2, NOTCH1, NPM1, NRAS, NTRK1, PALB2, PDGFRA, PDGFRB, PIK3CA, PMS2, PTCH1, PTEN, PTPN11, RAFT, RARA, RB1, RET, RHEB, RHOA, RIT1, ROS1, SMAD4, SMARCB1, SMO, SRC, STK11, TERT, TMPRSS2, TP53, TSC1, TSC2, and VHL;

(v) at least one genotype of the first plurality of genotypes consists of a mutation to a gene selected from the group consisting of BRAF, EGFR, ERBB2, and KRAS; or

(vi) at least one genotype of the first plurality of genotypes consists of a mutation to a gene selected from the group consisting of mutation V600E to gene BRAF, mutation T790M to gene EGFR, mutation delL747-P753insS to gene EGFR, mutation A775_G776insYVMA to gene ERBB2, and mutation G12D to gene KRAS; or

(vii) each genotype of the first plurality of genotypes consists of a mutation to a gene selected from the group consisting of MTOR, MPL, NRAS, PARP1, AKT3, DNMT3A, MSH2, IDH1, VHL, MLH1, MYD88, CTNNB1, ATR, PIK3CA, FGFR3, PDGFRA, KIT, FBXW7, APC, GABRG2, NPM1, EGFR, MET, BRAF, EZH2, JAK2, GNAQ, RET, PTEN, ATM, KRAS, PTPN11, FLT3, RB1, PARP2, ARHGAP5, AKT1, RAD51, IDH2, TP53, NF1, SMAD4, AKT2, ERCC1, and GNAS;

(viii) each genotype of the first plurality of genotypes consists of a mutation to a gene selected from the group consisting of AKT1, ATM, BRAF, CDKN2A, CSF1R, EGFR, ERBB2 ("HER2"), ERBB4 ("HER4"), FGFR1, FGFR2, FGFR3, GNA11, HRAS, JAK2, JAK3, KDR, KIT, KRAS, MET, NOTCH1, NRAS, PDGFRA, PIK3CA, PTEN, RET, and STK11;

(ix) each genotype of the first plurality of genotypes consists of a mutation to a gene selected from the group consisting of ABL1, AKT1, ALK, APC, AR, ARID1A, ARAF, ATM, BCL2, BCR, BRAF, BRC42, BRCA1, BRCA2, BRIP1, CCND1, CCND2, CCNE1, CDH1, CDK4, CDK6, CDKN2A, CDKN2B, CSF1R, CTNNB1, DDR2, EGFR, ERBB2, ERBB3, ERBB4, ESR1, ETV1, ETV4, ETV6, EWSR1, EZH2, FANCA, FANCC, FANCD2, FANCE, FANCF, FANCG, FANCL, FBXW7, FGFR1, FGFR2, FGFR3, FLT3, FOXL2, GATA3, GNA11, GNAQ, GNAS, HER/ERBB2, HNF1A, HPAS, HRAS, IDH1, IDH2, IHD2, JAK2, JAK3, KDR, KIT, KRAS, MAP2K1, MAP2K2, MET, MLH1, MLL, MPL, MSH2, MSH6, MTOR, MYC, MYCN, NF1, NF2, NFE2L2, NOTCH1, NPM1, NRAS, NTRK1, PALB2, PDGFRA, PDGFRB, PIK3CA, PMS2, PTCH1, PTEN, PTPN11, RAFT, RARA, RB1, RET, RHEB, RHOA, RIT1, ROS1, SMAD4, SMARCB1, SMO, SRC, STK11, TERT, TMPRSS2, TP53, TSC1, TSC2, and VHL;

(x) each genotype of the first plurality of genotypes consists of a mutation to a gene selected from the group consisting of BRAF, EGFR, ERBB2, and KRAS; or

(xi) each genotype of the first plurality of genotypes consists of a mutation to a gene selected from the group consisting of mutation V600E to gene BRAF, mutation T790M to gene EGFR, mutation delL747-P753insS to gene EGFR, mutation A775_G776insYVMA to gene ERBB2, and mutation G12D to gene KRAS; or

(xii) the first plurality of genotypes comprises mutation V600E to gene BRAF, mutation T790M to gene EGFR, mutation delL747-P753insS to gene EGFR, mutation A775_G776insYVMA to gene ERBB2, and mutation G12D to gene KRAS.

4. The control of any of claims 1-3, wherein:

(i) the first plurality of nucleotide sequences encodes 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 200, 300, 400, 500, 600, 700, 800, 900, or 1000 genotypes; or

(ii) the first plurality of nucleotide sequences encodes 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 200, 300, 400, 500, 600, 700, 800, 900, or 1000 genotypes and the second mixture of nucleic acids comprises a second plurality of nucleotide sequences encoding wild type genotypes corresponding to each genotype in the first plurality; and/or

(iii) the nucleic acids of the first mixture of nucleic acids and/or the nucleic acids of the second mixture have an average base pair (bp) length of about 100 bps to about 300 bps;
(iv) the first mixture of nucleic acids or the second mixture of nucleic acids comprise methylated DNA;
(v) the nucleic acids of the control are encapsulated in a liposome, a lipid, or a protein; and/or
(vi) the nucleic acids of the control comprise a SapI restriction enzyme overhang.

5. The control of any of claims 1-4, wherein the first and second mixtures of nucleic acids are prepared by a method comprising:

(i) obtaining DNA fragments, optionally by fragmenting input DNA;
(ii) amplifying the DNA fragments by PCR to generate an amplified library; and
(iii) digesting the amplified library with a restriction enzyme to generate a modified library.

6. A DNA library comprising a first mixture of nucleic acids and a second mixture of nucleic acids;

wherein the first mixture of nucleic acids comprises a first plurality of nucleotide sequences, each nucleotide sequence of the plurality encodes a genotype of a first plurality of genotypes, and each genotype of the first plurality of genotypes is associated with a neoplasm;
the second mixture of nucleic acids comprises a second plurality of nucleotide sequences, and each nucleotide sequence of the second plurality of nucleotide sequences encodes a genotype of a second plurality of genotypes, wherein the second plurality of genotypes comprises genotypes that are not associated with a disease;
the nucleic acids of the first mixture of nucleic acids or the nucleic acids of the second mixture have an average base pair (bp) length of about 50 bps to about 600 bps;
the nucleic acids of the first mixture of nucleic acids or second mixture of nucleic acids comprise one or more adaptors ligated to each end of the nucleic acid, wherein the adaptors comprise a restriction enzyme site, optionally a SapI site, and a non-human sequence, optionally a sequence from a nonintegrating RNA virus, such as a respiratory syncytial virus (RSV); and
wherein the restriction enzyme site is positioned to permit removal of the adaptor from the nucleic acids by the restriction enzyme.

7. A method of generating a control, comprising:

(i) obtaining DNA fragments, optionally by fragmenting input DNA;
(ii) amplifying the DNA fragments by PCR to generate an amplified library; and
(iii) digesting the amplified library with a restriction enzyme to generate a modified library.

8. The method of claim 7, further comprising:

a) treating the 5' end or 3' end or both of the DNA fragments with one or more end-repair enzymes to generate end-repaired DNA fragments;
b) dA-tailing the 3' end of the DNA fragments to generate dA-tailed DNA fragments; optionally further comprising: ligating one or more adaptors to each end of the end-repaired or dA-tailed DNA fragments to generate a DNA library, wherein the adaptors comprise a restriction enzyme site and a non-human sequence, optionally a sequence from a nonintegrating RNA virus, such as a respiratory syncytial virus (RSV), wherein when the amplified library is digested with the restriction enzyme, the restriction enzymes removes the one or more adaptors;
and/or
c) separating and purifying the DNA fragments with an average base pair (bp) length of about 50 bps to about 600 bps;
d) performing a test amplification prior to amplifying the DNA fragments by PCR to determine the mass of the amplifiable library, from which the complexity of the library can be estimated as well as the number of PCR cycles that can be used to amplify the library in a given reaction volume without reaching an amplicon concentration where that amplicon concentration is no longer approximately doubling with each cycle; optionally

wherein the number of PCR cycles that is determined based on the mass of the amplifiable input material is used in amplifying the DNA fragments by PCR to generate an amplified library.

9. The method of claim 7 or 8, wherein:

(i) the restriction enzyme site in the adaptors has the sequence 5'-GCTCTTC-3';
(ii) the primers used to amplify the DNA fragments by PCR:

    a) are biotinylated at the 5' end of the primer; or
    b) are phosphorothioated primers;

(iii) the input DNA is fragmented by sonication;
(iv) the input DNA is plasmid DNA, viral DNA, bacterial DNA, fungal DNA, animal DNA, genomic DNA, circulating cell-free DNA (cfDNA), circulating tumour DNA (ctDNA); and/or
(v) the input DNA is a mixture of genomic DNA and plasmid DNA.

10. The method of any of claims 7-9, wherein:

    (i) the modified library is mixed with additional nucleic acids to generate the control; or
    (ii) the input DNA is obtained from two different sources and the two input DNAs are mixed prior to fragmentation.

11. A control for use in identifying a genotype, comprising a first mixture of amplified nucleic acids;

    wherein the first mixture of nucleic acids comprises a first plurality of nucleotide sequences, each nucleotide sequence of the plurality encodes a genotype of a first plurality of genotypes, and each genotype of the first plurality of genotypes is associated with a neoplasm;
    the first mixture of nucleic acids is substantially free of methylated DNA; and
    the nucleic acids of the first mixture of nucleic acids have an average base pair (bp) length of about 50 bps to about 600 bps.

12. The control of claim 11, wherein the first mixture of nucleic acids encodes substantially all of a human genome.

13. The control of claim 11 or 12, wherein:

    (i) each genotype of the first plurality of genotypes is a single nucleotide polymorphism, point mutation, premature stop codon, trinucleotide repeat, translocation, somatic rearrangement, allelomorph, single nucleotide variant, coding insertion or deletion ("indel"), splice variant, regulatory variant, copy number variant, or gene fusion.
    (ii) at least one genotype of the first plurality of genotypes consists of a mutation to a gene selected from the group consisting of MTOR, MPL, NRAS, PARP1, AKT3, DNMT3A, MSH2, IDH1, VHL, MLH1, MYD88, CTNNB1, ATR, PIK3CA, FGFR3, PDGFRA, KIT, FBXW7, APC, GABRG2, NPM1, EGFR, MET, BRAF, EZH2, JAK2, GNAQ, RET, PTEN, ATM, KRAS, PTPN11, FLT3, RB1, PARP2, ARHGAP5, AKT1, RAD51, IDH2, TP53, NF1, SMAD4, AKT2, ERCC1, and GNAS;
    (iii) at least one genotype of the first plurality of genotypes consists of a mutation to a gene selected from the group consisting of AKT1, ATM, BRAF, CDKN2A, CSF1R, EGFR, ERBB2 ("HER2"), ERBB4 ("HER4"), FGFR1, FGFR2, FGFR3, GNA11, HRAS, JAK2, JAK3, KDR, KIT, KRAS, MET, NOTCH1, NRAS, PDGFRA, PIK3CA, PTEN, RET, and STK11;
    (iv) at least one genotype of the first plurality of genotypes consists of a mutation to a gene selected from the group consisting of ABL1, AKT1, ALK, APC, AR, ARID1A, ARAF, ATM, BCL2, BCR, BRAF, BRC42, BRCA1, BRCA2, BRIP1, CCND1, CCND2, CCNE1, CDH1, CDK4, CDK6, CDKN2A, CDKN2B, CSF1R, CTNNB1, DDR2, EGFR, ERBB2, ERBB3, ERBB4, ESR1, ETV1, ETV4, ETV6, EWSR1, EZH2, FANCA, FANCC, FANCD2, FANCE, FANCF, FANCG, FANCL, FBXW7, FGFR1, FGFR2, FGFR3, FLT3, FOXL2, GATA3, GNA11, GNAQ, GNAS, HER/ERBB2, HNF1A, HPAS, HRAS, IDH1, IDH2, IHD2, JAK2, JAK3, KDR, KIT, KRAS, MAP2K1, MAP2K2, MET, MLH1, MLL, MPL, MSH2, MSH6, MTOR, MYC, MYCN, NF1, NF2, NFE2L2, NOTCH1, NPM1, NRAS, NTRK1, PALB2, PDGFRA, PDGFRB, PIK3CA, PMS2, PTCH1, PTEN, PTPN11, RAFT, RARA, RB1, RET, RHEB, RHOA, RIT1, ROS1, SMAD4, SMARCB1, SMO, SRC, STK11, TERT, TMPRSS2, TP53, TSC1, TSC2, and VHL;
    (v) at least one genotype of the first plurality of genotypes consists of a mutation to a gene selected from the group consisting of BRAF, EGFR, ERBB2, and KRAS; or
    (vi) at least one genotype of the first plurality of genotypes consists of a mutation to a gene selected from the group consisting of mutation V600E to gene BRAF, mutation T790M to gene EGFR, mutation delL747-P753insS to gene EGFR, mutation A775_G776insYVMA to gene ERBB2, and mutation G12D to gene KRAS; or
    (vii) each genotype of the first plurality of genotypes consists of a mutation to a gene selected from the group consisting of MTOR, MPL, NRAS, PARP1, AKT3, DNMT3A, MSH2, IDH1, VHL, MLH1, MYD88, CTNNB1, ATR, PIK3CA, FGFR3, PDGFRA, KIT, FBXW7, APC, GABRG2, NPM1, EGFR, MET, BRAF, EZH2, JAK2, GNAQ, RET, PTEN, ATM, KRAS, PTPN11, FLT3, RB1, PARP2, ARHGAP5, AKT1, RAD51, IDH2, TP53, NF1, SMAD4, AKT2, ERCC1, and GNAS;

(viii) each genotype of the first plurality of genotypes consists of a mutation to a gene selected from the group consisting of AKT1, ATM, BRAF, CDKN2A, CSF1R, EGFR, ERBB2 ("HER2"), ERBB4 ("HER4"), FGFR1, FGFR2, FGFR3, GNA11, HRAS, JAK2, JAK3, KDR, KIT, KRAS, MET, NOTCH1, NRAS, PDGFRA, PIK3CA, PTEN, RET, and STK11;

(ix) each genotype of the first plurality of genotypes consists of a mutation to a gene selected from the group consisting of ABL1, AKT1, ALK, APC, AR, ARID1A, ARAF, ATM, BCL2, BCR, BRAF, BRC42, BRCA1, BRCA2, BRIP1, CCND1, CCND2, CCNE1, CDH1, CDK4, CDK6, CDKN2A, CDKN2B, CSF1R, CTNNB1, DDR2, EGFR, ERBB2, ERBB3, ERBB4, ESR1, ETV1, ETV4, ETV6, EWSR1, EZH2, FANCA, FANCC, FANCD2, FANCE, FANCF, FANCG, FANCL, FBXW7, FGFR1, FGFR2, FGFR3, FLT3, FOXL2, GATA3, GNA11, GNAQ, GNAS, HER/ERBB2, HNF1A, HPAS, HRAS, IDH1, IDH2, IHD2, JAK2, JAK3, KDR, KIT, KRAS, MAP2K1, MAP2K2, MET, MLH1, MLL, MPL, MSH2, MSH6, MTOR, MYC, MYCN, NF1, NF2, NFE2L2, NOTCH1, NPM1, NRAS, NTRK1, PALB2, PDGFRA, PDGFRB, PIK3CA, PMS2, PTCH1, PTEN, PTPN11, RAFT, RARA, RB1, RET, RHEB, RHOA, RIT1, ROS1, SMAD4, SMARCB1, SMO, SRC, STK11, TERT, TMPRSS2, TP53, TSC1, TSC2, and VHL;

(x) each genotype of the first plurality of genotypes consists of a mutation to a gene selected from the group consisting of BRAF, EGFR, ERBB2, and KRAS; or

(xi) each genotype of the first plurality of genotypes consists of a mutation to a gene selected from the group consisting of mutation V600E to gene BRAF, mutation T790M to gene EGFR, mutation delL747-P753insS to gene EGFR, mutation A775_G776insYVMA to gene ERBB2, and mutation G12D to gene KRAS; or

(xii) the first plurality of genotypes comprises mutation V600E to gene BRAF, mutation T790M to gene EGFR, mutation delL747-P753insS to gene EGFR, mutation A775_G776insYVMA to gene ERBB2, and mutation G12D to gene KRAS.

14. The control of any of claims 11-13, wherein:

(i) the first plurality of nucleotide sequences encodes 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 200, 300, 400, 500, 600, 700, 800, 900, or 1000 genotypes;
(ii) the nucleic acids of the first mixture of nucleic acids have an average base pair (bp) length of about 100 bps to about 300 bps;
(iii) the nucleic acids of the control are encapsulated in a liposome, a lipid, or a protein;
(iv) the nucleic acids of the control comprise a SapI restriction enzyme overhang.

15. The control of any of claims 11-14, wherein the first mixture of nucleic acids is prepared by a method comprising:

(i) obtaining DNA fragments, optionally by fragmenting input DNA;
(ii) amplifying the DNA fragments by PCR to generate an amplified library; and
(iii) digesting the amplified library with a restriction enzyme to generate a modified library.

16. A control for use in determining the ploidy of a chromosome in a fetus, comprising:

a first mixture of nucleic acids comprising a first nucleotide sequence and a second nucleotide sequence, wherein the first nucleotide sequence has sequence homology with the chromosome; the second nucleotide sequence has sequence homology with a different chromosome; and the ratio of the copy number of the first nucleotide sequence to the copy number of the second nucleotide sequence is greater than 1: 1;
a second mixture of nucleic acids comprising a first nucleotide sequence and a second nucleotide sequence, wherein the ratio of the copy number of the first nucleotide sequence to the copy number of the second nucleotide sequence is 1: 1; and

the nucleic acids of the first mixture of nucleic acids or the nucleic acids of the second mixture of nucleic acids comprise amplified cfDNA and have an average base pair (bp) length of about 50 bps to about 600 bps.

# Figure 1

## A

SAPI RESTRICTION ENZYME

RECOGNITION SEQUENCE
(NOT A PALINDROME; 1 IN 8,192 BASES)

## B

ccfDNA AMPLIFICATION STRATEGY (1/3)

ccfDNA END POLISHING

dA-TAILING

ADAPTER LIGATION   (NGS WGS USES SIMILAR ADAPTERS)

PCR

# Figure 1 (continued)

## C

REVISED ADAPTER

RSV-BASED; THEN EDITED TO REMOVE ADDITIONAL OVERLAPS
PAGE-PURIFIED TO IMPROVE LIGATION EFFICIENCY

DERIVED FROM RSV SEQUENCE

DELTA C: -22.56 kcal/mole  EASE PAIRS 13

```
5'   GAAGAGCCCTATATGGGATGATGTGACCGTGGT
     ||||||||||||||   :   :      :
3'  TGTTGTCGGGATATGTTGTGCGGTCCGTTTTAGT
```

SAPL SITE

40°C ANNEALING TEMPERATURE

DERIVED FROM RSV SEQUENCE

# Figure 2

## A

## B

WITHOUT REMOVAL

# Figure 2 (continued)
## C

## D

#20 AMPLIFIED
#20 SAPL
#20 AXP AMPLIFIED
#64 AXP AMPLIFIED
#64 AXP/SAPL
cDNA AMPLIFIED
cDNA SAPL

# Figure 3

## A

PCR REANNEALING

RANDOM ccfDNA INSERT

DENATURE, ANNEAL, EXTEND

Tm 1    DESIRED AMPLIFICATION

IF THE CONCENTRATION OF PRODUCT BECOMES TOO HIGH (AND PRIMERS TOO LOW),
ENDS WITH DIFFERENT INSERTS (HIGH COMPLEXITY) CAN RANDOMLY REANNEAL INSTEAD

Tm 2 (> Tm 1)

## B

REANNEALING AFFECTS SAPL DIGESTION

SAPL DIGESTION

5′  ... N G C T C T T C T 1 2 3 4 5 ...  3′
3′  ... N C G A G A A G A 1 2 3 4 5 ...  5′

SAPL DIGESTION FAILS DUE TO MISMATCH

5′  ... N G C T C T T C T 1 2 3 4 5 ...  3′
3′  ... N C G A G A A G A I II III IV V ...  5′

# Figure 3 (continued)

## C

# Figure 4

## A

## B

# Figure 5

ccfDNA AMPLIFICATION STRATEGY (2/3)

PCR AMPLIFIED ccfDNA

Figure 6

A

## Figure 6 (continued)

## B

ccfDNA AMPLIFICATION STRATEGY

PURIFICATION

END POLISHING

INPUT ccfDNA SEQUENCES ARE RECOVERED

## Figure 7

# Figure 8

## Figure 9

## Figure 10

# Figure 11

## A

## B

# Figure 12

## A

RUN SUMMARY

| 11.1G | 88 |
|-------|-----|
| TOTAL BASES | KEY SIGNAL |

82,497,993

TOTAL READS

| 134 bp | 144 bp | 164 bp |
|--------|--------|--------|
| MEAN | MEDIAN | MODE |

READ LENGTH

91%

ISP LOADING

62%

USABLE READS

ISP DENSITY

ISP SUMMARY

READ LENGTH HISTOGRAM

MATT1-517- SERACARE_GROUP1
LOADING DENSITY (AVG ~ 91%)

10656 WELLS / 15456 WELLS

| 91% LOADING | 134,759,483 | 9% EMPTY WELLS |
| 100% ENRICHMENT | 134,591,651 | 0% NO TEMPLATE |
| 68% CLONAL | 91,367,483 | 32% POLYCLONAL |
| 90% FINAL LIBRARY | 82,497,993 | 1% TEST FRAGMENTS / 0% ADAPTER DIMER / 9% LOW QUALITY |

## Figure 12 (continued)

A

CONSENSUS KEY 1-MER - LIBRARY AVE. PEAK = 88

| ADDRESSABLE WELLS | 148,155,732 | |
|---|---|---|
| WITH ISPS | 134,759,483 | 91.0% |
| LIVE | 134,591,651 | 99.9% |
| TEST FRAGMENT | 715,233 | 00.5% |
| LIBRARY | 133,876,418 | 99.5% |
| **LIBRARY ISPs** | **133,876,418** | |
| FILTERED: POLYCLONAL | 43,224,168 | 32.3% |
| FILTERED: LOW QUALITY | 8,883,061 | 06.6% |
| FILTERED: ADAPTER DIMER | 17,533 | 00.0% |
| **FINAL LIBRARY ISPs** | **82,497,993** | **61.6%** |

NOTES: FOR PLANNING RUNS INITIALLY INTENDED TO BE ANALYSED BY THE IONA SOFTWARE - ION CHEF ONLY.

| BARCODE NAME | SAMPLE | BASES | ≥Q20 | READS | MEAN READ LENGTH |
|---|---|---|---|---|---|
| NO BARCODE | NONE | 103,512,933 | 81,503,204 | 737,590 | 140 BP |
| PMH001A | NONE | 912,749,107 | 725,082,036 | 7,105,945 | 128 BP |
| PMH002A | NONE | 1,343,415,415 | 1,050,636,635 | 10,070,704 | 133 BP |
| PMH003A | NONE | 911,183,708 | 716,977,013 | 6,845,892 | 133 BP |
| PMH004A | NONE | 1,320,818,159 | 1,015,228,287 | 9,553,656 | 138 BP |
| PMH005A | NONE | 1,002,855,246 | 756,053,496 | 7,131,840 | 141 BP |
| PMH010A | NONE | 19,810,924 | 15,295,582 | 150,052 | 132 BP |
| PMH013A | NONE | 994,993,653 | 765,471,671 | 7,459,197 | 133 BP |
| PMH014A | NONE | 1,345,158,534 | 1,036,415,568 | 9,933,419 | 135 BP |
| PMH015A | NONE | 760,983,295 | 599,331,023 | 5,810,900 | 131 BP |
| PMH016A | NONE | 778,765,939 | 614,957,642 | 5,729,328 | 136 BP |

# Figure 12 (continued)

## B

| PMH018A | NONE | 1,564,747,880 | 1,206,293,719 | 11,897,018 | 132 bp |
|---------|------|---------------|---------------|------------|--------|

| TEST FRAGMENT | READS | PERCENT 50AQ17 | READ LENGTH HISTOGRAM |
|---------------|-------|----------------|----------------------|
| TF_C | 462,366 | 80% | |
| TF_1 | 199,749 | 90% | |

# Figure 12 (continued)

## C

ALIGNMENT SUMMARY (ALIGNED TO HOMO SAPIENS)

| 10.6 G | 3.4X |
|---|---|
| TOTAL ALIGNMENT BASES | AVERAGE COVERAGE DEPTH OF REFERENCE |

81,687,951

96% ALIGNED BASES

4% UNALIGNED

|  | COUNT | % |
|---|---|---|
| TOTAL READS | 81,687,951 | ~ |
| ALIGNED READS | 80,324,934 | 98.3% |
| UNALIGNED READS | 1,363,017 | 1.7% |

98.2%

MEAN RAW ACCURACY 1X

### ALIGNMENT QUALITY

| | AQ17 | AQ20 | PERFECT |
|---|---|---|---|
| TOTAL NUMBER OF BASES [Mbp] | 8.69 G | 7.14 G | 5.49 G |
| MEAN LENGTH [bp] | 123 | 108 | 87 |
| LONGEST ALIGNMENT [bp] | 333 | 333 | 313 |
| MEAN COVERAGE DEPTH | 2.8 | 2.3 | 1.8 |

# Figure 12 (continued)

## C

## REFERENCES CITED IN THE DESCRIPTION

**Patent documents cited in the description**

- US 62423574

**Non-patent literature cited in the description**

- **AIGRAIN et al.** *BMC Genomics,* 13 June 2016, vol. 17, 458
- **BOLAND, G. M et al.** *Oncotarget,* 2015, vol. 5